(19) Europäisches Patentamt
European Patent Office
Office européen des brevets

(11) EP 1 304 374 A1

(12) **EUROPEAN PATENT APPLICATION**
published in accordance with Art. 158(3) EPC

(43) Date of publication:
**23.04.2003 Bulletin 2003/17**

(21) Application number: **01950004.0**

(22) Date of filing: **17.07.2001**

(51) Int Cl.⁷: **C12N 15/09**, C12Q 1/68

(86) International application number:
**PCT/JP01/06153**

(87) International publication number:
**WO 02/006467 (24.01.2002 Gazette 2002/04)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **18.07.2000 JP 2000218039**

(71) Applicant: **BML, Inc.**
**Tokyo 151-0051 (JP)**

(72) Inventors:
- **HATTORI, Hiroaki,**
  **General Laboratory, BML, Inc.**
  **Kawagoe-shi, Saitama 350-1101 (JP)**
- **TSUJI, Masahiro**
  **Minami-ku, Sapporo-shi, Hokkaido 005-002 (JP)**
- **OKADA, Tomoo**
  **Nerima-ku, Tokyo 176-0012 (JP)**
- **NAGANO, Makoto,**
  **General Laboratory, BML, Inc.**
  **Kawagoe-shi, Saitama 350-1101 (JP)**
- **EGASHIRA, Tohru,**
  **General Laboratory, BML, Inc.**
  **Kawagoe-shi, Saitama 350-1101 (JP)**
- **ISHIHARA, Mitsuaki,**
  **General Laboratory, BML, Inc.**
  **Kawagoe-shi, Saitama 350-1101 (JP)**
- **IWASAKI, Tadao, General Laboratory, BML, INC.**
  **Kawagoe-shi, Saitama 350-1101 (JP)**

(74) Representative: **Bohmann, Armin K., Dr.**
**Bohmann & Loosen,**
**Anwaltssozietät,**
**Sonnenstrasse 8**
**80331 München (DE)**

(54) **METHOD OF DETECTING LIPID METABOLIC ERRORS**

(57) The intended object of the present invention is to provide a more widely applicable means of diagnosing familial hyperlipemia with certainty. It has been found that this object can be attained by providing a method of detecting abnormality of lipid metabolism, wherein risk factors concerning abnormalities of lipid metabolism are detected on the basis of the correlation of 65 specific types of LDL receptor gene mutations with the abnormalities of lipid metabolism, as well as a method of detecting disease(s) by detecting risk factors for arteriosclerosis and/or ischemic heart diseases by employing as indices the abnormalities of lipid metabolism thus detected.

EP 1 304 374 A1

## Description

Technical Field

The present invention relates to a method of detecting diseases, and more particularly to a method of detecting abnormalities or errors of lipid metabolism through employment of certain gene mutations as indices.

Background Art

Errors of metabolism of serum lipids cause abnormal serum lipid levels, and possibly lead to undesirable pathological conditions. In particular, arteriosclerosis and ischemic heart diseases are typical diseases caused by abnormalities of serum lipid metabolism. However, a wide diversity of clinical symptoms are caused by abnormal metabolism of serum lipids causative of these specific pathological conditions, and thus their diagnoses are also diversified.

Of serum lipid metabolic disorders or errors, familial hypercholesterolemia (FH) is known to be caused by qualitative or quantitative abnormalities of the low-density-lipoprotein (LDL) receptor (R). FH presents high cholesterol levels—particularly high LDL cholesterol levels—in blood, and is a typical autosomal dominant hereditary disease characterized by early coronary heart disease. In other words, familial hypercholesterolemia is frequently responsible for coronary heart diseases, *inter alia,* those occurring during childhood.

FH manifests coronary heart disease in males in their 20s, and in post-menopausal women in their 40s. Homozygous familial hypercholesterolemia patients, who inherit two abnormal alleles from their parents, are rare (about 1 in one million individuals) and show very high serum cholesterol levels of about 1,000 mg/dL. Diagnosis of homozygous familial hypercholesterolemia is not difficult. In contrast, heterozygous familial hypercholesterolemia patients, who inherit one abnormal allele from their parents, are encountered at a frequency of about 1 in 500 individuals. In Japan, the number of patients suffering heterozygous familial hypercholesterolemia is estimated to be about 250,000. However, depending on the site of the LDL receptor gene at which abnormality exists, heterozygous FH exhibits different activities of LDL receptor protein in terms of the protein's ability to bind a ligand LDL and take it into cells. As a result of these differences in activity, heterozygous FH patients are known to follow a variety of courses leading to the development of arteriosclerosis. As has also been known, different types of abnormality lead to arteriosclerosis of different severity levels. In general, therapy of familial hypercholesterolemia is preferably initiated as early as possible in the juvenile stage by administering appropriate drugs, such as HMG-CoA reductase inhibitors, agents of fibrate series, and antioxidants, so as to prevent the onset of coronary atherosclerosis or arteriosclerosis, which aggravates with age. However, difficulty in diagnosis of heterozygous FH in childhood tends to impede early detection and treatment of FH. Precise diagnosis of FH would also be effective for enabling early diagnosis of potential FH in other family members; FH is dominantly inherited, and if FH is found in a certain family member, other family members can be predicted to suffer FH.

As mentioned above, current diagnosis indices for familial hypercholesterolemia include, among others, high blood cholesterol level, presence or absence of tendonous xanthoma, early coronary events, and familial history. All indices other than familial history and high blood cholesterol level are concerned with conditions of hypercholesterolemia of considerably advanced stage. Particularly for successful diagnosis of FH heterozygotes during childhood, in which elevation in serum cholesterol is not necessarily significant, use of only these diagnosis indices is insufficient.

In addition to the above-mentioned diagnosis indices, a new means for determining familial hypercholesterolemia through measuring LDL receptor activity has presently been suggested, in which tissue or cells collected from a living body are cultured to thereby induce expression of the LDL receptors of the cells, and binding, uptake, and catabolism of radioisotope-labeled LDL are measured for comparison with the case of cells from healthy subjects. Another approach is direct determination of the presence or absence of mutations of LDL receptors through PCR using genomic DNA obtained from patients. However, the former approach involves the problem that it does not lend itself to general practice, as it requires a special facility for handling a radioisotope, as well as skill to perform intricate manipulation procedure. Also, the latter approach has the drawback in that, since more than about 350 types of LDL receptor gene mutations have so far been identified, determination of respective mutations one by one through, for example, PCR raises problems in terms of time and cost. Currently, in order to render ensured diagnosis of FH, these approaches, which can be employed only in limited areas, are the only means to be employed.

As described above, from the viewpoint of prevention of coronary diseases and arteriosclerosis, FH must be detected as early as possible, before the typical clinical findings of FH have become clear in the patient. Thus, provision of a widely-applicable technique that enables an established diagnosis of FH would be considered a great contribution to early detection of FH.

Accordingly, an object of the present invention is to provide useful means for rendering an established diagnosis of FH in broader clinical situations.

Disclosure of the Invention

The present inventors have noted that familial hypercholesterolemia, known to present elevated blood LDL level, is primarily caused by abnormality occurring in the LDL receptor gene; a blueprint of LDL receptor protein expressed on the cell membrane of hepatocytes or peripheral cells so as to intake LDL. Such abnormality produces an LDL receptor which is abnormal in quality; i.e., incapability to bind blood LDL, or in quantity; i.e., incapability to express sufficient amount of blood LDL receptor protein on the membrane. The inventors have also noted that the causes bringing about these qualitative and quantitative abnormalities reside in abnormalities in the LDL receptor gene itself, and have continued careful studies. As a result, the present inventors have found that the above-mentioned object of the invention can be attained through identification of the mentioned abnormalities responsible for Japanese FH, along with provision of a method for detecting relevant abnormal genes.

Accordingly, the present invention provides a method of detecting abnormality of lipid metabolism (hereinafter may be referred to as the present abnormality detection method), in which a risk factor concerning abnormality of lipid metabolism is detected through correlating one or more gene mutations selected from the group consisting of the below-described 1 through 65 gene mutations with abnormality in lipid metabolism. Moreover, the present invention provides a method of detecting a disease, by detecting a risk factor for an arteriosclerosis and/or ischemic heart disease employing, as an index, abnormality of lipid metabolism detected through the present abnormality detection method (hereinafter may be referred to as the present disease detection method, and the present abnormality detection method and the present disease detection method may be collectively referred to as the present detection method):

1. a mutation of a low-density lipoprotein receptor gene coding for low-density lipoprotein receptor protein, the mutation occurring at a site coding for amino acid residue 25, cysteine, of the low-density lipoprotein receptor protein;
2. a deletion mutation occurring in nucleotides 156 to 160 of the low-density lipoprotein receptor gene;
3. a mutation of the low-density lipoprotein receptor gene occurring at a site coding for amino acid residue 50, glycine, of the low-density lipoprotein receptor protein encoded by the gene;
4. a mutation of the low-density lipoprotein receptor gene occurring at a site coding for amino acid residue 74, cysteine, of the low-density lipoprotein receptor protein encoded by the gene;
5. a deletion mutation occurring in nucleotide 314, cytosine, of the low-density lipoprotein receptor gene;
6. a mutation of the low-density lipoprotein receptor gene occurring at a site coding for amino acid residue 81, glutamine, of the low-density lipoprotein receptor protein encoded by the gene;
7. a mutation of the low-density lipoprotein receptor gene occurring at a site coding for amino acid residue 88, cysteine, of the low-density lipoprotein receptor protein encoded by the gene;
8. a mutation of the low-density lipoprotein receptor gene occurring at a site coding for amino acid residue 90, glutamine, of the low-density lipoprotein receptor protein encoded by the gene;
9. a mutation of the low-density lipoprotein receptor gene occurring at a site coding for amino acid residue 94, arginine, of the low-density lipoprotein receptor protein encoded by the gene;
10. a deletion mutation occurring in nucleotides 355 to 361, of the low-density lipoprotein receptor gene;
11. a mutation of the low-density lipoprotein receptor gene occurring at a site coding for amino acid residue 100, cysteine, of the low-density lipoprotein receptor protein encoded by the gene;
12. a deletion mutation occurring in nucleotides 382 and 383, of the low-density lipoprotein receptor gene;
13. an insertion mutation occurring at a position of nucleotide 390 of the low-density lipoprotein receptor gene;
14. a mutation of the low-density lipoprotein receptor gene occurring at a site coding for amino acid residue 113, cysteine, of the low-density lipoprotein receptor protein encoded by the gene;
15. a mutation of the low-density lipoprotein receptor gene occurring at a site coding for amino acid residue 115, aspartic acid, of the low-density lipoprotein receptor protein encoded by the gene;
16. a mutation of the low-density lipoprotein receptor gene occurring at a site coding for amino acid residue 119, glutamic acid, of the low-density lipoprotein receptor protein encoded by the gene;
17. a deletion mutation occurring in nucleotides 578 to 584, of the low-density lipoprotein receptor gene;
18. an insertion mutation occurring at a position of nucleotide 682 of the low-density lipoprotein receptor gene;
19. a deletion mutation occurring in nucleotides 526 to 529, of the low-density lipoprotein receptor gene;
20. an insertion mutation occurring at a position of nucleotide 661 of the low-density lipoprotein receptor gene;
21. a mutation of the low-density lipoprotein receptor gene occurring at a site coding for amino acid residue 207, glutamic acid, of the low-density lipoprotein receptor protein encoded by the gene;
22. an insertion mutation occurring at a position of nucleotide 944 of the low-density lipoprotein receptor gene;
23. a deletion mutation occurring in nucleotide 948, cytosine, of the low-density lipoprotein receptor gene;
24. a mutation of the low-density lipoprotein receptor gene occurring at a site coding for amino acid residue 317, cysteine, of the low-density lipoprotein receptor protein encoded by the gene;

25. a deletion mutation occurring in nucleotides 1114 to 1134, of the low-density lipoprotein receptor gene;
26. an insertion mutation occurring at a position of nucleotide 1062 of the low-density lipoprotein receptor gene;
27. a mutation of the low-density lipoprotein receptor gene occurring at a site coding for amino acid residue 336, glutamic acid, of the low-density lipoprotein receptor protein encoded by the gene;
28. a mutation of the low-density lipoprotein receptor gene occurring at a site coding for amino acid residue 337, cysteine, of the low-density lipoprotein receptor protein encoded by the gene;
29. a mutation of the low-density lipoprotein receptor gene occurring at a site coding for amino acid residue 356, cysteine, of the low-density lipoprotein receptor protein encoded by the gene;
30. a mutation of the low-density lipoprotein receptor gene occurring at a site coding for amino acid residues 351 to 354, glutamic acid - glycine - glycine - tyrosine, of the low-density lipoprotein receptor protein encoded by the gene;
31. a mutation of the low-density lipoprotein receptor gene occurring at a site coding for amino acid residue 358, cysteine, of the low-density lipoprotein receptor protein encoded by the gene;
32. a mutation occurring at nucleotide -10 (guanine) in the 5'-end-side acceptor region in intron 8 of the low-density lipoprotein receptor gene;
33. a mutation of the low-density lipoprotein receptor gene occurring at a site coding for amino acid residue 382, phenylalanine, of the low-density lipoprotein receptor protein encoded by the gene;
34. a mutation occurring in nucleotide 1599, of the low-density lipoprotein receptor gene;
35. a deletion mutation occurring in nucleotides 1202 to 1204, of the low-density lipoprotein receptor gene;
36. a mutation of the low-density lipoprotein receptor gene occurring at a site coding for amino acid residue 385, arginine, of the low-density lipoprotein receptor protein encoded by the gene;
37. a mutation of the low-density lipoprotein receptor gene occurring at a site coding for amino acid residue 387, glutamic acid, of the low-density lipoprotein receptor protein encoded by the gene;
38. an insertion mutation occurring at a position of nucleotide 1242 of the low-density lipoprotein receptor gene;
39. a mutation of the low-density lipoprotein receptor gene occurring at a site coding for amino acid residue 401, leucine, of the low-density lipoprotein receptor protein encoded by the gene;
40. a mutation of the low-density lipoprotein receptor gene occurring at a site coding for amino acid residue 410, alanine, of the low-density lipoprotein receptor protein encoded by the gene;
41. a mutation of the low-density lipoprotein receptor gene occurring at a site coding for amino acid residue 412, aspartic acid, of the low-density lipoprotein receptor protein encoded by the gene;
42. a mutation of the low-density lipoprotein receptor gene occurring at a site coding for amino acid residue 512, tryptophan, of the low-density lipoprotein receptor protein encoded by the gene;
43. a deletion mutation occurring in nucleotides 1652 to 1662, of the low-density lipoprotein receptor gene;
44. a deletion mutation occurring in nucleotide 1655, thymine, of the low-density lipoprotein receptor gene;
45. an insertion mutation occurring at a position of nucleotide 1687 of the low-density lipoprotein receptor gene;
46. a mutation of the low-density lipoprotein receptor gene occurring at a site coding for amino acid residue 547, leucine, of the low-density lipoprotein receptor protein encoded by the gene;
47. a mutation from guanine to another base, occurring at nucleotide +1 (guanine) in a splice donor site of intron 11 starting from the nucleotide that is one base downstream the nucleotide 1705 of the low-density lipoprotein receptor protein gene;
48. a mutation from thymine to another base, occurring at nucleotide +2 (thymine) in a splice donor site of intron 12 starting from the nucleotide that is one base downstream the nucleotide 1845 of the low-density lipoprotein receptor protein gene;
49. a mutation of the low-density lipoprotein receptor gene occurring at a site coding for amino acid residue 556, tryptophan, of the low-density lipoprotein receptor protein encoded by the gene;
50. a mutation of the low-density lipoprotein receptor gene occurring at a site coding for amino acid residue 570, asparagine, of the low-density lipoprotein receptor protein encoded by the gene;
51. an insertion mutation occurring at a position of nucleotide 1779 of the low-density lipoprotein receptor gene;
52. a mutation of the low-density lipoprotein receptor gene occurring at a site coding for amino acid residue 587, proline, of the low-density lipoprotein receptor protein encoded by the gene;
53. a mutation of the low-density lipoprotein receptor gene occurring at a site coding for amino acid residue 591, alanine, of the low-density lipoprotein receptor protein encoded by the gene;
54. a deletion mutation occurring in nucleotides 1870 to 1872, of the low-density lipoprotein receptor gene;
55. a mutation of the low-density lipoprotein receptor gene occurring at a site coding for amino acid residue 612, arginine, of the low-density lipoprotein receptor protein encoded by the gene;
56. a deletion mutation occurring in nucleotide 1963, of the low-density lipoprotein receptor gene;
57. an insertion mutation occurring at a position of nucleotide 2035 of the low-density lipoprotein receptor gene;
58. a mutation of the low-density lipoprotein receptor gene occurring at a site coding for amino acid residue 664, proline, of the low-density lipoprotein receptor protein encoded by the gene;

59. a mutation of the low-density lipoprotein receptor gene occurring at a site coding for amino acid residue 693, glutamic acid, of the low-density lipoprotein receptor protein encoded by the gene;
60. a deletion mutation occurring in nucleotides 2320 to 2340, of the low-density lipoprotein receptor gene;
61. a mutation of the low-density lipoprotein receptor gene occurring at a site coding for amino acid residue 779, valine, of the low-density lipoprotein receptor protein encoded by the gene;
62. a mutation of the low-density lipoprotein receptor gene occurring at a site coding for amino acid residue 790, lysine, of the low-density lipoprotein receptor protein encoded by the gene;
63. an insertion mutation occurring at a position of nucleotide 2412 of the low-density lipoprotein receptor gene;
64. a mutation of the low-density lipoprotein receptor gene occurring at a site coding for amino acid residue 829, alanine, of the low-density lipoprotein receptor protein encoded by the gene; and
65. a mutation of the low-density lipoprotein receptor gene occurring at a site coding for amino acid residue 316, glutamic acid, of the low-density lipoprotein receptor protein encoded by the gene.

As used herein, amino acid residues are expressed on the basis of the three-letter code system or the one-letter code system. For the sake of convenience, the following may be referred to: alanine [Ala (according to the three-letter code system, the same applies hereunder), A (according to the one-letter code system, the same applies hereunder)], valine [Val, V], leucine [Leu, L], isoleucine [Ile, I], proline [Pro, P], phenylalanine [Phe, F] tryptophan [Trp, W], methionine [Met, M], glycine [Gly, G], serine [Ser, S], threonine [Thr, T], cysteine [Cys, C], glutamine [Gln, Q], asparagine [Asn, N], tyrosine [Tyr, Y], lysine [Lys, K], arginine [Arg, R], histidine [His, H], aspartic acid [Asp, D], glutamic acid [Glu, E].
As used herein, "A100V," for example, refers to a mutation of the 100th amino acid residue, alanine, in the sequence of native amino acid, such that the residue has been substituted by valine.

Brief Description of the Drawings

Fig. 1 shows electrophoresis patterns showing abnormalities in exons 2, 3, 4, and 7 of the LDL receptor gene;
Fig. 2 shows electrophoresis patterns showing abnormalities in exons 8, 9, 11, and 12 of the LDL-R gene;
Fig. 3 shows electrophoresis patterns showing abnormalities in exons 13, 14, 16, 17, and 18 of the LDL-R gene;
Fig. 4 shows a mutation of the LDL-R gene occurring at a site coding for amino acid residue 25, cysteine, of the LDL-R protein encoded by the gene;
Fig. 5 shows a deletion mutation occurring in nucleotides 156 to 160 of the LDL-R gene;
Fig. 6 shows a mutation of the LDL-R gene occurring at a site coding for amino acid residue 50, glycine, of the LDL-R protein encoded by the gene;
Fig. 7 shows a mutation of the LDL-R gene occurring at a site coding for amino acid residue 74, cysteine, of the LDL-R protein encoded by the gene;
Fig. 8 shows another mutation of the LDL-R gene occurring at a site coding for amino acid residue 74; cysteine, of the LDL-R protein encoded by the gene.
Fig. 9 shows a deletion mutation occurring in nucleotide 314, cytosine, of the LDL-R gene;
Fig. 10 shows a mutation of the LDL-R gene occurring at a site coding for amino acid residue 81, glutamine, of the LDL-R protein encoded by the gene;
Fig. 11 shows a mutation of the LDL-R gene occurring at a site coding for amino acid residue 88, cysteine, of the LDL-R protein encoded by the gene;
Fig. 12 shows a mutation of the LDL-R gene occurring at a site coding for amino acid residue 90, glutamine, of the LDL-R protein encoded by the gene;
Fig. 13 shows a mutation of the LDL-R gene occurring at a site coding for amino acid residue 94, arginine, of the LDL-R protein encoded by the gene;
Fig. 14 shows a deletion mutation occurring in nucleotides 355 to 361, of the LDL-R gene;
Fig. 15 shows a mutation of the LDL-R gene occurring at a site coding for amino acid residue 100, cysteine, of the LDL-R protein encoded by the gene;
Fig. 16 shows another mutation of the LDL-R gene occurring at a site coding for amino acid residue 100, cysteine, of the LDL-R protein encoded by the gene;
Fig. 17 shows a deletion mutation occurring in nucleotides 382 and 383, of the LDL-R gene;
Fig. 18 shows an insertion mutation occurring at a position of nucleotide 390 of the LDL-R gene;
Fig. 19 shows a mutation of the LDL-R gene occurring at a site coding for amino acid residue 113, cysteine, of the LDL-R protein encoded by the gene;
Fig. 20 shows another mutation of the LDL-R gene occurring at a site coding for amino acid residue 113, cysteine, of the LDL-R protein encoded by the gene;
Fig. 21 shows a mutation of the LDL-R gene occurring at a site coding for amino acid residue 115, aspartic acid, of the LDL-R protein encoded by the gene;
Fig. 22 shows a deletion mutation occurring in nucleotides 578 to 584, of the LDL-R gene;
Fig. 23 shows an insertion mutation occurring at a position of nucleotide 682 of the LDL-R gene;
Fig. 24 shows a deletion mutation occurring in nucleotides 526 to 529, of the LDL-R gene;
Fig. 25 shows an insertion mutation occurring at a position of nucleotide 661 of the LDL-R gene;
Fig. 26 shows a mutation of the LDL-R gene occurring at a site coding for amino acid residue 207, glutamic acid, of the LDL-R protein encoded by the gene;
Fig. 27 shows another mutation of the LDL-R gene occurring at a site coding for amino acid residue 207, glutamic acid, of the LDL-R protein encoded by the gene;
Fig. 28 shows an insertion mutation occurring at a position of nucleotide 944 of the LDL-R gene;
Fig. 29 shows a deletion mutation occurring in nucleotide 948, cytosine, of the LDL-R gene;
Fig. 30 shows a mutation of the LDL-R gene occurring at a site coding for amino acid residue 317, cysteine, of the LDL-R protein encoded by the gene;
Fig. 31 shows another mutation of the LDL-R gene occurring at a site coding for amino acid residue 317, cysteine, of the LDL-R protein encoded by the gene;
Fig. 32 shows a deletion mutation occurring in nucleotides 1114 to 1134, of the LDL-R gene;
Fig. 33 shows an insertion mutation occurring at a position of nucleotide 1062 of the LDL-R gene;
Fig. 34 shows a mutation of the LDL-R gene occurring at a site coding for amino acid residue 336, glutamic acid, of the LDL-R protein encoded by the gene;
Fig. 35 shows a mutation of the LDL-R gene occurring at a site coding for amino acid residue 337, cysteine, of the LDL-R protein encoded by the gene;
Fig. 36 shows a mutation of the LDL-R gene occurring at a site coding for amino acid residue 356, cysteine, of the LDL-R protein encoded by the gene;
Fig. 37 shows a mutation of the LDL-R gene occurring at a site coding for amino acid residues 351 to 354 of the LDL-R protein encoded by the gene;
Fig. 38 shows a mutation of the LDL-R gene occurring at a site coding for amino acid residue 358, cysteine, of the LDL-R protein encoded by the gene;
Fig. 39 shows a mutation occurring at nucleotide -10 (guanine) in the 5'-end-side acceptor region in intron 8 of the LDL-R gene;
Fig. 40 shows a mutation of the LDL-R gene occurring at a site coding for amino acid residue 382, phenylalanine, of the LDL-R protein encoded by the gene;
Fig. 41 shows a deletion mutation occurring in nucleotides 1202 to 1204, of the LDL-R gene;
Fig. 42 shows a mutation of the LDL-R gene occurring at a site coding for amino acid residue 385, arginine, of the LDL-R protein encoded by the gene;
Fig. 43 shows a mutation of the LDL-R gene occurring at a site coding for amino acid residue 387, glutamic acid, of the LDL-R protein encoded by the gene;
Fig. 44 shows an insertion mutation occurring at a position of nucleotide 1242 of the LDL-R gene;
Fig. 45 shows a mutation of the LDL-R gene occurring at a site coding for amino acid residue 401, leucine, of the LDL-R protein encoded by the gene;
Fig. 46 shows a mutation of the LDL-R gene occurring at a site coding for amino acid residue 410, alanine, of the LDL-R protein encoded by the gene;
Fig. 47 shows a mutation of the LDL-R gene occurring at a site coding for amino acid residue 412, aspartic acid, of the LDL-R protein encoded by the gene;
Fig. 48 shows a mutation of the LDL-R gene occurring at a site coding for amino acid residue 512, tryptophan, of the LDL-R protein encoded by the gene;
Fig. 49 shows a deletion mutation occurring in nucleotide 1599, of the LDL-R gene;
Fig. 50 shows an insertion mutation occurring at a position of nucleotide 1687 of the LDL-R gene;
Fig. 51 shows a deletion mutation occurring in nucleotides 1652 to 1662, of the LDL-R gene;
Fig. 52 shows a deletion mutation occurring in nucleotide 1655, thymine, of the LDL-R gene;
Fig. 53 shows a mutation of the LDL-R gene occurring at a site coding for amino acid residue 547, leucine, of the LDL-R protein encoded by the gene;
Fig. 54 shows a mutation from guanine to another base, occurring at nucleotide +1 (guanine) in a splice donor site of intron 11 starting from the nucleotide that is one base downstream the nucleotide 1705 of the LDL-R gene;
Fig. 55 shows another mutation from guanine to another base, occurring at nucleotide +1 (guanine) in a splice donor site of intron 11 starting from the nucleotide that is one base downstream the nucleotide 1705 of the LDL-R gene;

Fig. 56 shows a mutation of the LDL-R gene occurring at a site coding for amino acid residue 570, asparagine, of the LDL-R protein encoded by the gene;
Fig. 57 shows an insertion mutation occurring at a position of nucleotide 1779 of the LDL-R gene;
Fig. 58 shows a mutation of the LDL-R gene occurring at a site coding for amino acid residue 587, proline, of the LDL-R protein encoded by the gene;
Fig. 59 shows a mutation of the LDL-R gene occurring at a site coding for amino acid residue 591, alanine, of the LDL-R protein encoded by the gene;
Fig. 60 shows a deletion mutation occurring in nucleotides 1870 to 1872, of the LDL-R gene;
Fig. 61 shows a mutation of the LDL-R gene occurring at a site coding for amino acid residue 612, arginine, of the LDL-R protein encoded by the gene;
Fig. 62 shows a deletion mutation occurring in nucleotide 1963, of the LDL-R gene;
Fig. 63 shows an insertion mutation occurring at a position of nucleotide 2035 of the LDL-R gene;
Fig. 64 shows a mutation of the LDL-R gene occurring at a site coding for amino acid residue 693, glutamic acid, of the LDL-R protein encoded by the gene;
Fig. 65 shows a deletion mutation occurring in nucleotides 2320 to 2340, of the LDL-R gene;
Fig. 66 shows a mutation of the LDL-R gene occurring at a site coding for amino acid residue 779, valine, of the LDL-R protein encoded by the gene;
Fig. 67 shows a mutation of the LDL-R gene occurring at a site coding for amino acid residue 790, lysine, of the LDL-R protein encoded by the gene;
Fig. 68 shows an insertion mutation occurring at a position of nucleotide 2412 of the LDL-R gene;
Fig. 69 shows a mutation of the LDL-R gene occurring at a site coding for amino acid residue 316, glutamic acid, of the LDL-R protein encoded by the gene;
Fig. 70 shows a genetic polymorphism of the LDL-R gene in which nucleotide 81, T, is substituted by C;
Fig. 71 shows a genetic polymorphism of the LDL-R gene in which nucleotide 636, C, is substituted by T;
Fig. 72 shows a genetic polymorphism of the LDL-R gene in which nucleotide 969, C, is substituted by T;
Fig. 73 shows a genetic polymorphism of the LDL-R gene in which nucleotide 1002, C, is substituted by T;
Fig. 74 shows a genetic polymorphism of the LDL-R gene in which nucleotide 1195, C, is substituted by T;
Fig. 75 shows a genetic polymorphism of the LDL-R gene in which nucleotide 1725, C, is substituted by T;
Fig. 76 shows a genetic polymorphism of the LDL-R gene in which nucleotide 1773, T, is substituted by C; and
Fig. 77 shows a genetic polymorphism of the LDL-R gene in which nucleotide 1959, C, is substituted by T.

Best Modes for Carrying Out the Invention

Modes of the present invention will be described hereafter.

As mentioned above, the present invention is directed to a method of detecting arteriosclerosis and/or ischemic heart disease through detection of a risk factor for abnormality of lipid metabolism, and the underlying concept of the present invention resides in detection of abnormalities in the low-density lipoprotein receptor (hereinafter may be referred to as LDL-R) gene, which is an arteriosclerosis-associated gene.

Analyses of the LDL-R gene and the LDL-R protein encoded by the LDL-R gene have already been performed (Yamamoto T, *et al*., Cell 39, 27-38, 1984). The LDL-R nucleotide sequence and its corresponding amino acid sequence are shown in SEQ ID NO: 1.

Detailed analyses of relationships between mutations of the LDL-R gene and pathological phenomena—such as arteriosclerosis and ischemic heart diseases—that are considered to be attributable to abnormalities occurring in LDL-R will identify mutations of LDL-R gene useful in the practice of the detection method of the present invention. For example, gene mutations of interest can be identified if analysis of mutation sites or mutation frequency of the LDL-R gene or analysis of functions of the protein bearing the mutation is performed on combinations of "patients suffering arteriosclerosis or ischemic heart disease" and "healthy subjects." Specific procedures of such analyses will be described in the "Examples" section hereinbelow.

In the context of the present invention, "gene mutation" means alteration occurring in a gene in the human chromosome, and more specifically means that the nucleotide sequence of the gene differs from that of the wild-type gene (the nucleotide sequence of a normal gene). When specific sites of a gene in its nucleotide sequence differ from one another in an individual-dependent manner, such events can generally be referred to as "genetic polymorphism." According to the present invention, such genetic polymorphism also falls within the meaning of the "gene mutation." The "gene mutation" can be identified through analyses, by employment of various approaches, of mutation frequency of the gene, expression level of mRNA, expression level of protein, functions of the protein, etc. Such gene mutation has been considered to occur at a frequency of about 1 in several hundreds of bases on average, and can be identified through direct or indirect analysis of a gene. Familial analysis regarding the gene mutation thus identified will determine whether a chromosome (an allele) is inherited from the paternal side or from the maternal side.

The alteration occurring in a mutation site of a gene is inherited from either the paternal side or the maternal side. When a base in the mutation site has undergone substitution to thereby manifest substitution of both alleles by different bases as compared with the case of the wild-type gene, such a case is referred to as a "homozygote," whereas when the nucleotide sequence of one allele differs from that of the wild-type gene, such a case is referred to as a "heterozygote."

As described above, the present inventors have so far identified the following genetic abnormalities which are found in the LDL-R gene and are correlated with risk factors concerning arteriosclerosis:

1. a mutation of an LDL-R gene coding for LDL-R protein, the mutation occurring at a site coding for amino acid residue 25, cysteine, of the LDL-R protein (for example, in the LDL-R gene, nucleotide 137, guanine, is substituted by cytosine, and the mentioned cysteine is changed to serine);
2. a deletion mutation occurring in nucleotides 156-160 of the LDL-R gene;
3. a mutation of the LDL-R gene occurring at a site coding for amino acid residue 50, glycine, of the LDL-R protein encoded by the gene (for example, in the LDL-R gene, nucleotide 211, guanine, is substituted by adenine, and the mentioned glycine is changed to arginine);
4. a mutation of the LDL-R gene occurring at a site coding for amino acid residue 74, cysteine, of the LDL-R protein encoded by the gene (for example, (1) in the LDL-R gene, nucleotide 283, thymine, is substituted by adenine, and the mentioned cysteine is changed to serine, and (2) in the LDL-R gene, nucleotide 285, cytosine, is substituted by adenine, with the site coding for cysteine having been changed into a stop codon);
5. a deletion mutation occurring in nucleotide 314, cytosine, of the LDL-R gene;
6. a mutation of the LDL-R gene occurring at a site coding for amino acid residue 81, glutamine, of the LDL-R protein encoded by the gene (for example, in the LDL-R gene, nucleotide 304, cytosine, is substituted by thymine, with the site coding for glutamine having been changed into a stop codon);
7. a mutation of the LDL-R gene occurring at a site coding for amino acid residue 88, cysteine, of the LDL-R protein encoded by the gene (for example, in the LDL-R gene, nucleotide 326, guanine, is substituted by cytosine, and the mentioned cysteine is changed to serine);
8. a mutation of the LDL-R gene occurring at a site coding for amino acid residue 90, glutamine, of the LDL-R protein encoded by the gene (for example, in the LDL-R gene, nucleotide 331, cytosine, is substituted by thymine, with the site coding for glutamine having been changed into a stop codon);
9. a mutation of the LDL-R gene occurring at a site coding for amino acid residue 94, arginine, of the LDL-R protein encoded by the gene (for example, in the LDL-R gene, nucleotide 344, guanine, is substituted by adenine, and the mentioned arginine is changed to histidine);
10. a deletion mutation occurring in nucleotides 355-361, of the LDL-R gene;
11. a mutation of the LDL-R gene occurring at a site coding for amino acid residue 100, cysteine, of the LDL-R protein encoded by the gene (for example, (1) in the LDL-R gene, nucleotide 361, thymine, is substituted by guanine, and the mentioned cysteine is changed to glycine, and (2) in the LDL-R gene, nucleotide 363, cytosine, is substituted by adenine, with the site coding for cysteine having been changed into a stop codon);
12. a deletion mutation occurring in nucleotides 382-383, of the LDL-R gene;
13. an insertion mutation occurring at a position of nucleotide 390 of the LDL-R gene;
14. a mutation of the LDL-R gene occurring at a site coding for amino acid residue 113, cysteine, of the LDL-R protein encoded by the gene (for example, (1) in the LDL-R gene, nucleotide 401, guanine, is substituted by thymine, and the mentioned cysteine is changed to phenylalanine, and (2) in the LDL-R gene, nucleotide 400, thymine, is substituted by cytosine, and the mentioned cysteine is changed to arginine);
15. a mutation of the LDL-R gene occurring at a site coding for amino acid residue 115, aspartic acid, of the LDL-R protein encoded by the gene (for example, in the LDL-R gene, nucleotide 406, guanine, is substituted by adenine, and the mentioned aspartic acid is changed to asparagine);
16. a mutation of the LDL-R gene occurring at a site coding for amino acid residue 119, glutamic acid, of the LDL-R protein encoded by the gene (for example, in the LDL-R gene, nucleotide 418, guanine, is substituted by adenine, and the mentioned glutamic acid is changed to lysine);
17. a deletion mutation occurring in nucleotides 578-584, of the LDL-R gene;
18. an insertion mutation occurring at a position of nucleotide 682 of the LDL-R gene;
19. a deletion mutation occurring in nucleotides 526-529, of the LDL-R gene;
20. an insertion mutation occurring at a position of nucleotide 661 of the LDL-R gene;
21. a mutation of the LDL-R gene occurring at a site coding for amino acid residue 207, glutamic acid, of the LDL-R protein encoded by the gene (for example, (1) in the LDL-R gene, nucleotide 862, guanine, is substituted by adenine, and the mentioned glutamic acid is changed to lysine, and (2) in the LDL-R gene, nucleotide 682, guanine, is substituted by cytosine, and the mentioned glutamic acid is changed to glutamine);
22. an insertion mutation occurring at a position of nucleotide 944 of the LDL-R gene;

23. a deletion mutation occurring in nucleotide 948, cytosine, of the LDL-R gene;
24. a mutation of the LDL-R gene occurring at a site coding for amino acid residue 317, cysteine, of the LDL-R protein encoded by the gene (for example, (1) in the LDL-R gene, nucleotide 1012, thymine, is substituted by adenine, and the mentioned cysteine is changed to serine, and (2) in the LDL-R gene, nucleotide 1012, thymine, is substituted by cytosine, and the mentioned cysteine is changed to arginine);
25. a deletion mutation occurring in nucleotides 1114-1134, of the LDL-R gene;
26. an insertion mutation occurring at a position of nucleotide 1062 of the LDL-R gene;
27. a mutation of the LDL-R gene occurring at a site coding for amino acid residue 336, glutamic acid, of the LDL-R protein encoded by the gene (in the LDL-R gene, nucleotide 1069, guanine, is substituted by thymine, with the site coding for glutamic acid having been changed into a stop codon);
28. a mutation of the LDL-R gene occurring at a site coding for amino acid residue 337, cysteine, of the LDL-R protein encoded by the gene (for example, in the LDL-R gene, nucleotide 1072, thymine, is substituted by cytosine, and the mentioned cysteine is changed to arginine);
29. a mutation of the LDL-R gene occurring at a site coding for amino acid residue 356, cysteine, of the LDL-R protein encoded by the gene (for example, in the LDL-R gene, nucleotide 1130, guanine, is substituted by adenine, and the mentioned cysteine is changed to tyrosine);
30. a mutation of the LDL-R gene occurring at a site coding for amino acid residues 351-354, glutamic acid - glycine - glycine - tyrosine, of the LDL-R protein encoded by the gene;
31. a mutation of the LDL-R gene occurring at a site coding for amino acid residue 358, cysteine, of the LDL-R protein encoded by the gene (for example, in the LDL-R gene, nucleotide 1136, guanine, is substituted by adenine, and the mentioned cysteine is changed to tyrosine);
32. a mutation occurring at nucleotide -10 (guanine) in the 5'-end-side acceptor region in intron 8 of the LDL-R gene;
33. a mutation of the LDL-R gene occurring at a site coding for amino acid residue 382, phenylalanine, of the LDL-R protein encoded by the gene (for example, in the LDL-R gene, nucleotide 1207, thymine, is substituted by cytosine, and the mentioned phenylalanine is changed to leucine);
34. a mutation occurring in nucleotide 1599, of the LDL-R gene;
35. a deletion mutation occurring in nucleotides 1202-1204, of the LDL-R gene;
36. a mutation of the LDL-R gene occurring at a site coding for amino acid residue 385, arginine, of the LDL-R protein encoded by the gene (for example, in the LDL-R gene, nucleotide 1216, cytosine, is substituted by thymine, and the mentioned arginine is changed to tryptophan);
37. a mutation of the LDL-R gene occurring at a site coding for amino acid residue 387, glutamic acid, of the LDL-R protein encoded by the gene (for example, in the LDL-R gene, nucleotide 1222, guanine, is substituted by adenine, and the mentioned glutamic acid is changed to lysine);
38. an insertion mutation occurring at a position of nucleotide 1242 of the LDL-R gene;
39. a mutation of the LDL-R gene occurring at a site coding for amino acid residue 401, leucine, of the LDL-R protein encoded by the gene (for example, in the LDL-R gene, nucleotide 1265, thymine, is substituted by guanine, and the mentioned leucine is changed to arginine);
40. a mutation of the LDL-R gene occurring at a site coding for amino acid residue 410, alanine, of the LDL-R protein encoded by the gene (for example, in the LDL-R gene, nucleotide 1291, guanine, is substituted by adenine, and the mentioned alanine is changed to threonine);
41. a mutation of the LDL-R gene occurring at a site coding for amino acid residue 412, aspartic acid, of the LDL-R protein encoded by the gene (for example, in the LDL-R gene, nucleotide 1297, guanine, is substituted by cytosine, and the mentioned aspartic acid is changed to histidine);
42. a mutation of the LDL-R gene occurring at a site coding for amino acid residue 512, tryptophan, of the LDL-R protein encoded by the gene (for example, in the LDL-R gene, nucleotide 1599, guanine, is substituted by adenine, and the mentioned site coding for tryptophan is changed to a stop codon);
43. a deletion mutation occurring in nucleotides 1652-1662, of the LDL-R gene;
44. a deletion mutation occurring in nucleotide 1655, thymine, of the LDL-R gene;
45. an insertion mutation occurring at a position of nucleotide 1687 of the LDL-R gene;
46. a mutation of the LDL-R gene occurring at a site coding for amino acid residue 547, leucine, of the LDL-R protein encoded by the gene (for example, in the LDL-R gene, nucleotide 1702, cytosine, is substituted by guanine, and the mentioned leucine is changed to valine);
47. a mutation occurring at nucleotide +1 (guanine) in a splice donor site of intron 11 starting from the nucleotide that is next to nucleotide 1705 of the LDL-R gene;
48. a mutation occurring at nucleotide +2 (thymine) in a splice donor site of intron 12 starting from the nucleotide that is next to nucleotide 1845 of the LDL-R gene;
49. a mutation of the LDL-R gene occurring at a site coding for amino acid residue 556, tryptophan, of the LDL-R protein encoded by the gene (for example, in the LDL-R gene, nucleotide 1731, guanine, is substituted by thymine, and the mentioned tryptophan is changed to cysteine);

50. a mutation of the LDL-R gene occurring at a site coding for amino acid residue 570, asparagine, of the LDL-R protein encoded by the gene (for example, in the LDL-R gene, nucleotide 1772, adenine, is substituted by guanine, and the mentioned asparagine is changed to serine);

51. an insertion mutation occurring at a position of nucleotide 1779 of the LDL-R gene;

52. a mutation of the LDL-R gene occurring at a site coding for amino acid residue 587, proline, of the LDL-R protein encoded by the gene (for example, in the LDL-R gene, nucleotide 1822, cytosine, is substituted by thymine, and the mentioned proline is changed to serine);

53. a mutation of the LDL-R gene occurring at a site coding for amino acid residue 591, alanine, of the LDL-R protein encoded by the gene (for example, in the LDL-R gene, nucleotide 1834, guanine, is substituted by thymine, and the mentioned alanine is changed to serine);

54. a deletion mutation occurring in nucleotides 1870-1872, of the LDL-R gene;

55. a mutation of the LDL-R gene occurring at a site coding for amino acid residue 612, arginine, of the LDL-R protein encoded by the gene (for example, in the LDL-R gene, nucleotide 1897, cytosine, is substituted by thymine, and the mentioned arginine is changed to cysteine);

56. a deletion mutation occurring in nucleotide 1963, of the LDL-R gene;

57. an insertion mutation occurring at a position of nucleotide 2035 of the LDL-R gene;

58. a mutation of the LDL-R gene occurring at a site coding for amino acid residue 664, proline, of the LDL-R protein encoded by the gene (for example, in the LDL-R gene, nucleotide 2054, cytosine, is substituted by thymine, and the mentioned proline acid is changed to leucine);

59. a mutation of the LDL-R gene occurring at a site coding for amino acid residue 693, glutamic acid, of the LDL-R protein encoded by the gene (for example, in the LDL-R gene, nucleotide 2140, guanine, is substituted by adenine, and the mentioned glutamic acid is changed to lysine);

60. a deletion mutation occurring in nucleotides 2320-2340, of the LDL-R gene;

61. a mutation of the LDL-R gene occurring at a site coding for amino acid residue 779, valine, of the LDL-R protein encoded by the gene (for example, in the LDL-R gene, nucleotide 2398, guanine, is substituted by adenine, and the mentioned valine is changed to methionine);

62. a mutation of the LDL-R gene occurring at a site coding for amino acid residue 790, lysine, of the LDL-R protein encoded by the gene (for example, in the LDL-R gene, nucleotide 2431, adenine, is substituted by thymine, and the mentioned site coding for lysine is changed to a stop codon);

63. an insertion mutation occurring at a position of nucleotide 2412 of the LDL-R gene;

64. a mutation of the LDL-R gene occurring at a site coding for amino acid residue 829, alanine, of the LDL-R protein encoded by the gene (for example, in the LDL-R gene, nucleotide 2579, cytosine, is substituted by thymine, and the mentioned alanine is changed to valine); and

65. a mutation of the LDL-R gene occurring at a site coding for amino acid residue 316, glutamic acid, of the LDL-R protein encoded by the gene (for example, in the LDL-R gene, nucleotide 1010, thymine, is substituted by cytosine, and the mentioned glutamic acid is changed to glycine).

Alterations occurring in abnormal sites of a gene can be detected through any suitable conventional method, such as RFLP employing Southern blotting; PCR-RFLP; HET (hetero duplex analysis); DGGE (denaturing gradient gel electrophoresis); DS (direct sequencing); CCM (chemical cleavage mismatching); CDI (carbodiimide modification); an analysis technique for single-stranded conformation polymorphism making use of PCR (PCR-SSCP; hereinafter may be referred to simply as the SSCP method), or PCR/GC-clamping (see, for example, "Biomanual Series 1, Basic Techniques of Genetic Engineering," edited by Miyabi Yamamoto, published by Yodo-sha (1993), and particularly regarding the PCR/GC-clamping, see, for example, "Genomic Analysis: A Practical Approach," Myers, R. M., Shefield, V., and Cox, D. R. (1988), K. Davies, ed. IRL Press Limited, Oxford, pp. 95-139). Of these, PCR/GC-clamping is preferred, in that it enables convenient and accurate identification of a genetic abnormality.

Specifically, PCR/GC-clamping is a modified version of DGGE (note: DGGE is a method of detecting base substitution of DNA on polyacrylamide gel containing a DNA denaturant at linear gradient concentrations, wherein the method makes use of the mobility difference stemming from difference between the concentration of a DNA denaturant required for denaturing DNA of a double-stranded DNA fragment containing a base substitution and the concentration of the DNA denaturant required for denaturing DNA of a double-stranded DNA fragment containing no base substitution), and according to the modified version, the drawback involved in DGGE experienced in the case of a plurality of base substitutions; i.e., disabled detection of the base substitution of the domain lastly fused on the polyacrylamide gel, is overcome by ligating a region of high GC content (GC-clamp) with a DNA fragment carrying the base substitutions to be detected (see, for example, Shefield, V. C. *et al.* (1989) Proc. Natl. Acad. Sci. USA 86: 232-236).

Therefore, although the basic procedure of the PCR/GC-clamping technique is analogous to DGGE, there must be performed an additional step for adding a GC-clamp to the DNA fragment for which base substitution is to be detected.

According to the present invention, no particular limitations are imposed on the source of DNA molecules for which alteration in a genetically abnormal site of the LDL-R is to be detected, so long as it constitutes somatic cells of a test subject (patient). Blood samples such as peripheral blood samples or leukocyte samples are preferably used in the present invention.

From sample cells collected from a test subject, genomic DNA is extracted by a known method, and with regard to the thus-obtained genomic DNA, alteration occurring in a certain locus of the gene (specifically, substitution of a base at a specific abnormality-carrying locus of the gene) is detected.

When the above detection procedure reveals the presence of alteration in the mentioned specific locus of the gene, the alteration is correlated with lipid metabolic disorder or further with arteriosclerosis and/or ischemic heart disease, to thereby detect a risk factor for arteriosclerosis and/or ischemic heart disease. The concept of "detection of a risk factor for arteriosclerosis and/or ischemic heart disease" encompasses not only detection of the arteriosclerosis and/or ischemic heart disease currently suffered by the patient, but also high possibility of future onset of arteriosclerosis and/or ischemic heart disease. In other words, even in the case where arteriosclerosis or ischemic heart disease is not currently identified, if alteration is observed in a specific locus of the LDL-R gene, LDL-R cannot fully play its intrinsic roles in, for example, metabolism of serum lipids, thereby increasing the possibility of onset of arteriosclerosis and/or ischemic heart disease. Thus, according to the present detection method, such high possibility indicative of future arteriosclerosis and/or ischemic heart disease can also be detected as a risk factor for arteriosclerosis and/or ischemic heart disease.

When LDL-R does not function properly, serum lipids often show elevated LDL-C levels. Generally speaking, LDL-C is acknowledged to be "bad cholesterol," and therefore, clinical institutions caution subjects when LDL-C level in serum lipids is found high. Early proper treatment is important particularly for FH patients, but, as described above, heterozygous FH patients rarely show prominently high LDL-C levels during childhood. Therefore, partially as a result of popularization of the westernized dietary style in recent years, discernment between FH from transient hyperlipemia attributed to changes in dietary style of nonhereditary nature is difficult. When the present detection method is used to test a subject who is suspected whether he has FH because of his relatively high LDL-C level in serum lipids, risk factors concerning oneset of hidden arteriosclerosis and/or ischemic heart disease can be properly detected.

When the present detection method reveals one or more of the above-recited specific alterations in the LDL-R gene from a patient, and in addition, the patient exhibits pathological conditions closely related to arteriosclerosis and/or ischemic heart disease, such as diabetes or hypertension, lethal events caused by future arteriosclerosis can be prevented by starting a combination treatment of conventional therapy for such pathological conditions and therapy for arteriosclerosis. Moreover, when the present detection method reveals one or more of the above-recited specific alterations in the LDL-R gene from a patient who is seemingly a healthy subject, provision of measures for preventing the onset of arteriosclerosis or ischemic heart disease, such as providing suggestions for, or administration of, improved diet and proper exercise, may reduce risk factors for the onset of arteriosclerosis and/or ischemic heart disease of the patient.

In practice of the present detection method, when specific abnormality or abnormalities occurring in one or more specific loci of the LDL-R gene as newly identified by the present invention are detected in combination with conventionally identified abnormality or abnormalities occurring in one or more specific loci of the LDL-R gene, it is possible to attain more accurate detection of risk factors for the onset of arteriosclerosis and/or ischemic heart disease.

Examples

The present invention will next be described in more detail by way of examples.

<u>Method for analysis of the LDL-R gene</u>

<Preparation of monocytes>

An ACD-added peripheral blood sample (10 mL) collected from a test subject is placed to form a layer in a SEPARATE L (5 mL, product of Wako Pure Chemical Industries, Ltd.), and then subjected to specific gravity centrifugation at 1,200 rpm for 60 minutes, to thereby separate monocytes. By use of an RPMI medium (10 mL, product of Lifetech Oriental, Inc.), the obtained monocytes are centrifuged twice at 1,500 rpm for 10 minutes, and washed. Subsequently, an RPMI medium supplemented with 1%-fatty-acid-depleted bovine serum albumin (BSA) is added, to thereby prepare a solution containing $5 \times 10^6$ monocytes/mL. The thus-prepared monocytes are incubated in an incubator (5% $CO_2$) at 37°C for three days. Thereafter, the monocytes are washed twice with PBS, and suspended in PBS containing 1-mM $CaCl_2$ (0.5 mL). An aliquot (0.1 mL) of the monocyte suspension is placed in a Microfuge tube (product of Eppendorf), to which a Dil-LDL (10 μg/mL, product of Molecular Probes) is added for reaction at 37°C for two hours.

After completion of reaction, the cells are washed twice with PBS, and finally suspended in PBS supplemented with 1% BSA (0.1 mL). A diluted anti-LDL-R antibody (IgG-C7) solution (10 µL) is added to another aliquot (0.1 mL) of the monocyte suspension for reaction at 4°C for 30 minutes. After completion of reaction, three washings are performed with ice-cold PBS containing 1% BSA, to thereby remove unreacted antibodies. After the washings, PE-labeled anti-mouse IgG antibodies are added for reaction at 4°C for 30 minutes. After completion of reaction, three washings are performed with ice-cold PBS containing 1% BSA, to thereby remove unreacted antibodies and obtain 500 µL of a cell suspension in PBS supplemented with 1% BSA.

<Measurement by means of a flow cytometer>

Dil-LDL taken in cells and antibodies which have been bound to LDL-R expressed on the cell membrane are detected through measuring fluorescence intensity of the cells by means of a flow cytometer (FACScan, product of Becton Dickinson). Briefly, the cells which have been reacted as described above are subjected to flow cytometry under the following conditions: laser power = 15 W, excitation wavelength = 488 nm, wavelength = 530 nm, PMT voltage = 500 mV. In the measurement, gating is set for the lymphocyte region based on forward scatter (FSC) and side scatter (SSC) parameters for measurement of the intensity of FL1. The fluorescence intensity of the cells is obtained by measuring the fluorescence intensity of ten thousands cells. The thus-obtained fluorescence intensity of the cells is analyzed by use of analysis software CELLQUEST installed in the computer connected to the flow cytometer.

<Quantitation of the protein amount and activity of LDL-R>

As described above, fluorescence intensities of the cells obtained from an individual are measured by flow cytometry, and from the results of the measurement, average fluorescence intensities are calculated. The average fluorescence intensities are considered to represent the protein amount and the activity of the LDL-R expressed in the cells. Specifically, an average fluorescence intensity of the cells obtained by use of antibodies is considered to represent the protein amount of the LDL-R expressed in the cells, and the fluorescence intensity of the cells incorporating the fluorescence dye Dil-LDL is considered to represent the LDL-R activity. Both the protein amount and the activity of LDL-R are expressed in terms of percent (%) with respect to the average fluorescence intensity of cells obtained from 2 to 4 healthy subjects whose serum lipid levels are normal. That is, the protein amount and the activity of LDL-R of an individual are calculated by the following equations.

"Amount of LDL-R protein of an individual" (%) =

{"fluorescence intensity as measured for the individual

obtained by use of antibodies" / "average fluorescence

intensity as measured for healthy subjects by use of

antibodies"} × 100

"Activity of LDL-R of an individual" (%) =

{"fluorescence intensity as measured for the individual

obtained by use of Dil-LDL" / "average fluorescence intensity

as measured for healthy subjects by use of Dil-LDL"} × 100

<PCR>

Genomic DNA of an individual is extracted from peripheral leukocytes by use of a DNA extraction kit (QIAamp DNA Blood kit, product of Qiagen). The nucleic acids in the promoter region and exon regions of exon 1 to exon 18 of LDL-R gene are amplified by use of the following oligonucleotides (see also SEQ ID NOs: 2-43):

promoter region: GAGTGGGAATCAGAGCTTCACGGGT (SEQ ID NO: 2)

CCACGTCATTTACAGCATTTCAATG (SEQ ID NO: 3)

exon 1: ACTCCTCCCCCTGCTAGAAACCTCA (SEQ ID NO: 4)

TTCTGGCGCTTGGAGCAAGCCTTAC (SEQ ID NO: 5)

exon 2: CCTTTCTCCTTTTCCTCTCTCTCAG (SEQ ID NO: 6)

AAAATAAATGCATATCATGCCCAAA (SEQ ID NO: 7)

exon 3: TGACAGTTCAATCCTGTCTCTTCTG (SEQ ID NO: 8)

ATAGCAAAGGCAGGGCCACACTTAC (SEQ ID NO: 9)

exon 4A: GTTGGGAGACTTCACACGGTGATGG (SEQ ID NO: 10)

ACTTAGGCAGTGGAACTCGAAGGCC (SEQ ID NO: 11)

exon 4B: CCCCAGCTGTGGGCCTGCGACAACG (SEQ ID NO: 12)

GGGGGAGCCCAGGGACAGGTGATAG (SEQ ID NO: 13)

exon 5: CAACACACTCTGTCCTGTTTTCCAG (SEQ ID NO: 14)

GGAAAAACCAGATGGCCAGCGCTCAC (SEQ ID NO: 15)

exon 6: TCCTTCCTCTCTCTGGCTCTCACAG (SEQ ID NO: 16)

GCAAGCCGCCTGCACCGAGACTCAC (SEQ ID NO: 17)

exon 7: AGTCTGACTCCCTGGCCCTGCGCAG (SEQ ID NO: 18)

AGGGCTCAGTCCACCGGGGAATCAC (SEQ ID NO: 19)

exon 8: CCAAGCCTCTTTCTCTCTCTTCCAG (SEQ ID NO: 20)

CCACCCGCCGCCTTCCCGTGCTCAC (SEQ ID NO: 21)

exon 9: TCCATCGACGGGTCCCCTCTGACCC (SEQ ID NO: 22)

AGCCCTCATCTCACCTGCGGGCCAA (SEQ ID NO: 23)

exon 10A: AGATGAGGGCTCCTGGTGCGATGCC (SEQ ID NO: 24)

GCCCTTGGTATCCGCAACAGAGACA (SEQ ID NO: 25)

exon 10B: GATCCACAGCAACATCTACTGGACC (SEQ ID NO: 26)

AGCCCTCAGCGTCGTGGATACGCAC (SEQ ID NO: 27)

exon 11: CAGCTATTCTCTGCTCTCCCACCAG (SEQ ID NO: 28)

TGGGACGGCTGTCCTCGCAACATAC (SEQ ID NO: 29)

exon 12: GCACGTGACCTCTCCTTATCCACTT (SEQ ID NO: 30)

CACCTAAGTGCTTCGATCTCGTACG (SEQ ID NO: 31)

exon 13: GTCATCTTCCTTGCTGCCTGTTTAG (SEQ ID NO: 32)

GTTTCCACAAGGAGGTTTCAAGGTT (SEQ ID NO: 33)

exon 14: CCTGACTCCGCTTCTTCTGCCCCAG (SEQ ID NO: 34)

CGCAGAAACAAGGCGTGTGCCACAC (SEQ ID NO: 35)

exon 15: GAAGGGCCTGCAGGCACGTGGCACT (SEQ ID NO: 36)

GTGTGGTGGCGGGCCCAGTCTTTAC (SEQ ID NO: 37)

exon 16: CCTCACTCTTGCTTCTCTCCTGCAG (SEQ ID NO: 38)

CGCTGGGGGACCGGCCCGCGCTTAC (SEQ ID NO: 39)

exon 17: TGACAGAGCGTGCCTCTCCCTACAG (SEQ ID NO: 40)

GCTTTCTAGAGAGGGTCACACTCAC (SEQ ID NO: 41)

exon 18: TCCGCTGTTTACCATTTGTTGGCAG (SEQ ID NO: 42)

AATAAAACAAGGCCGGCGAGGTCTC (SEQ ID NO: 43)

Mutations of the LDL-R gene can be analyzed through denaturing gradient gel electrophoresis (DGGE) on polyacrylamide gel. Briefly, PCR is performed by use of oligonucleotides prepared by adding a GC clamp of 40 bp to antisense primers in the following steps: mixing 0.5 µL DNA (0.5 g DNA) with 49 µL PCR reaction mixture (10mM Tris-HCl (pH 8.4), 50mM KCl, 0.2mM dNTP, each of the primers (50 pmol)) and 0.5 µL Taq DNA polymerase (2.5 unit, product of Roche); denaturing the mixture (95°C, 5 minutes); performing 25 cycles of treatment, each cycle consisting of denaturation (95°C, 30 seconds), annealing (65°C, 30 seconds), and elongation (72°C, 90 seconds); and elongating (72°C, 10 minutes). Then, the PCR product is electrophoresed by use of 3% agarose gel, and the resultant gel is stained with ethidium bromide. The electrophoresis bands are investigated by means of a UV trans-illuminator.

<Mutation analysis of the gene through denaturing gradient gel electrophoresis (DGGE) on polyacrylamide gel>

Mutations of the LDL-R gene are analyzed through a modified version of the denaturing gradient polyacrylamide gel electrophoresis described by Top, *et al.* (Top B., et al., Hum Genet, 91; 480-484, 1993). Briefly, each of the exons is amplified by use of the corresponding primer which has been added to a GC clamp, and the obtained PCR product is electrophoresed at 150 V for 16 hours by use of 9% polyacrylamide gel with a denaturing gradient (40 to 80%). After completion of electrophoresis, the gel is stained with ethidium bromide, and the obtained bands are investigated by means of a UV trans-illuminator.

As a result of the above-described DGGE, a PCR product carrying a mutation is detected as an abnormal band pattern. Specifically, in the case of a heterozygote in which one allele carries a mutation, a heteroduplex band and a homoduplex band are detected, whereas in the case of a homozygote in which both alleles carry mutations, a homoduplex band is detected at a locus different from that determined for the gene of a wild type.

<Determination of nucleotide sequence>

Each of the exons showing abnormal band patterns (i.e., a homoduplex band at a locus different from that determined for the gene of a wild type, or a heteroduplex band) as a result of the above-described DGGE is subjected to PCR direct sequencing, to thereby determine its nucleotide sequence. Briefly, the exon is again amplified through PCR, and the PCR product is electrophoresed on 3% agarose gel, and cut out from the gel. Subsequently, the thus-obtained PCR product of interest is purified by use of a QIAamp (product of Qiagen), and the purified PCR product is labeled with a fluorescent marker by use of a Big Dye Terminator Cycle Sequence Kit (product of Applied Biosystems), and nucleotide sequencing is performed by use of an ABI 377 DNA sequencer (product of PE Biosystems).

Clinical analysis

<The amount of serum lipid and LDL-R protein and LDL-R activity of patients suffering FH>

LDL-R of 73 patients (31 male, 42 female) who had been clinically diagnosed as suffering familial hyperlipemia (FH) was analyzed. The patients' data are as follows: age = 41.4 ± 14.7 years old (mean ± SD), serum cholesterol = 280.0 ± 65.9 mg/dL, neutral lipid = 102.7 ± 56.0 mg/dL, HDL cholesterol = 46.6 ± 18.5 mg/dL, amount of LDL-R protein = 54.7 ± 20.9%, LDL-R activity = 59.1 ± 14.1%.

Mutation analysis of LDL-R gene through DGGE

<Analysis of the LDL-R gene of patients suffering FH>

In the above-described mutation analysis through DGGE, the following exons were found to exhibit abnormal band patterns: 2 abnormal band patterns for exon 2, 3 for exon 3, 20 for exon 4, and 4 for exon 7 (details of these are shown in the electrophoresis patterns in Fig. 1); 7 for exon 8, 9 for exon 9, and 6 for exon 12 (details of these are shown in the electrophoresis patterns in Fig. 2); and 3 for exon 13, 3 for exon 14, 1 for exon 16, 3 for exon 17, and 1 for exon 18 (details of these are shown in the electrophoresis patterns in Fig. 3). Nucleotide sequences of the exons of the LDL-R genes of the patients suffering FH were determined through sequencing by use of dideoxynucleotide labeled with [$^{35}$S] dATP or sequencing by use of deoxynucleotide labeled with a fluorescent marker.

As a result, FH case 1 was found to have a substitution G → C at nucleotide 137 (G) in exon 2 of the LDL-R gene, which resulted in a mutation of the LDL-R protein such that the amino acid residue Cys encoded by the 25th codon was replaced by Ser (Fig. 4).

These results have clarified that a risk factor indicating abnormality in lipid metabolism can be detected through correlation between the gene mutation occurring at a site coding for amino acid residue 25, cysteine, of the LDL-R protein and abnormality in lipid metabolism.

FH case 2 was found to have a loss of 5 bases corresponding to nucleotides 156 to 160 (CCAGG) in exon 2 of the LDL-R gene, producing a stop codon at codon 31 of the site of deletion (Fig. 5). This deletion mutation anticipates production of an abnormal LDL-R protein consisting of 30 amino acid residues.

Thus, the results have clarified that a risk factor indicating abnormality in lipid metabolism can be detected through correlation between the deletion mutation occurring in nucleotides 156 to 160 of the LDL-R gene and abnormality in lipid metabolism.

FH case 3 was found to have a substitution G → A at nucleotide 211 (G) in exon 3 of the LDL-R gene, which resulted in a mutation of the LDL-R protein such that the amino acid residue Gly encoded by the 50th codon was replaced by Arg (Fig. 6).

These results have clarified that a risk factor indicating abnormality in lipid metabolism can de detected through correlation between the gene mutation occurring at a site coding for amino acid residue 50, glycine, of the LDL-R protein and abnormality in lipid metabolism.

FH case 4 was found to have a substitution T → A at nucleotide 283 (T) in exon 3 of the LDL-R gene, which resulted in a mutation of the LDL-R protein such that the amino acid residue Cys encoded by the 74th codon was replaced by Ser (Fig. 7).

FH case 5 was found to have a substitution C → A at nucleotide 285 (C) in exon 3 of the LDL-R gene, which resulted in a mutation of the LDL-R protein wherein the 74th codon coding for the amino acid residue Cys was changed to a stop codon (Fig. 8). This base substitution anticipates production of an abnormal LDL-R protein consisting of 73 amino acid residues.

The results from FH cases 4 and 5 have clarified that a risk factor indicating abnormality in lipid metabolism can de detected through correlation between the gene mutation occurring at a site coding for amino acid residue 74, cysteine, of the LDL-R protein and abnormality in lipid metabolism.

FH case 6 was found to have a loss of a base (C) corresponding to nucleotide 314 in exon 4 of the LDL-R gene, producing a stop codon at the position counting 101 downstream of the codon 84 of the site of deletion (Fig. 9). This deletion mutation anticipates production of an abnormal LDL-R protein consisting of 183 amino acid residues.

Thus, the results have clarified that a risk factor indicating abnormality in lipid metabolism can de detected through correlation between the deletion mutation occurring at nucleotide 314, cytosine, of the LDL-R gene and abnormality in lipid metabolism.

FH case 7 was found to have a substitution C → T at nucleotide 304 (C) in exon 4 of the LDL-R gene, which resulted in a mutation of the LDL-R protein wherein the 81st codon coding for the amino acid residue Gln was changed to a stop codon (Fig. 10). This base substitution anticipates production of an abnormal LDL-R protein consisting of 80 amino acid residues.

These results have clarified that a risk factor indicating abnormality in lipid metabolism can de detected through correlation between the gene mutation occurring at a site coding for amino acid residue 81, glutamine, of the LDL-R protein and abnormality in lipid metabolism.

FH case 8 was found to have a substitution G → C at nucleotide 326 (G) in exon 4 of the LDL-R gene, which resulted in a mutation of the LDL-R protein such that the amino acid residue Cys encoded by the 88th codon was replaced by Ser (Fig. 11).

These results have clarified that a risk factor indicating abnormality in lipid metabolism can de detected through correlation between the gene mutation occurring at a site coding for amino acid residue 88, cysteine, of the LDL-R protein and abnormality in lipid metabolism.

FH case 9 was found to have a substitution C → T at nucleotide 331 (C) in exon 4 of the LDL-R gene, which resulted in a mutation of the LDL-R protein wherein the 90th codon coding for amino acid residue Gln was changed to a stop codon (Fig. 12). This base substitution anticipates production of an abnormal LDL-R protein consisting of 89 amino acid residues.

These results have clarified that a risk factor indicating abnormality in lipid metabolism can de detected through correlation between the gene mutation occurring at a site coding for amino acid residue 90, glutamine, of the LDL-R protein and abnormality in lipid metabolism.

FH case 10 was found to have a substitution G → A at nucleotide 344 (G) in exon 4 of the LDL-R gene, which resulted in a mutation of the LDL-R protein such that the amino acid residue Arg encoded by the 94th codon was replaced by His (Fig. 13).

These results have clarified that a risk factor indicating abnormality in lipid metabolism can de detected through correlation between the gene mutation occurring at a site coding for amino acid residue 94, arginine, of the LDL-R protein and abnormality in lipid metabolism.

FH case 11 was found to have a loss of 7 bases corresponding to nucleotides 355 to 361 (GGGAAGT) in exon 4 of the LDL-R gene, producing a stop codon at the position counting 85 downstream of the codon 98 of the site of deletion (Fig. 14). This deletion mutation anticipates production of an abnormal LDL-R protein consisting of 181 amino acid residues.

Thus, the results have clarified that a risk factor indicating abnormality in lipid metabolism can de detected through correlation between the deletion mutation occurring in nucleotides 355 to 361 of the LDL-R gene and abnormality in lipid metabolism.

FH case 12 was found to have a substitution T → G at nucleotide 361 (T) in exon 4 of the LDL-R gene, which resulted in a mutation of the LDL-R protein such that the amino acid residue Cys encoded by the 100th codon was replaced by Gly (Fig. 15).

FH case 13 was found to have a substitution C → A at nucleotide 363 (C) in exon 4 of the LDL-R gene, which resulted in a mutation of the LDL-R protein wherein the 100th codon coding for amino acid residue Cys was changed to a stop codon (Fig. 16). This base substitution anticipates production of an abnormal LDL-R protein consisting of 99 amino acid residues.

The results from FH cases 12 and 13 have clarified that a risk factor indicating abnormality in lipid metabolism can de detected through correlation between the gene mutation occurring at a site coding for amino acid residue 100, cysteine, of the LDL-R protein and abnormality in lipid metabolism.

FH case 14 was found to have a loss of two bases (TG) corresponding to nucleotides 382 and 383 in exon 4 of the LDL-R gene, with a codon coding for amino acid residue 107, Cys, having being replaced by a stop codon (Fig. 17). This deletion mutation anticipates production of an abnormal LDL-R protein consisting of 106 amino acid residues.

Thus, the results have clarified that a risk factor indicating abnormality in lipid metabolism can de detected through correlation between the deletion mutation occurring in nucleotides 382 and 383 of the LDL-R gene and abnormality in lipid metabolism.

FH case 15 was found to have an insertion of a base (C) at nucleotide 390 in exon 4 of the LDL-R gene, producing a stop codon at the position counting 49 downstream of the codon 110 of the site of insertion (Fig. 18). This insertion mutation anticipates production of an abnormal LDL-R protein consisting of 157 amino acid residues.

Thus, the results have clarified that a risk factor indicating abnormality in lipid metabolism can de detected through correlation between the insertion mutation occurring at a position corresponding to nucleotide 390 of the LDL-R gene and abnormality in lipid metabolism.

FH case 16 was found to have a substitution G → T at nucleotide 401 (G) in exon 4 of the LDL-R gene, which resulted in a mutation of the LDL-R protein such that the amino acid residue Cys encoded by the 113rd codon was replaced by Phe (Fig. 19).

FH case 17 was found to have a substitution T → C at nucleotide 400 (T) in exon 4 of the LDL-R gene, which resulted in a mutation of the LDL-R protein such that the amino acid residue Cys encoded by the 113rd codon was replaced by Arg (Fig. 20).

The results from FH cases 16 and 17 have clarified that a risk factor indicating abnormality in lipid metabolism can de detected through correlation between the gene mutation occurring at a site coding for amino acid residue 113, cysteine, of the LDL-R protein and abnormality in lipid metabolism.

FH case 18 was found to have a substitution G → A at nucleotide 406 (G) in exon 4 of the LDL-R gene, which resulted in a mutation of the LDL-R protein such that the amino acid residue Asp encoded by the 115th codon was replaced by Asn (Fig. 21).

These results have clarified that a risk factor indicating abnormality in lipid metabolism can de detected through correlation between the gene mutation occurring at a site coding for amino acid residue 115, aspartic acid, of the LDL-R protein and abnormality in lipid metabolism.

FH case 19 was found to have a substitution G → A at nucleotide 418 (G) in exon 4 of the LDL-R gene, which resulted in a mutation of the LDL-R protein such that the amino acid residue Glu encoded by the 119th codon was replaced by Lys.

These results have clarified that a risk factor indicating abnormality in lipid metabolism can de detected through correlation between the gene mutation occurring at a site coding for amino acid residue 119, glutamic acid, of the LDL-R protein and abnormality in lipid metabolism.

FH case 20 was found to have a loss of 7 bases corresponding to nucleotides 578 to 584 (ACAGTAG) in exon 4 of the LDL-R gene, producing a stop codon at the position counting 11 downstream of the codon 172 of the site of deletion (Fig. 22). This deletion mutation anticipates production of an abnormal LDL-R protein consisting of 180 amino acid residues.

Thus, the results have clarified that a risk factor indicating abnormality in lipid metabolism can be detected through correlation between the deletion mutation occurring in nucleotides 578 to 584 of the LDL-R gene and abnormality in lipid metabolism.

FH case 21 was found to have an insertion of 14 bases (AGGACAAATCTGAC) at nucleotide 682 in exon 4 of the LDL-R gene, producing a stop codon at the position counting 147 downstream of the codon 207 of the site of insertion (Fig. 23). This insertion mutation anticipates production of an abnormal LDL-R protein consisting of 352 amino acid residues.

Thus, the results have clarified that a risk factor indicating abnormality in lipid metabolism can be detected through correlation between the insertion mutation occurring at a position corresponding to nucleotide 682 of the LDL-R gene and abnormality in lipid metabolism.

FH case 22 was found to have a loss of 4 bases corresponding to nucleotides 526 to 529 (GGCT) in exon 4 of the LDL-R gene, producing a stop codon at the position counting 30 downstream of the codon 155 of the site of deletion (Fig. 24). This deletion mutation anticipates production of an abnormal LDL-R protein consisting of 182 amino acid residues.

Thus, the results have clarified that a risk factor indicating abnormality in lipid metabolism can be detected through correlation between the deletion mutation occurring in nucleotides 526 to 529 of the LDL-R gene and abnormality in lipid metabolism.

FH case 23 was found to have an insertion of 21 bases (GACTGCAAGGACAAATCTGAC) at nucleotide 661 in exon 4 of the LDL-R gene, and this insertion did not cause a frame shift in codons for amino acid residues (inframe mutation of 21 bases; Fig. 25). The present 21-base insertion was a repetition of seven amino acid residues AspCys-LysAspLysSerAsp encoded by 200th to 206th codons. This insertion mutation anticipates production of an abnormal LDL-R protein composed of amino acid residues in a number 7 greater than those constituting a native LDL-R protein.

Thus, the results have clarified that a risk factor indicating abnormality in lipid metabolism can be detected through correlation between the insertion mutation occurring at a position corresponding to nucleotide 661 of the LDL-R gene and abnormality in lipid metabolism.

FH case 24 was found to have a substitution G → A at nucleotide 682 (G) in exon 4 of the LDL-R gene, which resulted in a mutation of the LDL-R protein such that the amino acid residue Glu encoded by the 207th codon was replaced by Lys (Fig. 26).

FH case 25 was found to have a substitution G → C at nucleotide 682 (G) in exon 4 of the LDL-R gene, which resulted in a mutation of the LDL-R protein such that the amino acid residue Glu encoded by the 207th codon was replaced by Gln (Fig. 27).

The results from FH cases 24 and 25 have clarified that a risk factor indicating abnormality in lipid metabolism can de detected through correlation between the gene mutation occurring at a site coding for amino acid residue 207, glutamic acid, of the LDL-R protein and abnormality in lipid metabolism.

FH case 26 was found to have an insertion of a base (A) at nucleotide 944 in exon 7 of the LDL-R gene, producing a stop codon at the position counting 17 downstream of the codon 294 of the site of insertion (Fig. 28). This insertion mutation anticipates production of an abnormal LDL-R protein consisting of 309 amino acid residues.

Thus, the results have clarified that a risk factor indicating abnormality in lipid metabolism can be detected through correlation between the insertion mutation occurring at a position corresponding to nucleotide 944 of the LDL-R gene and abnormality in lipid metabolism.

FH case 27 was found to have a loss of a base (C) corresponding to nucleotide 948 in exon 7 of the LDL-R gene, producing a stop codon at the position counting 53 downstream of the codon of the site of deletion (Fig. 29). This deletion mutation anticipates production of an abnormal LDL-R protein consisting of 334 amino acid residues.

Thus, the results have clarified that a risk factor indicating abnormality in lipid metabolism can be detected through correlation between the deletion mutation occurring at nucleotide 948, cytosine, of the LDL-R gene and abnormality in lipid metabolism.

FH case 28 was found to have a substitution T → A at nucleotide 1012 (T) in exon 7 of the LDL-R gene, which resulted in a mutation of the LDL-R protein such that the amino acid residue Cys encoded by the 317th codon was replaced by Ser (Fig. 30).

FH case 29 was found to have a substitution T → C at nucleotide 1012 (T) in exon 7 of the LDL-R gene, which resulted in a mutation of the LDL-R protein such that the amino acid residue Cys encoded by the 317th codon was replaced by Arg (Fig. 31).

The results from FH cases 28 and 29 have clarified that a risk factor indicating abnormality in lipid metabolism can de detected through correlation between the gene mutation occurring at a site coding for amino acid residue 317, cysteine, of the LDL-R protein and abnormalityin lipid metabolism.

FH case 30 was found to have a loss of 21 bases corresponding to nucleotides 1114 to 1134 in exon 8 of the LDL-R gene, without producing a frame shift, resulting in a deletion mutation of 21 bases (Fig. 32). This deletion mutation anticipates production of an abnormal LDL-R protein lacking the amino acid residues Glu-Gly-Gly-Tyr-Lys-Cys-Gln encoded by the 351st to 357th codons as compared with normal LDL-R protein.

Thus, the results have clarified that a risk factor indicating abnormality in lipid metabolism can be detected through correlation between the deletion mutation occurring in nucleotides 1114 to 1134 of the LDL-R gene and abnormality in lipid metabolism.

FH case 31 was found to have an insertion of a base (T) at nucleotide 1062 in exon 8 of the LDL-R gene, producing a stop codon at the position counting 3 downstream of the codon 333 of the site of insertion (Fig. 33). This insertion mutation anticipates production of an abnormal LDL-R protein consisting of 334 amino acid residues.

Thus, the results have clarified that a risk factor indicating abnormality in lipid metabolism can be detected through correlation between the insertion mutation occurring at a position corresponding to nucleotide 1062 of the LDL-R gene and abnormality in lipid metabolism.

FH case 32 was found to have a substitution G → T at nucleotide 1069 (G) in exon 8 of the LDL-R gene, which resulted in a mutation of the LDL-R protein wherein the 336th codon coding for the amino acid residue Glu was changed to a stop codon (Fig. 34). This base substitution anticipates production of an abnormal LDL-R protein consisting of 335 amino acid residues.

These results have clarified that a risk factor indicating abnormality in lipid metabolism can be detected through correlation between the gene mutation occurring at a site coding for amino acid residue 336, glutamic acid, of the LDL-R protein encoded by the LDL-R gene and abnormality in lipid metabolism.

FH case 33 was found to have a substitution T → C at nucleotide 1072 (T) in exon 8 of the LDL-R gene, which resulted in a mutation of the LDL-R protein such that the amino acid residue Cys encoded by the 337th codon was replaced by Arg (Fig. 35).

These results have clarified that a risk factor indicating abnormality in lipid metabolism can be detected through correlation between the gene mutation occurring at a site coding for amino acid residue 337, cysteine, of the LDL-R protein encoded by the LDL-R gene and abnormality in lipid metabolism.

FH case 34 was found to have a substitution G → A at nucleotide 1130 (G) in exon 8 of the LDL-R gene, which resulted in a mutation of the LDL-R protein such that the amino acid residue Cys encoded by the 356th codon was replaced by Tyr (Fig. 36).

These results have clarified that a risk factor indicating abnormality in lipid metabolism can be detected through correlation between the gene mutation occurring at a site coding for amino acid residue 356, cysteine, of the LDL-R protein encoded by the LDL-R gene and abnormality in lipid metabolism.

FH case 35 was found to have a loss of 9 bases corresponding to nucleotides 1115 to 1223 (AGGGTGGCT) and an insertion of 6 bases (CACTGA) in exon 8 of the LDL-R gene (Fig. 37), without producing a frame shift, resulting in a mutation of the LDL-R protein such that the amino acid sequence Glu-Gly-Gly-Tyr encoded by the 351st to 354th codons was replaced by a sequence Ala-Leu-Asn. This mutation anticipates production of an abnormal LDL-R protein lacking one amino acid residue as compared with the normal LDL-R protein.

These results have clarified that a risk factor indicating abnormality in lipid metabolism can be detected through correlation between the gene mutation occurring at a site coding for amino acid sequence 351 to 354, glutamic acid - glycine - glycine - tyrosine, of the LDL-R protein encoded by the LDL-R gene and abnormality in lipid metabolism.

FH case 36 was found to have a substitution G → A at nucleotide 1136 (G) in exon 8 of the LDL-R gene, which resulted in a mutation of the LDL-R protein such that the amino acid residue Cys encoded by the 358th codon was replaced by Tyr (Fig. 38).

These results have clarified that a risk factor indicating abnormality in lipid metabolism can be detected through correlation between the gene mutation occurring at a site coding for amino acid residue 358, cysteine, of the LDL-R protein encoded by the LDL-R gene and abnormality in lipid metabolism.

FH case 37 was found to have a substitution G → A at nucleotide -10 (G) in the 5'-end-side acceptor region in intron 8 of the LDL-R gene (Fig. 39). This region is considered to play an important role in the formation of a lariat structure of mRNA during translation of the mRNA.

These results have clarified that a risk factor indicating abnormality in lipid metabolism can be detected through correlation between the mutation of guanine at nucleotide -10 (G) in the 5'-end-side acceptor region in intron 8 of the LDL-R gene and abnormality in lipid metabolism.

FH case 38 was found to have a substitution T → C at nucleotide 1207 (T) in exon 9 of the LDL-R gene, which resulted in a mutation of the LDL-R protein such that the amino acid residue Phe encoded by the 382nd codon was replaced by Leu (Fig. 40).

These results have clarified that a risk factor indicating abnormality in lipid metabolism can be detected through correlation between the gene mutation occurring at a site coding for amino acid residue 382, phenylalanine, of the LDL-R protein encoded by the LDL-R gene and abnormality in lipid metabolism.

FH case 39 was found to have a loss of 3 bases (TCT) corresponding to nucleotides 1202 to 1204 in exon 9 of the LDL-R gene, without producing a frame shift, which resulted in a mutation of the LDL-R protein such that the amino acid residue Phe encoded by the 381st codon was deleted (Fig. 41). This deletion mutation anticipates production of an abnormal LDL-R protein lacking one amino acid residue as compared with the normal LDL-R protein.

Thus, the results have clarified that a risk factor indicating abnormality in lipid metabolism can be detected through correlation between the deletion mutation occurring in nucleotides 1202 to 1204 of the LDL-R gene and abnormality in lipid metabolism.

FH case 40 was found to have a substitution C → T at nucleotide 1216 (C) in exon 9 of the LDL-R gene, which resulted in a mutation of the LDL-R protein such that the amino acid residue Arg encoded by the 385th codon was replaced by Trp (Fig. 42).

These results have clarified that a risk factor indicating abnormality in lipid metabolism can be detected through correlation between the gene mutation occurring at a site coding for amino acid residue 385, arginine, of the LDL-R protein encoded by the LDL-R gene and abnormality in lipid metabolism.

FH case 41 was found to have a substitution G → A at nucleotide 1222 (G) in exon 9 of the LDL-R gene, which resulted in a mutation of the LDL-R protein such that the amino acid residue Glu encoded by the 387th codon was replaced by Lys (Fig. 43).

These results have clarified that a risk factor indicating abnormality in lipid metabolism can be detected through correlation between the gene mutation occurring at a site coding for amino acid residue 387, glutamic acid, of the LDL-R protein encoded by the LDL-R gene and abnormality in lipid metabolism.

FH case 42 was found to have an insertion of 5 bases corresponding to nucleotides 1242 to 1246 (GGACC) in exon 9 of the LDL-R gene, producing a stop codon at the position counting 15 downstream of the codon 393 of the site of insertion (Fig. 44). This insertion mutation anticipates production of an abnormal LDL-R protein consisting of 407 amino acid residues.

Thus, the results have clarified that a risk factor indicating abnormality in lipid metabolism can be detected through correlation between the insertion mutation occurring at a position corresponding to nucleotide 1242 of the LDL-R gene and abnormality in lipid metabolism.

FH case 43 was found to have a substitution T → G at nucleotide 1265 (T) in exon 9 of the LDL-R gene, which resulted in a mutation of the LDL-R protein such that the amino acid residue Leu encoded by the 401st codon was replaced by Arg (Fig. 45).

These results have clarified that a risk factor indicating abnormality in lipid metabolism can be detected through correlation between the gene mutation occurring at a site coding for amino acid residue 401, leucine, of the LDL-R protein encoded by the LDL-R gene and abnormality in lipid metabolism.

FH case 44 was found to have a substitution G → A at nucleotide 1291 (G) in exon 9 of the LDL-R gene, which resulted in a mutation of the LDL-R protein such that the amino acid residue Ala encoded by the 410th codon was replaced by Thr (Fig. 46).

These results have clarified that a risk factor indicating abnormality in lipid metabolism can be detected through correlation between the gene mutation occurring at a site coding for amino acid residue 410, alanine, of the LDL-R protein encoded by the LDL-R gene and abnormality in lipid metabolism.

FH case 45 was found to have a substitution G → C at nucleotide 1297 (G) in exon 9 of the LDL-R gene, which resulted in a mutation of the LDL-R protein such that the amino acid residue Asp encoded by the 412nd codon was replaced by His (Fig. 47).

These results have clarified that a risk factor indicating abnormality in lipid metabolism can be detected through correlation between the gene mutation occurring at a site coding for amino acid residue 412, aspartic acid, of the LDL-R protein encoded by the LDL-R gene and abnormality in lipid metabolism.

FH case 46 was found to have a substitution G → A at nucleotide 1599 (G) in exon 11 of the LDL-R gene, which resulted in a mutation of the LDL-R protein such that the amino acid residue Trp encoded by the 512nd codon was replaced by Arg (Fig. 48). This substitution anticipates production of an abnormal LDL-R protein consisting of 511 amino acid residues. These results have clarified that a risk factor indicating abnormality in lipid metabolism can be detected through correlation between the gene mutation occurring at a site coding for amino acid residue 512, tryptophan, of the LDL-R protein encoded by the LDL-R gene and abnormality in lipid metabolism.

FH case 47 was found to have a loss of a base (G) corresponding to nucleotide 1599 in exon 11 of the LDL-R gene, with a codon coding for amino acid residue 512 having being replaced by a stop codon (Fig. 49). This deletion mutation anticipates production of an abnormal LDL-R protein consisting of 511 amino acid residues.

Thus, the results have clarified that a risk factor indicating abnormality in lipid metabolism can be detected through correlation between the deletion mutation occurring in nucleotide 1599 of the LDL-R gene and abnormality in lipid metabolism.

FH case 48 was found to have an insertion of a base (C) at nucleotide 1687 in exon 11 of the LDL-R gene, producing a stop codon at the position counting 16 downstream of the codon 542 of the site of insertion (Fig. 50). This insertion mutation anticipates production of an abnormal LDL-R protein consisting of 556 amino acid residues.

Thus, the results have clarified that a risk factor indicating abnormality in lipid metabolism can be detected through correlation between the insertion mutation occurring at a position corresponding to nucleotide 1687 of the LDL-R gene and abnormality in lipid metabolism.

FH case 49 was found to have a loss of 11 bases corresponding to nucleotides 1652 to 1662 (ACATCTACTCG) in exon 11 of the LDL-R gene, producing a stop codon at the position counting 4 downstream of the codon 530 of the site of deletion (Fig. 51). This deletion mutation anticipates production of an abnormal LDL-R protein consisting of 533 amino acid residues.

Thus, the results have clarified that a risk factor indicating abnormality in lipid metabolism can be detected through correlation between the deletion mutation occurring in nucleotides 1652 to 1662 of the LDL-R gene and abnormality in lipid metabolism.

FH case 50 was found to have a loss of a base (T) corresponding to nucleotide 1655 in exon 11 of the LDL-R gene, producing a stop codon at the position counting 28 downstream of the codon 531 of the site of deletion (Fig. 52). This deletion mutation anticipates production of an abnormal LDL-R protein consisting of 557 amino acid residues.

Thus, the results have clarified that a risk factor indicating abnormality in lipid metabolism can be detected through correlation between the deletion mutation occurring at nucleotide 1655, thymine, of the LDL-R gene and abnormality in lipid metabolism.

FH case 51 was found to have a substitution C → G at nucleotide 1702 (C) in exon 11 of the LDL-R gene, which resulted in a mutation of the LDL-R protein such that the amino acid residue Leu encoded by the 547th codon was replaced by Val (Fig. 53).

These results have clarified that a risk factor indicating abnormality in lipid metabolism can be detected through correlation between the gene mutation occurring at a site coding for amino acid residue 547, leucine, of the LDL-R protein encoded by the LDL-R gene and abnormality in lipid metabolism.

FH cases 52 and 53 were found to have substitution mutations G → C and G → T, respectively, at nucleotide +1 (guanine) in a splice donor site of intron 11 of the LDL-R gene, causing abnormal splicing, which resulted in inhibiting normal translation of mRNA. These substitution mutations anticipate production of abnormal mRNA.

These results have clarified that a risk factor indicating abnormality in lipid metabolism can be detected through correlation between abnormality in lipid metabolism and the gene mutation, from guanine to another base, of nucleotide +1 (G) in a splice donor site of intron 11 starting from the nucleotide that is one base downstream the nucleotide 1705 of the LDL-R gene.

FH case 54 was found to have a substitution mutation T → G, at nucleotide +2 (T) in a splice donor site of intron 12 of the LDL-R gene, causing abnormal splicing, which resulted in inhibiting normal translation of mRNA. This substitution mutation anticipates production of abnormal mRNA.

These results have clarified that a risk factor indicating abnormality in lipid metabolism can be detected through correlation between abnormality in lipid metabolism and the gene mutation, from thymine to another base, of nucleotide +2 (T) in a splice donor site of intron 12 starting from the nucleotide that is one base downstream the nucleotide 1845 of the LDL-R gene.

FH case 55 was found to have a substitution G → T at nucleotide 1731 (G) in exon 12 of the LDL-R gene, which resulted in a mutation of the LDL-R protein such that the amino acid residue Trp encoded by the 556th codon was replaced by Cys.

These results have clarified that a risk factor indicating abnormality in lipid metabolism can be detected through correlation between the gene mutation occurring at a site coding for amino acid residue 556, tryptophan, of the LDL-R protein encoded by the LDL-R gene and abnormality in lipid metabolism.

FH case 56 was found to have a substitution A → G at nucleotide 1772 (A) in exon 12 of the LDL-R gene, which resulted in a mutation of the LDL-R protein such that the amino acid residue Asn encoded by the 570th codon was replaced by Ser (Fig. 56).

These results have clarified that a risk factor indicating abnormality in lipid metabolism can be detected through correlation between the gene mutation occurring at a site coding for amino acid residue 570, asparagine, of the LDL-R protein encoded by the LDL-R gene and abnormality in lipid metabolism.

FH case 57 was found to have an insertion of a base (G) at nucleotide 1779 in exon 12 of the LDL-R gene, producing a stop codon at the position counting 10 downstream of the codon 572 of the site of insertion (Fig. 57). This insertion mutation anticipates production of an abnormal LDL-R protein consisting of 580 amino acid residues.

Thus, the results have clarified that a risk factor indicating abnormality in lipid metabolism can be detected through correlation between the insertion mutation occurring at a position corresponding to nucleotide 1779 of the LDL-R gene and abnormality in lipid metabolism.

FH case 58 was found to have a substitution C → T at nucleotide 1822 (C) in exon 12 of the LDL-R gene, which resulted in a mutation of the LDL-R protein such that the amino acid residue Pro encoded by the 587th codon was replaced by Ser (Fig. 58).

These results have clarified that a risk factor indicating abnormality in lipid metabolism can be detected through correlation between the gene mutation occurring at a site coding for amino acid residue 587, proline, of the LDL-R protein encoded by the LDL-R gene and abnormality in lipid metabolism.

FH case 59 was found to have a substitution G → T at nucleotide 1834 (G) in exon 12 of the LDL-R gene, which resulted in a mutation of the LDL-R protein such that the amino acid residue Ala encoded by the 591st codon was replaced by Ser (Fig. 59).

These results have clarified that a risk factor indicating abnormality in lipid metabolism can be detected through correlation between the gene mutation occurring at a site coding for amino acid residue 591, alanine, of the LDL-R protein encoded by the LDL-R gene and abnormality in lipid metabolism.

FH case 60 was found to have a loss of 3 bases (TCA) corresponding to nucleotides 1870 to 1872 in exon 13 of the LDL-R gene, without having produced a frame shift, which resulted in a mutation of the LDL-R protein such that the amino acid residue Ile encoded by the 603rd codon was deleted (Fig. 60). This deletion mutation anticipates production of an abnormal LDL-R protein lacking one amino acid residue as compared with the normal LDL-R protein.

Thus, the results have clarified that a risk factor indicating abnormality in lipid metabolism can be detected through correlation between the deletion mutation occurring in nucleotides 1870 to 1872 of the LDL-R gene and abnormality in lipid metabolism.

FH case 61 was found to have a substitution C → T at nucleotide 1897 (C) in exon 13 of the LDL-R gene, which resulted in a mutation of the LDL-R protein such that the amino acid residue Arg encoded by the 612nd codon was replaced by Cys (Fig. 61).

These results have clarified that a risk factor indicating abnormality in lipid metabolism can be detected through correlation between the gene mutation occurring at a site coding for amino acid residue 612, arginine, of the LDL-R protein encoded by the LDL-R gene and abnormality in lipid metabolism.

FH case 62 was found to have a loss of a base corresponding to nucleotide 1963 (T) in exon 13 of the LDL-R gene, producing a stop codon at the position counting 10 downstream of the codon 643 of the site of deletion (Fig. 62). This deletion mutation anticipates production of an abnormal LDL-R protein consisting of 642 amino acid residues.

Thus, the results have clarified that a risk factor indicating abnormality in lipid metabolism can be detected through correlation between the deletion mutation occurring in nucleotide 1963 of the LDL-R gene and abnormality in lipid metabolism.

FH case 63 was found to have an insertion of a base (T) at nucleotide 2035 in exon 14 of the LDL-R gene, producing a stop codon at the position counting 38 downstream of the codon of the site of insertion (Fig. 63). This insertion mutation anticipates production of an abnormal LDL-R protein consisting of 694 amino acid residues.

Thus, the results have clarified that a risk factor indicating abnormality in lipid metabolism can be detected through correlation between the insertion mutation occurring at a position corresponding to nucleotide 2035 of the LDL-R gene and abnormality in lipid metabolism.

FH case 64 was found to have a substitution C → T at nucleotide 2054 (C) in exon 14 of the LDL-R gene, which resulted in a mutation of the LDL-R protein such that the amino acid residue Pro encoded by the 664th codon was replaced by Leu.

These results have clarified that a risk factor indicating abnormality in lipid metabolism can be detected through correlation between the gene mutation occurring at a site coding for amino acid residue 664, proline, of the LDL-R protein encoded by the LDL-R gene and abnormality in lipid metabolism.

FH case 65 was found to have a substitution G → A at nucleotide 2140 (G) in exon 14 of the LDL-R gene, which resulted in a mutation of the LDL-R protein such that the amino acid residue Glu encoded by the 693rd codon was replaced by Lys (Fig. 64).

These results have clarified that a risk factor indicating abnormality in lipid metabolism can be detected through correlation between the gene mutation occurring at a site coding for amino acid residue 693, glutamic acid, of the LDL-R protein and abnormality in lipid metabolism.

FH case 66 was found to have a loss of 21 bases (GACGTTGCTGGCAGAGGAAAT) corresponding to nucleotides 2320 to 2340 in exon 16 of the LDL-R gene. This deletion did not cause any frame shift in codons for amino acid residues, and was a deletion mutation in which the 753rd to 759th codons composed of 21 nucleotides coding for 7 amino acid residues (Asp-Val-Ala-Gly-Arg-Gly-Asn) were deleted (Fig. 65). This deletion mutation anticipates production of an abnormal LDL-R protein composed of amino acid residues in a number 7 fewer than those constituting a native LDL-R protein.

Thus, the results have clarified that a risk factor indicating abnormality in lipid metabolism can be detected through correlation between the deletion mutation occurring in nucleotides 2320 to 2340 of the LDL-R gene and abnormality in lipid metabolism.

FH case 67 was found to have a substitution G → A at nucleotide 2398 (G) in exon 17 of the LDL-R gene, which resulted in a mutation of the LDL-R protein such that the amino acid residue encoded by the 779th codon, Val, was replaced by Ile (Fig. 66).

These results have clarified that a risk factor indicating abnormality in lipid metabolism can be detected through correlation between the gene mutation occurring at a site coding for amino acid residue 779, valine, of the LDL-R protein encoded by the LDL-R gene and abnormality in lipid metabolism.

FH case 68 was found to have a substitution A → T at nucleotide 2431 (A) in exon 17 of the LDL-R gene, which resulted in a mutation of the LDL-R protein wherein the 790th codon coding for the amino acid residue Lys was changed to a stop codon (Fig. 67). This base substitution anticipates production of an abnormal LDL-R protein consisting of 789 amino acid residues.

These results have clarified that a risk factor indicating abnormality in lipid metabolism can be detected through correlation between the gene mutation occurring at a site coding for amino acid residue 790, lysine, of the LDL-R protein encoded by the LDL-R gene and abnormality in lipid metabolism.

FH case 69 was found to have an insertion of a base (G) at nucleotide 2412 in exon 17 of the LDL-R gene, producing a stop codon at the position counting 13 downstream of the codon 783 of the site of insertion (Fig. 68). This insertion mutation anticipates production of an abnormal LDL-R protein consisting of 794 amino acid residues.

Thus, the results have clarified that a risk factor indicating abnormality in lipid metabolism can be detected through correlation between the insertion mutation occurring at a position corresponding to nucleotide 2412 of the LDL-R gene and abnormality in lipid metabolism.

FH case 70 was found to have a substitution C → T at nucleotide 2579 (C) in exon 18 of the LDL-R gene, which resulted in a mutation of the LDL-R protein such that the amino acid residue Ala encoded by the 829th codon was replaced by Val.

These results have clarified that a risk factor indicating abnormality in lipid metabolism can be detected through correlation between the gene mutation occurring at a site coding for amino acid residue 829, alanine, of the LDL-R protein encoded by the LDL-R gene and abnormality in lipid metabolism.

FH case 71 was found to have a substitution T → C at nucleotide 1010 (T) in exon 7 of the LDL-R gene, which resulted in a mutation of the LDL-R protein such that the amino acid residue Glu encoded by the 316th codon was replaced by Gly (Fig. 69).

These results have clarified that a risk factor indicating abnormality in lipid metabolism can be detected through correlation between the gene mutation occurring at a site coding for amino acid residue 316, glutamic acid, of the LDL-R protein encoded by the LDL-R gene and abnormality in lipid metabolism.

The above-described mutations of the LDL-R gene can be categorized into the following 5 groups: 1) a missense mutation; i.e., a base change that alters an amino acid; 2) a nonsense mutation; i.e., a base change that converts an amino acid to a stop codon; 3) a frame shift mutation; i.e., loss or gain of a nucleotide in a coding sequence of amino acids, to thereby shift a frame of translational codons, producing a stop codon on the downstream side; 4) an inframe mutation; i.e., loss or gain of a nucleotide in a coding sequence of amino acids, to thereby shift a frame of translational codons without producing a stop codon; and 5) a silent mutation; i.e., a base change that has no effect on amino acid sequence. The mutations of groups 1) to 4) result in a quantitatively or qualitatively abnormal LDL-R protein after synthesis, disabling the LDL-R protein in living organisms from exhibiting normal functions, and elevating the blood cholesterol level. A mutation of group 5) does not itself cause any quantitative or qualitative abnormalities of LDL-R protein, but in analysis, this mutation can be used as a risk factor that indicates a genetic polymorphism which may be related to an abnormal LDL-R gene (disease-associated gene mutation), so as to determine the level of risk of suffering a disease (arteriosclerosis or ischemic heart disease).

In relation to the analyses of the present study, the following polymorphisms of the LDL-R gene were identified as the mentioned disease-associated gene mutations:

Cys6Cys in which the 81st base has been changed from T to C (Fig. 70), Ser191Ser in which the 636th base has been changed from C to T (Fig. 71), Gly301Gly in which the 969th base has been changed from C to T (Fig. 72), Ile313Ile in which the 1002nd base has been changed from C to T (Fig. 73), Ile377Ile in which the 1195th base has been changed from C to T (Fig. 74), Leu554Leu in which the 1725th base has been changed from C to T (Fig. 75), Asn570Asn in which the 1773rd base has been changed from T to C (Fig. 76), and Ala585Ala in which the 1817th base has been changed from C to T and Val632Val in which the 1959th base has been changed from C to T (Fig. 77).

Industrial Applicability

The present invention contemplates provision of widely applicable means for attaining ensured diagnosis of familial hyperlipemia.

**Claims**

1. A method of detecting abnormality of lipid metabolism, which comprises a step of correlating abnormality in lipid metabolism with one or more gene mutations selected from the group consisting of the below-described 1 through 65 gene mutations, to thereby detect a risk factor concerning abnormality of lipid metabolism:

1) a mutation of a low-density lipoprotein receptor gene coding for low-density lipoprotein receptor protein, the mutation occurring at a site coding for amino acid residue 25, cysteine, of the low-density lipoprotein receptor protein;
2) a deletion mutation occurring in nucleotides 156 to 160 of the low-density lipoprotein receptor gene;
3) a mutation of the low-density lipoprotein receptor gene occurring at a site coding for amino acid residue 50, glycine, of the low-density lipoprotein receptor protein encoded by the gene;
4) a mutation of the low-density lipoprotein receptor gene occurring at a site coding for amino acid residue 74, cysteine, of the low-density lipoprotein receptor protein encoded by the gene;
5) a deletion mutation occurring in nucleotide 314, cytosine, of the low-density lipoprotein receptor gene;
6) a mutation of the low-density lipoprotein receptor gene occurring at a site coding for amino acid residue 81, glutamine, of the low-density lipoprotein receptor protein encoded by the gene;
7) a mutation of the low-density lipoprotein receptor gene occurring at a site coding for amino acid residue 88, cysteine, of the low-density lipoprotein receptor protein encoded by the gene;
8) a mutation of the low-density lipoprotein receptor gene occurring at a site coding for amino acid residue 90, glutamine, of the low-density lipoprotein receptor protein encoded by the gene;
9) a mutation of the low-density lipoprotein receptor gene occurring at a site coding for amino acid residue 94, arginine, of the low-density lipoprotein receptor protein encoded by the gene;
10) a deletion mutation occurring in nucleotides 355 to 361, of the low-density lipoprotein receptor gene;
11) a mutation of the low-density lipoprotein receptor gene occurring at a site coding for amino acid residue 100, cysteine, of the low-density lipoprotein receptor protein encoded by the gene;
12) a deletion mutation occurring in nucleotides 382 and 383, of the low-density lipoprotein receptor gene;
13) an insertion mutation occurring at a position of nucleotide 390 of the low-density lipoprotein receptor gene;
14) a mutation of the low-density lipoprotein receptor gene occurring at a site coding for amino acid residue 113, cysteine, of the low-density lipoprotein receptor protein encoded by the gene;
15) a mutation of the low-density lipoprotein receptor gene occurring at a site coding for amino acid residue 115, aspartic acid, of the low-density lipoprotein receptor protein encoded by the gene;
16) a mutation of the low-density lipoprotein receptor gene occurring at a site coding for amino acid residue 119, glutamic acid, of the low-density lipoprotein receptor protein encoded by the gene;
17) a deletion mutation occurring in nucleotides 578 to 584, of the low-density lipoprotein receptor gene;
18) an insertion mutation occurring at a position of nucleotide 682 of the low-density lipoprotein receptor gene;
19) a deletion mutation occurring in nucleotides 526 to 529, of the low-density lipoprotein receptor gene;
20) an insertion mutation occurring at a position of nucleotide 661 of the low-density lipoprotein receptor gene;
21) a mutation of the low-density lipoprotein receptor gene occurring at a site coding for amino acid residue 207, glutamic acid, of the low-density lipoprotein receptor protein encoded by the gene;
22) an insertion mutation occurring at a position of nucleotide 944 of the low-density lipoprotein receptor gene;
23) a deletion mutation occurring in nucleotide 948, cytosine, of the low-density lipoprotein receptor gene;
24) a mutation of the low-density lipoprotein receptor gene occurring at a site coding for amino acid residue 317, cysteine, of the low-density lipoprotein receptor protein encoded by the gene;
25) a deletion mutation occurring in nucleotides 1114 to 1134, of the low-density lipoprotein receptor gene;
26) an insertion mutation occurring at a position of nucleotide 1062 of the low-density lipoprotein receptor gene;
27) a mutation of the low-density lipoprotein receptor gene occurring at a site coding for amino acid residue 336, glutamic acid, of the low-density lipoprotein receptor protein encoded by the gene;
28) a mutation of the low-density lipoprotein receptor gene occurring at a site coding for amino acid residue 337, cysteine, of the low-density lipoprotein receptor protein encoded by the gene;
29) a mutation of the low-density lipoprotein receptor gene occurring at a site coding for amino acid residue 356, cysteine, of the low-density lipoprotein receptor protein encoded by the gene;
30) a mutation of the low-density lipoprotein receptor gene occurring at a site coding for amino acid residues 351 to 354, glutamic acid - glycine - glycine - tyrosine, of the low-density lipoprotein receptor protein encoded by the gene;
31) a mutation of the low-density lipoprotein receptor gene occurring at a site coding for amino acid residue 358, cysteine, of the low-density lipoprotein receptor protein encoded by the gene;
32) a mutation occurring at nucleotide -10 (guanine) in the 5'-end-side acceptor region in intron 8 of the low-density lipoprotein receptor gene;
33) a mutation of the low-density lipoprotein receptor gene occurring at a site coding for amino acid residue 382, phenylalanine, of the low-density lipoprotein receptor protein encoded by the gene;
34) a mutation occurring in nucleotide 1599, of the low-density lipoprotein receptor gene;
35) a deletion mutation occurring in nucleotides 1202 to 1204, of the low-density lipoprotein receptor gene;
36) a mutation of the low-density lipoprotein receptor gene occurring at a site coding for amino acid residue 385, arginine, of the low-density lipoprotein receptor protein encoded by the gene;
37) a mutation of the low-density lipoprotein receptor gene occurring at a site coding for amino acid residue 387, glutamic acid, of the low-density lipoprotein receptor protein encoded by the gene;
38) an insertion mutation occurring at a position of nucleotide 1242 of the low-density lipoprotein receptor gene;
39) a mutation of the low-density lipoprotein receptor gene occurring at a site coding for amino acid residue 401, leucine, of the low-density lipoprotein receptor protein encoded by the gene;
40) a mutation of the low-density lipoprotein receptor gene occurring at a site coding for amino acid residue 410, alanine, of the low-density lipoprotein receptor protein encoded by the gene;
41) a mutation of the low-density lipoprotein receptor gene occurring at a site coding for amino acid residue 412, aspartic acid, of the low-density lipoprotein receptor protein encoded by the gene;
42) a mutation of the low-density lipoprotein receptor gene occurring at a site coding for amino acid residue 512, tryptophan, of the low-density lipoprotein receptor protein encoded by the gene;
43) a deletion mutation occurring in nucleotides 1652 to 1662, of the low-density lipoprotein receptor gene;
44) a deletion mutation occurring in nucleotide 1655, thymine, of the low-density lipoprotein receptor gene;
45) an insertion mutation occurring at a position of nucleotide 1687 of the low-density lipoprotein receptor gene;
46) a mutation of the low-density lipoprotein receptor gene occurring at a site coding for amino acid residue 547, leucine, of the low-density lipoprotein receptor protein encoded by the gene;
47) a mutation from guanine to another base, occurring at nucleotide +1 (guanine) in a splice donor site of intron 11 starting from the nucleotide that is one base downstream the nucleotide 1705 of the low-density lipoprotein receptor protein gene;
48) a mutation from thymine to another base, occurring at nucleotide +2 (thymine) in a splice donor site of intron 12 starting from the nucleotide that is one base downstream the nucleotide 1845 of the low-density lipoprotein receptor protein gene;
49) a mutation of the low-density lipoprotein receptor gene occurring at a site coding for amino acid residue 556, tryptophan, of the low-density lipoprotein receptor protein encoded by the gene;
50) a mutation of the low-density lipoprotein receptor gene occurring at a site coding for amino acid residue 570, asparagine, of the low-density lipoprotein receptor protein encoded by the gene;
51) an insertion mutation occurring at a position of nucleotide 1779 of the low-density lipoprotein receptor gene;
52) a mutation of the low-density lipoprotein receptor gene occurring at a site coding for amino acid residue 587, proline, of the low-density lipoprotein receptor protein encoded by the gene;
53) a mutation of the low-density lipoprotein receptor gene occurring at a site coding for amino acid residue 591, alanine, of the low-density lipoprotein receptor protein encoded by the gene;
54) a deletion mutation occurring in nucleotides 1870 to 1872, of the low-density lipoprotein receptor gene;
55) a mutation of the low-density lipoprotein receptor gene occurring at a site coding for amino acid residue 612, arginine, of the low-density lipoprotein receptor protein encoded by the gene;
56) a deletion mutation occurring in nucleotide 1963, of the low-density lipoprotein receptor gene;
57) an insertion mutation occurring at a position of nucleotide 2035 of the low-density lipoprotein receptor gene;
58) a mutation of the low-density lipoprotein receptor gene occurring at a site coding for amino acid residue 664, proline, of the low-density lipoprotein receptor protein encoded by the gene;
59) a mutation of the low-density lipoprotein receptor gene occurring at a site coding for amino acid residue 693, glutamic acid, of the low-density lipoprotein receptor protein encoded by the gene;
60) a deletion mutation occurring in nucleotides 2320 to 2340, of the low-density lipoprotein receptor gene;
61) a mutation of the low-density lipoprotein receptor gene occurring at a site coding for amino acid residue 779, valine, of the low-density lipoprotein receptor protein encoded by the gene;
62) a mutation of the low-density lipoprotein receptor. gene occurring at a site coding for amino acid residue 790, lysine, of the low-density lipoprotein receptor protein encoded by the gene;
63) an insertion mutation occurring at a position of nucleotide 2412 of the low-density lipoprotein receptor gene;
64) a mutation of the low-density lipoprotein receptor gene occurring at a site coding for amino acid residue 829, alanine, of the low-density lipoprotein receptor protein encoded by the gene; and
65) a mutation of the low-density lipoprotein receptor gene occurring at a site coding for amino acid residue 316, glutamic acid, of the low-density lipoprotein receptor protein encoded by the gene.

**2.** The method of detecting abnormality of lipid metabolism as recited in claim 1, wherein said one or more gene mutations selected from said group include at least one of the following gene mutations:

1) a mutation of a low-density lipoprotein receptor gene coding for low-density lipoprotein receptor protein, the mutation occurring at a site coding for amino acid residue 25, cysteine, of the low-density lipoprotein receptor protein;

2) a deletion mutation occurring in nucleotides 156 to 160 of the low-density lipoprotein receptor gene;
3) a mutation of the low-density lipoprotein receptor gene occurring at a site coding for amino acid residue 50, glycine, of the low-density lipoprotein receptor protein encoded by the gene;
4) a mutation of the low-density lipoprotein receptor gene occurring at a site coding for amino acid residue 74, cysteine, of the low-density lipoprotein receptor protein encoded by the gene;
5) a deletion mutation occurring in nucleotide 314, cytosine, of the low-density lipoprotein receptor gene;
7) a mutation of the low-density lipoprotein receptor gene occurring at a site coding for amino acid residue 88, cysteine, of the low-density lipoprotein receptor protein encoded by the gene;
8) a mutation of the low-density lipoprotein receptor gene occurring at a site coding for amino acid residue 90, glutamine, of the low-density lipoprotein receptor protein encoded by the gene;
9) a mutation of the low-density lipoprotein receptor gene occurring at a site coding for amino acid residue 94, arginine, of the low-density lipoprotein receptor protein encoded by the gene;
10) a deletion mutation occurring in nucleotides 355 to 361, of the low-density lipoprotein receptor gene;
11) a mutation of the low-density lipoprotein receptor gene occurring at a site coding for amino acid residue 100, cysteine, of the low-density lipoprotein receptor protein encoded by the gene;
12) a deletion mutation occurring in nucleotides 382 and 383, of the low-density lipoprotein receptor gene;
13) an insertion mutation occurring at a position of nucleotide 390 of the low-density lipoprotein receptor gene;
14) a mutation of the low-density lipoprotein receptor gene occurring at a site coding for amino acid residue 113, cysteine, of the low-density lipoprotein receptor protein encoded by the gene;
15) a mutation of the low-density lipoprotein receptor gene occurring at a site coding for amino acid residue 115, aspartic acid, of the low-density lipoprotein receptor protein encoded by the gene;
17) a deletion mutation occurring in nucleotides 578 to 584, of the low-density lipoprotein receptor gene;
18) an insertion mutation occurring at a position of nucleotide 682 of the low-density lipoprotein receptor gene;
19) a deletion mutation occurring in nucleotides 526 to 529, of the low-density lipoprotein receptor gene;
22) an insertion mutation occurring at a position of nucleotide 944 of the low-density lipoprotein receptor,'gene;
23) a deletion mutation occurring in nucleotide 948, cytosine, of the low-density lipoprotein receptor gene;
24) a mutation of the low-density lipoprotein receptor gene occurring at a site coding for amino acid residue 317, cysteine, of the low-density lipoprotein receptor protein encoded by the gene;
25) a deletion mutation occurring in nucleotides 1114 to 1134, of the low-density lipoprotein receptor gene;
26) an insertion mutation occurring at a position of nucleotide 1062 of the low-density lipoprotein receptor gene;
27) a mutation of the low-density lipoprotein receptor gene occurring at a site coding for amino acid residue 336, glutamic acid, of the low-density lipoprotein receptor protein encoded by the gene;
28) a mutation of the low-density lipoprotein receptor gene occurring at a site coding for amino acid residue 337, cysteine, of the low-density lipoprotein receptor protein encoded by the gene;
29) a mutation of the low-density lipoprotein receptor gene occurring at a site coding for amino acid residue 356, cysteine, of the low-density lipoprotein receptor protein encoded by the gene;
32) a mutation occurring at nucleotide -10 (guanine) in the 5'-end-side acceptor region in intron 8 of the low-density lipoprotein receptor gene;
34) a mutation occurring in nucleotide 1599, of the low-density lipoprotein receptor gene;
35) a deletion mutation occurring in nucleotides 1202 to 1204, of the low-density lipoprotein receptor gene;
39) a mutation of the low-density lipoprotein receptor gene occurring at a site coding for amino acid residue 401, leucine, of the low-density lipoprotein receptor protein encoded by the gene;
43) a deletion mutation occurring in nucleotides 1652 to 1662, of the low-density lipoprotein receptor gene;
44) a deletion mutation occurring in nucleotide 1655, thymine, of the low-density lipoprotein receptor gene;
47) a mutation from guanine to another base, occurring at nucleotide +1 (guanine) in a splice donor site of intron 11 starting from the nucleotide that is one base downstream the nucleotide 1705 of the low-density lipoprotein receptor protein gene;
49) a mutation of the low-density lipoprotein receptor gene occurring at a site coding for amino acid residue 556, tryptophan, of the low-density lipoprotein receptor protein encoded by the gene;
50) a mutation of the low-density lipoprotein receptor gene occurring at a site coding for amino acid residue 570, asparagine, of the low-density lipoprotein receptor protein encoded by the gene;
51) an insertion mutation occurring at a position of nucleotide 1779 of the low-density lipoprotein receptor gene;
53) a mutation of the low-density lipoprotein receptor gene occurring at a site coding for amino acid residues 591, alanine, of the low-density lipoprotein receptor protein encoded by the gene;
54) a deletion mutation occurring in nucleotides 1870 to 1872, of the low-density lipoprotein receptor gene;
55) a mutation of the low-density lipoprotein receptor gene occurring at a site coding for amino acid residue 612, arginine, of the low-density lipoprotein receptor protein encoded by the gene;
56) a deletion mutation occurring in nucleotide 1963, of the low-density lipoprotein receptor gene;

57) an insertion mutation occurring at a position of nucleotide 2035 of the low-density lipoprotein receptor gene;
60) a deletion mutation occurring in nucleotides 2320 to 2340, of the low-density lipoprotein receptor gene;
61) a mutation of the low-density lipoprotein receptor gene occurring at a site coding for amino acid residue 779, valine, of the low-density lipoprotein receptor protein encoded by the gene;
63) an insertion mutation occurring at a position of nucleotide 2412 of the low-density lipoprotein receptor gene;
64) a mutation of the low-density lipoprotein receptor gene occurring at a site coding for amino acid residue 829, alanine, of the low-density lipoprotein receptor protein encoded by the gene; and
65) a mutation of the low-density lipoprotein receptor gene occurring at a site coding for amino acid residue 316, glutamic acid, of the low-density lipoprotein receptor protein encoded by the gene.

**3.** A method of detecting a disease, comprising detecting a risk factor for arteriosclerosis and/or ischemic heart disease through employment, as an index, the abnormality in lipid metabolism detected by the method for detecting abnormality of lipid metabolism as recited in claim 1 or 2.

Fig. 1

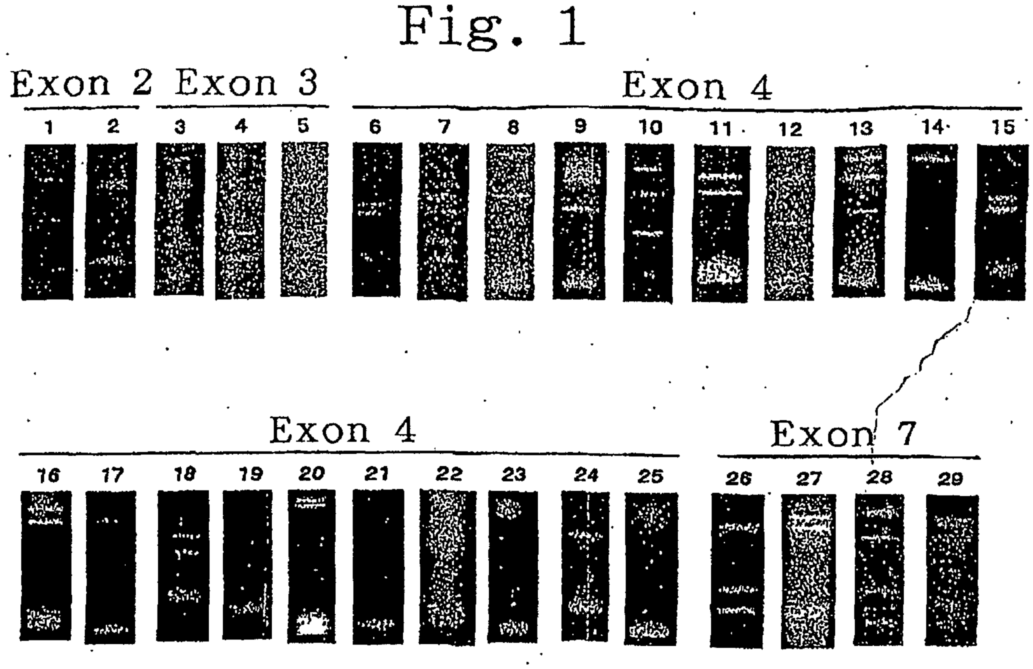
Exon 2
Exon 3
Exon 4
1 2 3 4 5 6 7 8 9 10 11 12 13 14 15
Exon 4
Exon 7
16 17 18 19 20 21 22 23 24 25 26 27 28 29

Fig. 2

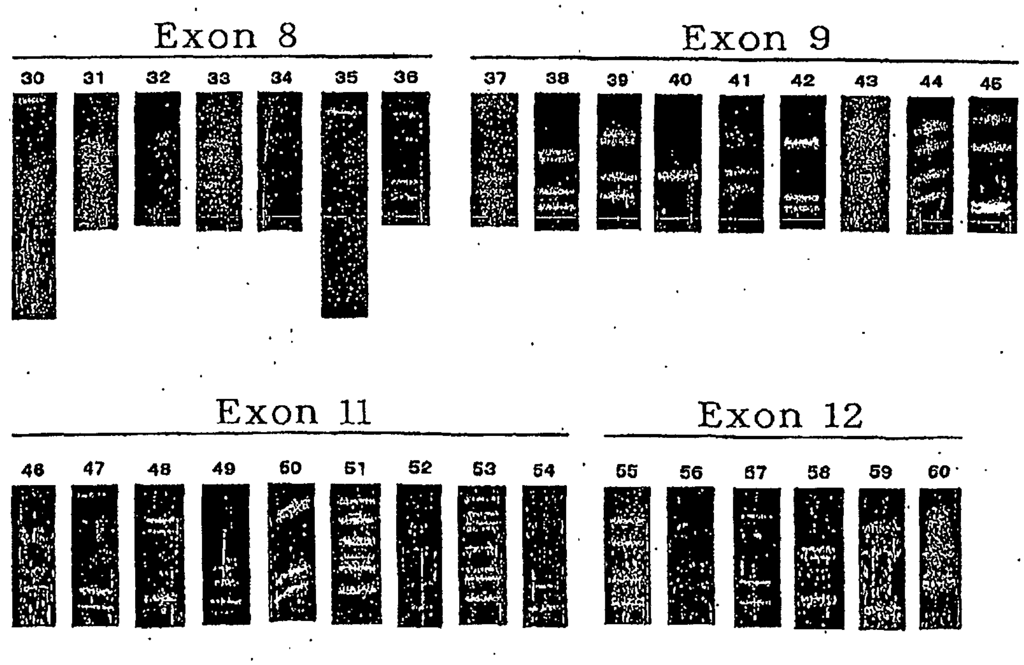
Exon 8
Exon 9
30 31 32 33 34 35 36 37 38 39 40 41 42 43 44 45
Exon 11
Exon 12
46 47 48 49 50 51 52 53 54 55 56 57 58 59 60

Fig. 3

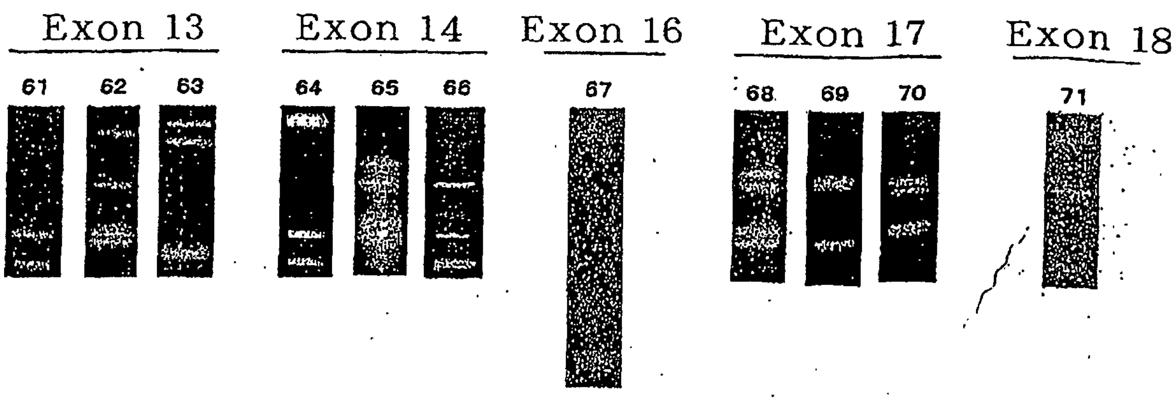
Exon 13
Exon 14
Exon 16
Exon 17
Exon 18
61
62
63
64
65
66
67
68
69
70
71

Fig. 4

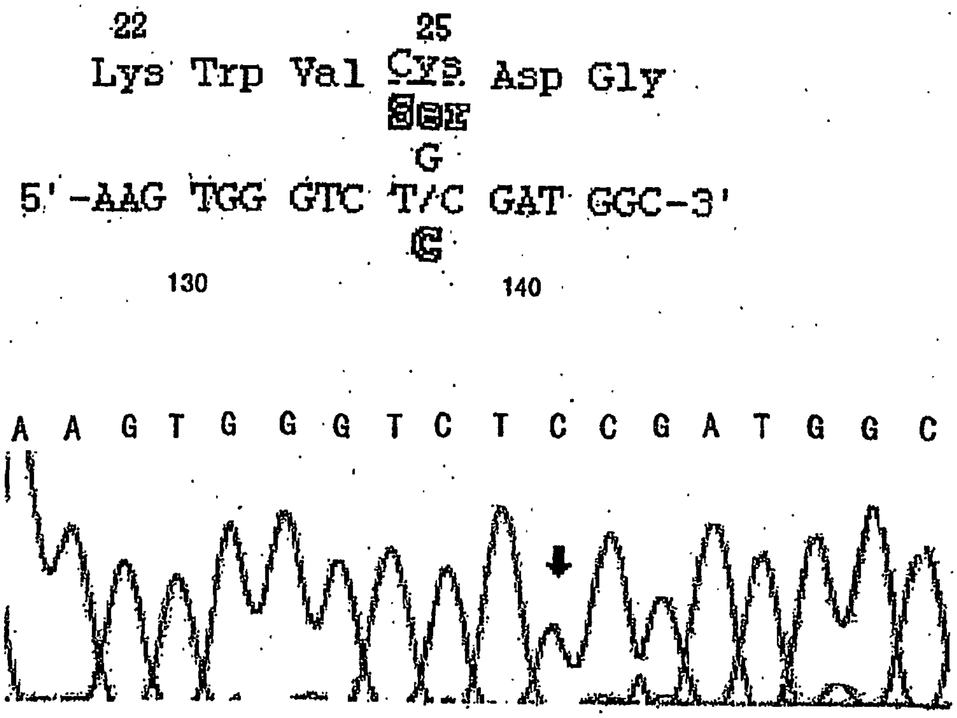
22
25
Lys Trp Val Cys Asp Gly
Ser
G
5'-AAG TGG GTC T/C GAT GGC-3'
G
130
140
A A G T G G G T C T C C G A T G G C

Fig. 5

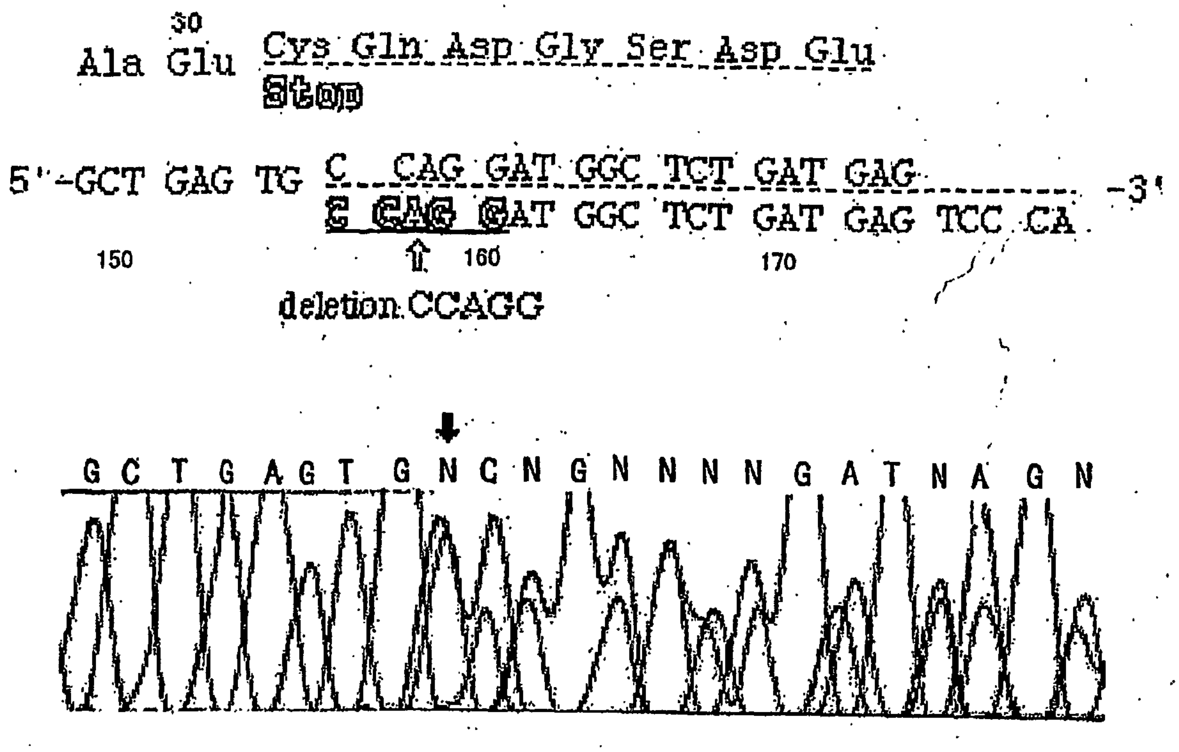
50
Ala Glu Cys Gln Asp Gly Ser Asp Glu
Stop
5'-GCT GAG TG C CAG GAT GGC TCT GAT GAG -3'
C CAG GAT GGC TCT GAT GAG TCC CA
150
160
170
deletion CCAGG
G C T G A G T G N C N G N N N N G A T N A G N

Fig. 6

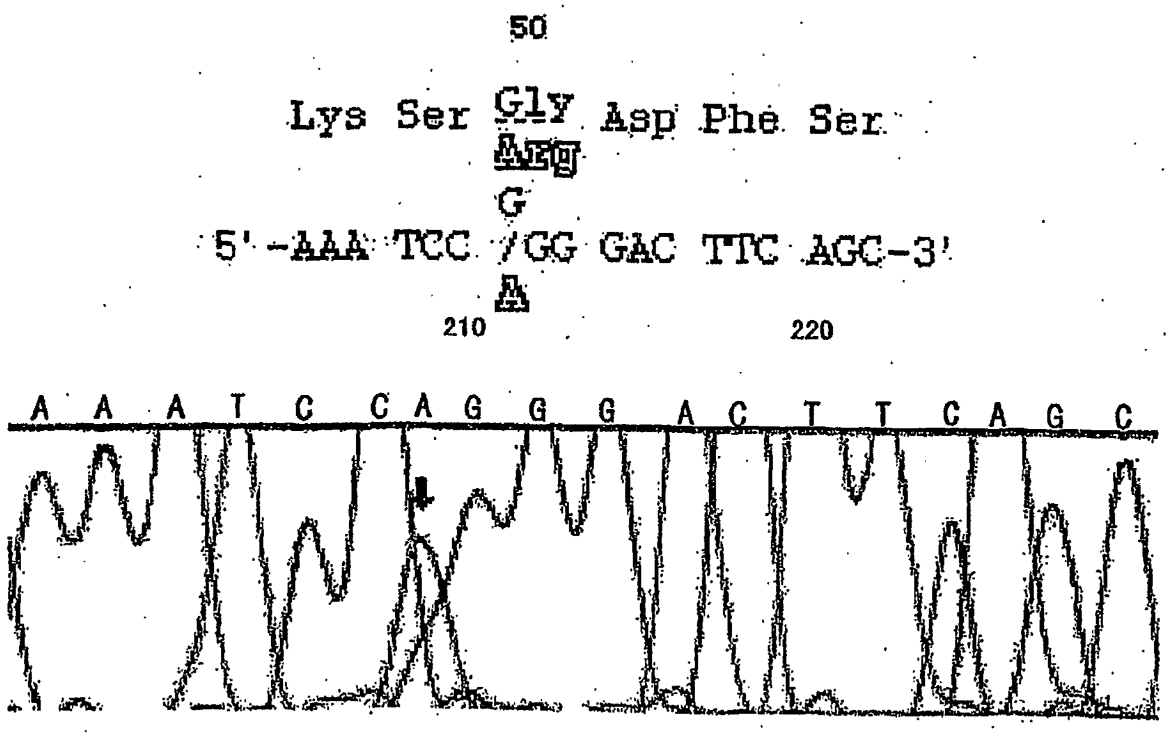
50
Lys Ser Gly Asp Phe Ser
Arg
G
5'-AAA TCC /GG GAC TTC AGC-3'
A
210
220
A A A T C C A G G G A C T T C A G C

# Fig. 7

70 75

Gly Gln Val Asp Cys/Ser Asp Asn Gly Ser Asp

T

5'-GGC CAA GTG GAC /GC GAC AAC GGC TCA GAC-3'

A

280 290 300

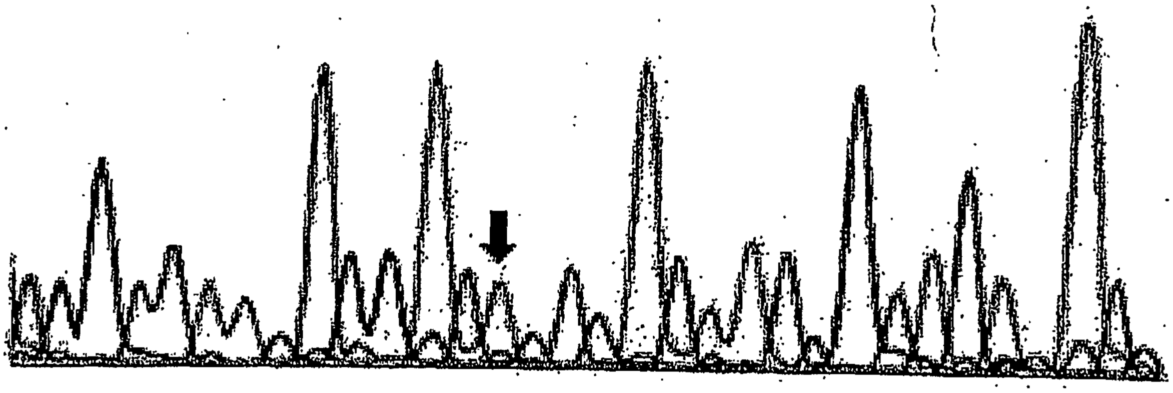

# Fig. 8

75

Val Asp Cys/stop Asp Asn Gly

C

5'-A GTG GAC TG/ GAC AAC GGC-3'

A

280 290

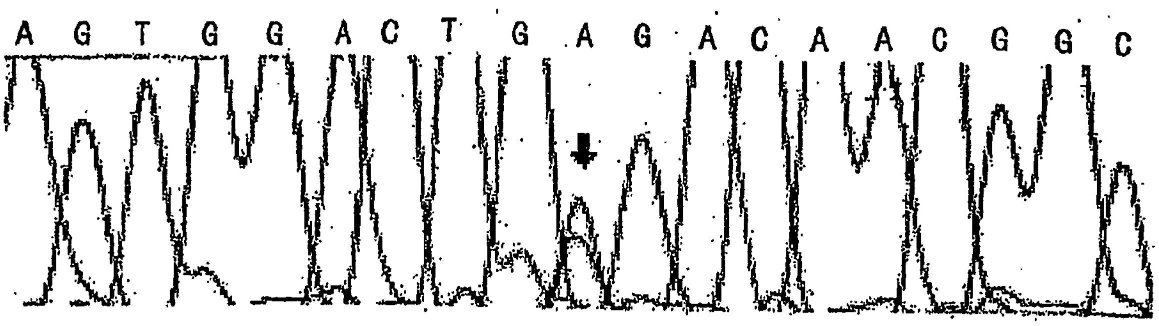

Fig. 9

5'-ctgcagGCCC CAAGACGTGCTCCCAGGACG -3'
CAAGACGTGCTCCCAGGACG

310 320 330

deletion C

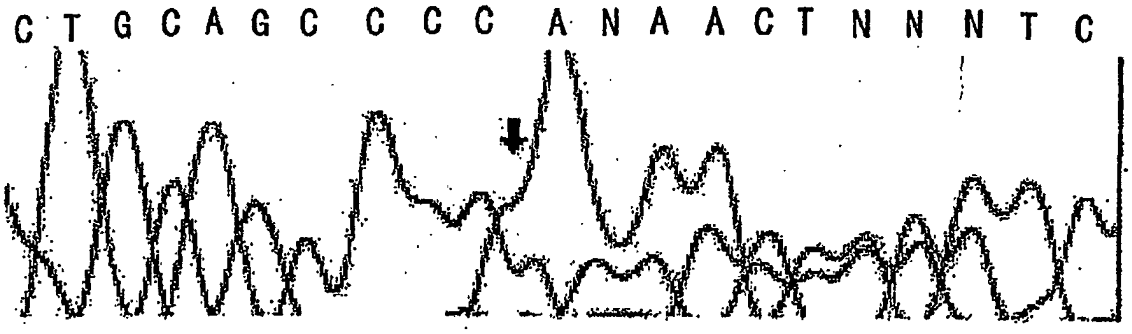


Fig. 10

80
Asp Glu Gln Gly
stop
C
5'-GAC GAG /AA GGC Tgt-3'
T
300 310

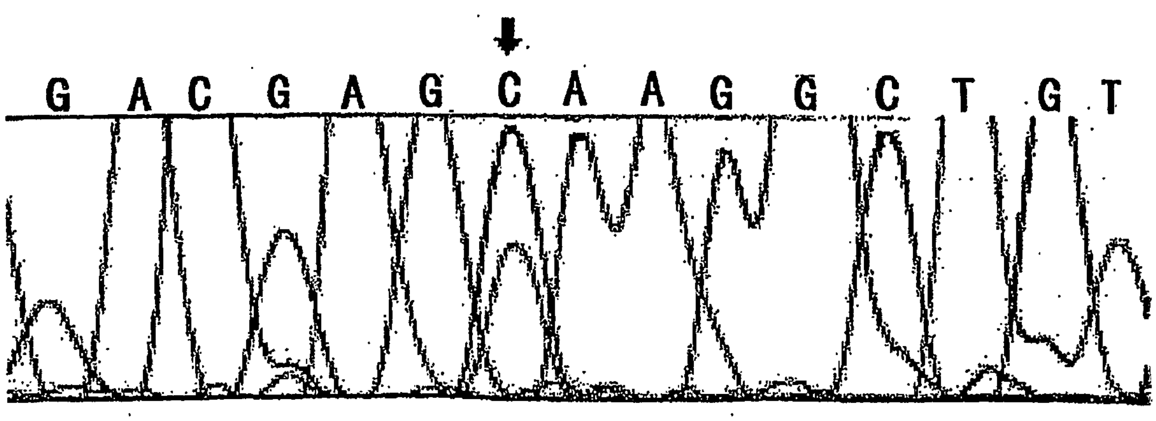

# Fig. 11

86 88 90

Lys Thr Ser Ser Gln

5'-AAG ACG TGC TCC CAG-3'

320 330

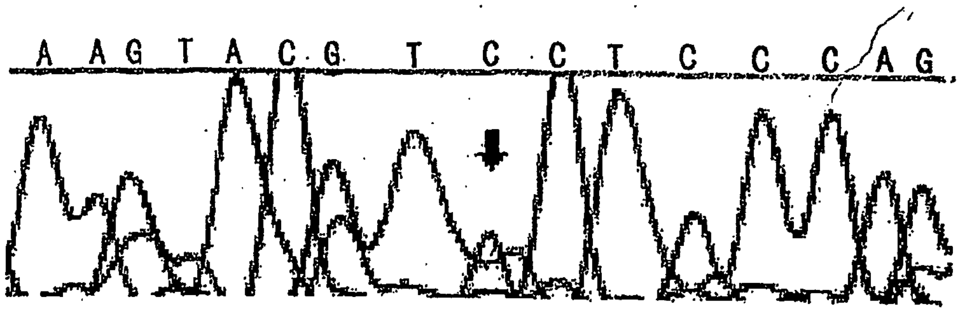


# Fig. 12

90

Cys Ser Gln Asp Glu Phe

stop

C

5'-TGC TCC /AG GAC GAG TTT-3'

T

330 340

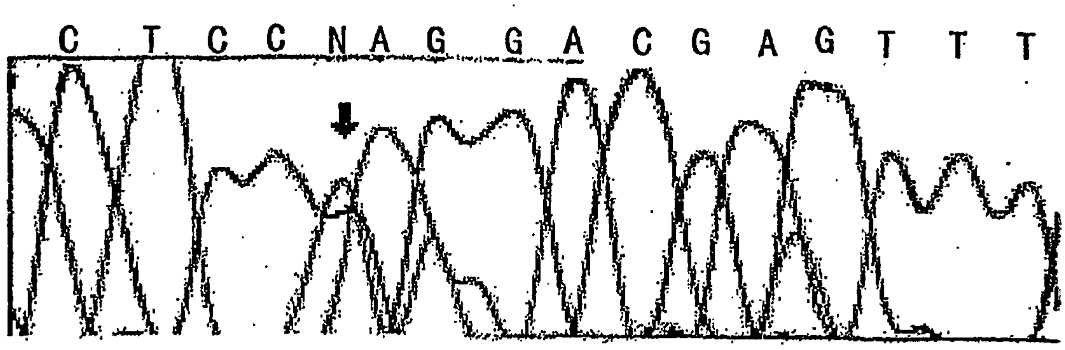

# Fig. 13

90 95
Gln Asp Glu Phe Arg/His Cys His Asp Gly

5'-C CAG GAC GAG TTT C/C TGC CAC GAT GGG-5' (G above, A below the C/C position)
330 340 350

3'-CCC ATC GTG GCA G/G AAA CTC GTC CTG G-5' (C above, T below the G/G position)
350 340 330

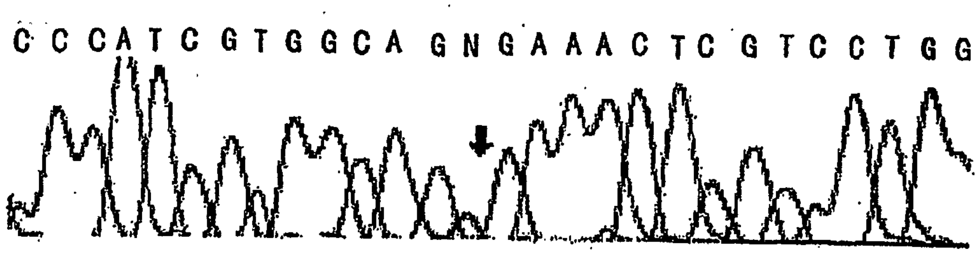


# Fig. 14

95
Cys His Asp Ala Ile Leu Gly Met

5'-TGC CAC GAT GGG AAG TGC ATC TCT CGG CAT G-3'
350 360 370

deletion GGGAAGT

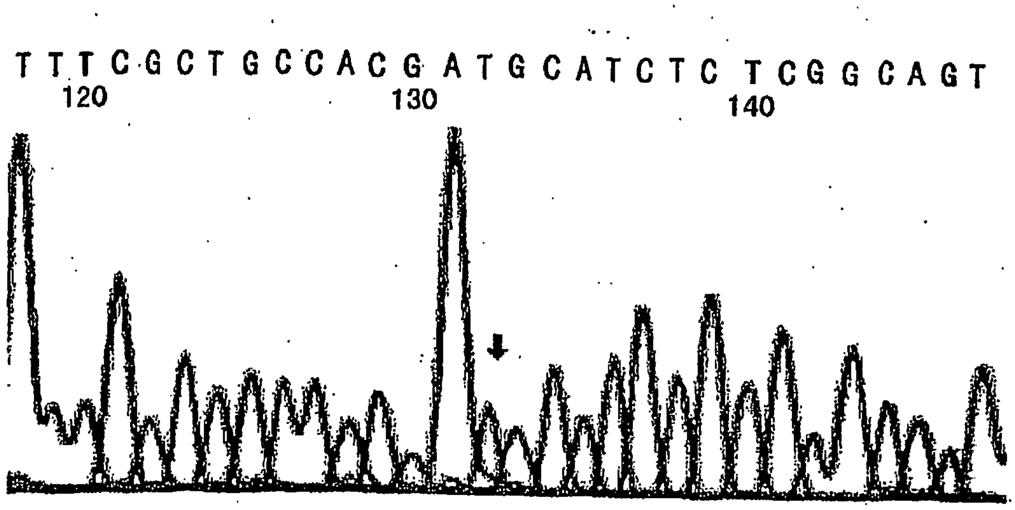

Fig. 15

100

Gly Lys Cys Ile Ser Arg

Gly

T

5'-GGG AAG /GC ATC TCT CGG-3'

G

360 370

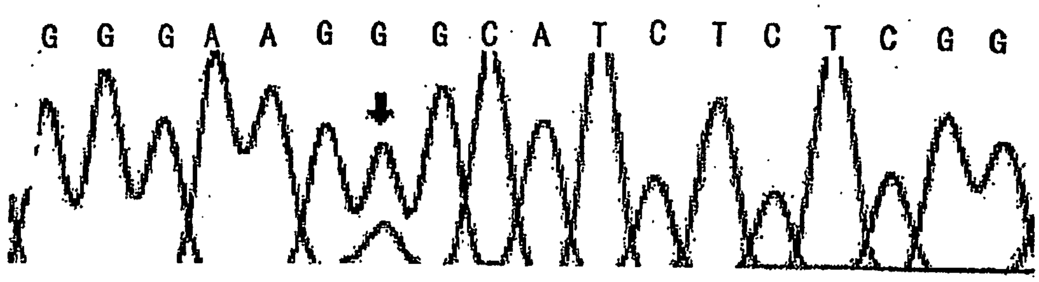


Fig. 16

100

Gly Lys Cys Ile Ser Arg

stop

C

5'-GGG AAG TG/ ATC TCT CGG-3'

A

360 370

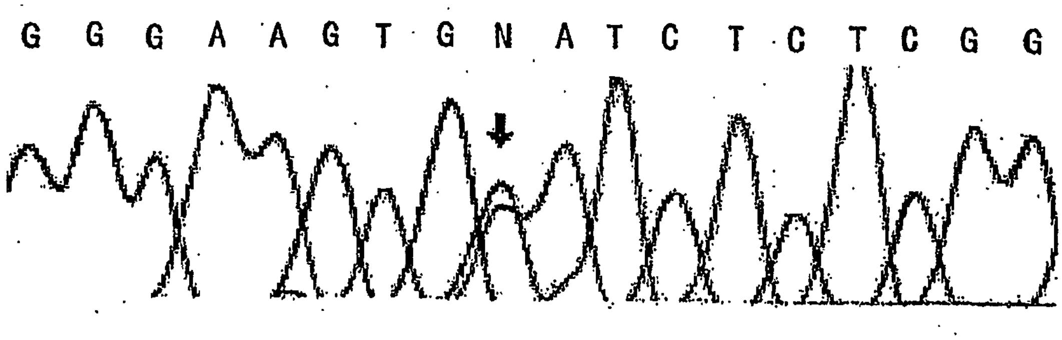

# Fig. 17

```
                106                               110
   Arg Gln Phe Val Cys Asp Ser Asp Arg
5'-CGG CAG TTC GTC TGT GAC TCA GAC CGG G-3'
   370          380          390
```

```
                106
   Arg Gln Phe Val stop
5'-CGG CAG TTC GTC TGA CTC AGA CCG GG-3'
   370          380  ⇧         390
```

deletion TG

C G G C A G T T C G T C T G A C T C A G A C C G G G
170 180

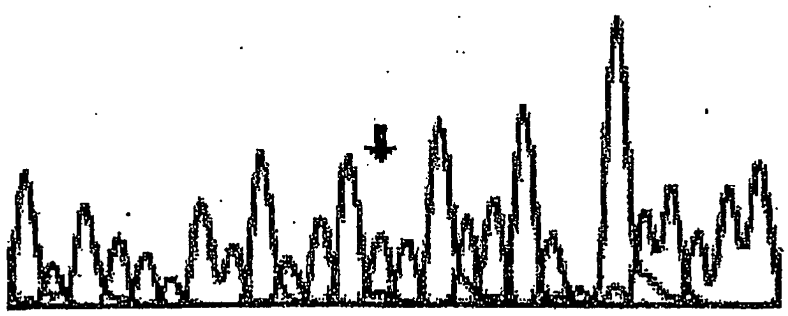

# Fig. 18

110

Cys Asp Ser Asp Arg Asp

5'- TGT GAC TCA GAC CGG GAC T -3'

390 400

110

Cys Asp Ser Arg Pro Gly

5'- TGT GAC TCC AGA CCG GGA CT -3'

390 400

TGTGACTCCAGACCGGGACT

180 190

insertion C

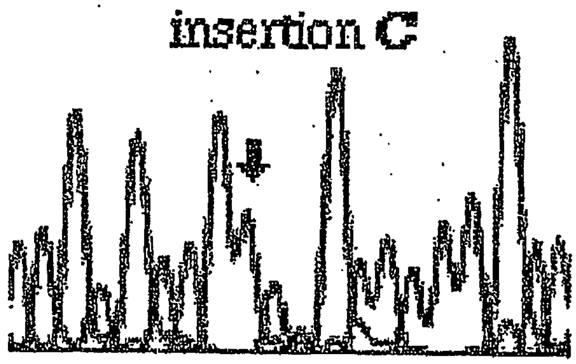

# Fig. 19

110 115

Ser Asp Arg Asp Cys Leu Asp

Phe

G

5'-TCA GAC CGG GAC T/C TTG GAC-3'

T

390 400

T C A G A C C G G G A C T G C T T G G A C

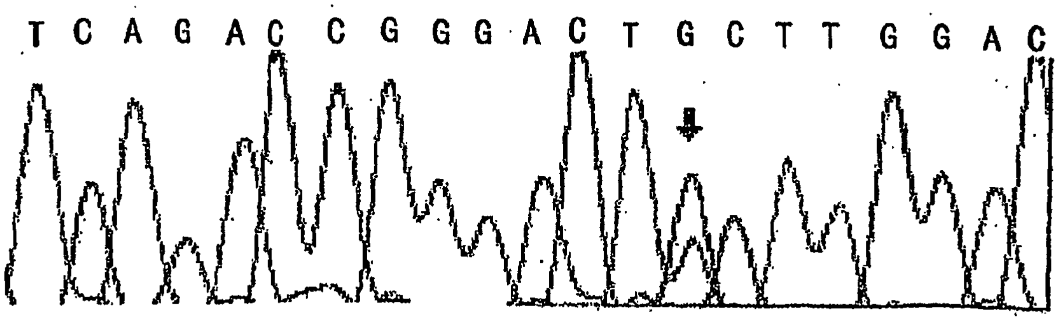

Fig. 20

110 115

Asp Arg Asp Arg Leu Asp Gly

5'-GAC CGG GAC CGC TTG GAC GGA-3'

391 400 410

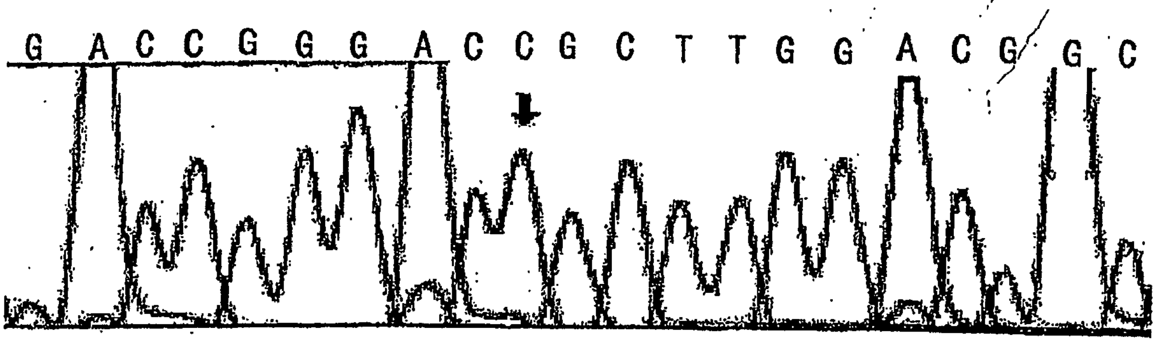


Fig. 21

110 115

Ser Asp Arg Asp Cys Leu Asp/Asn Gly Ser

G

5'-TCA GAC CGG GAC TGC TTG /AC GGC TCA-3'

A

390 400 410

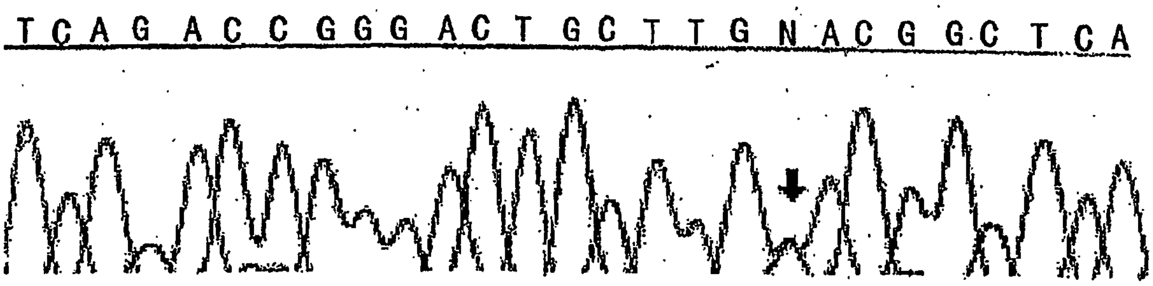

# Fig. 22

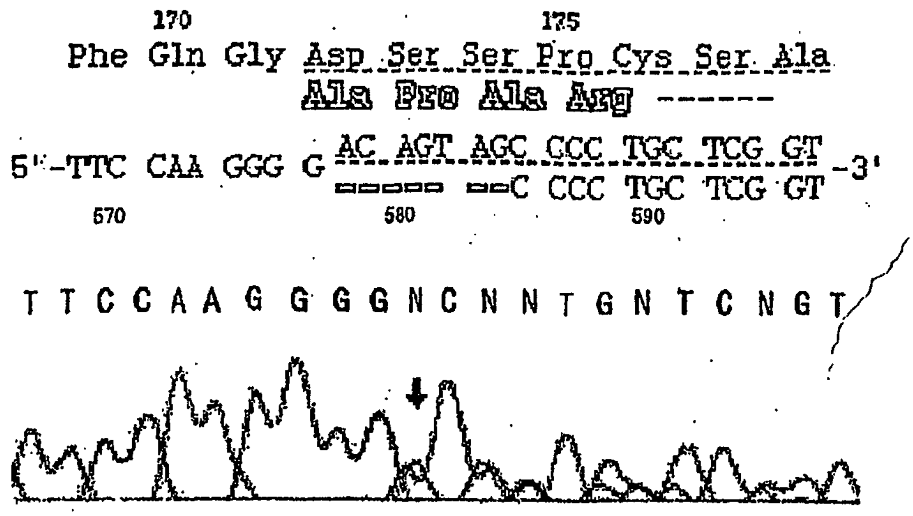


# Fig. 23

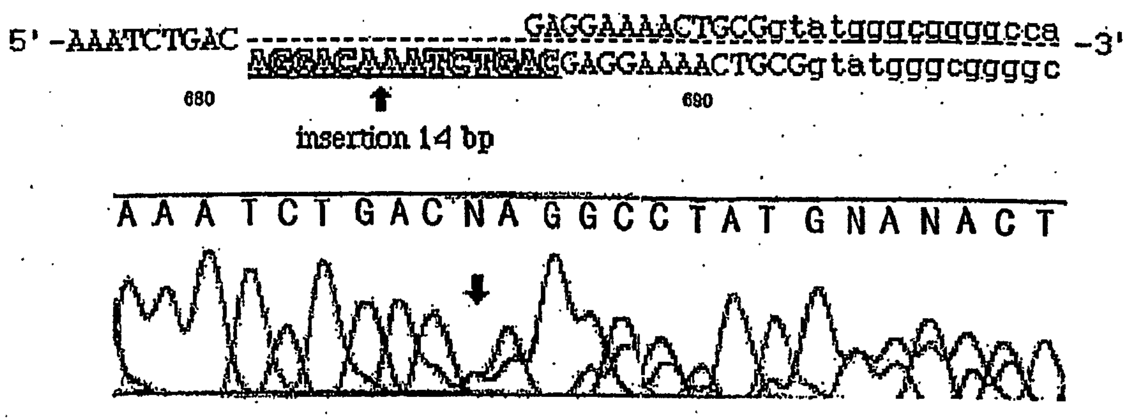


# Fig. 24

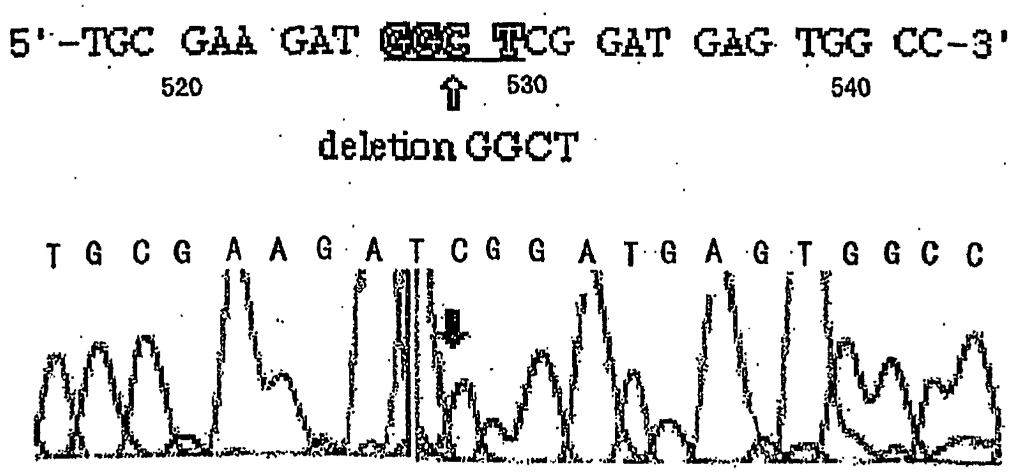

Fig. 25

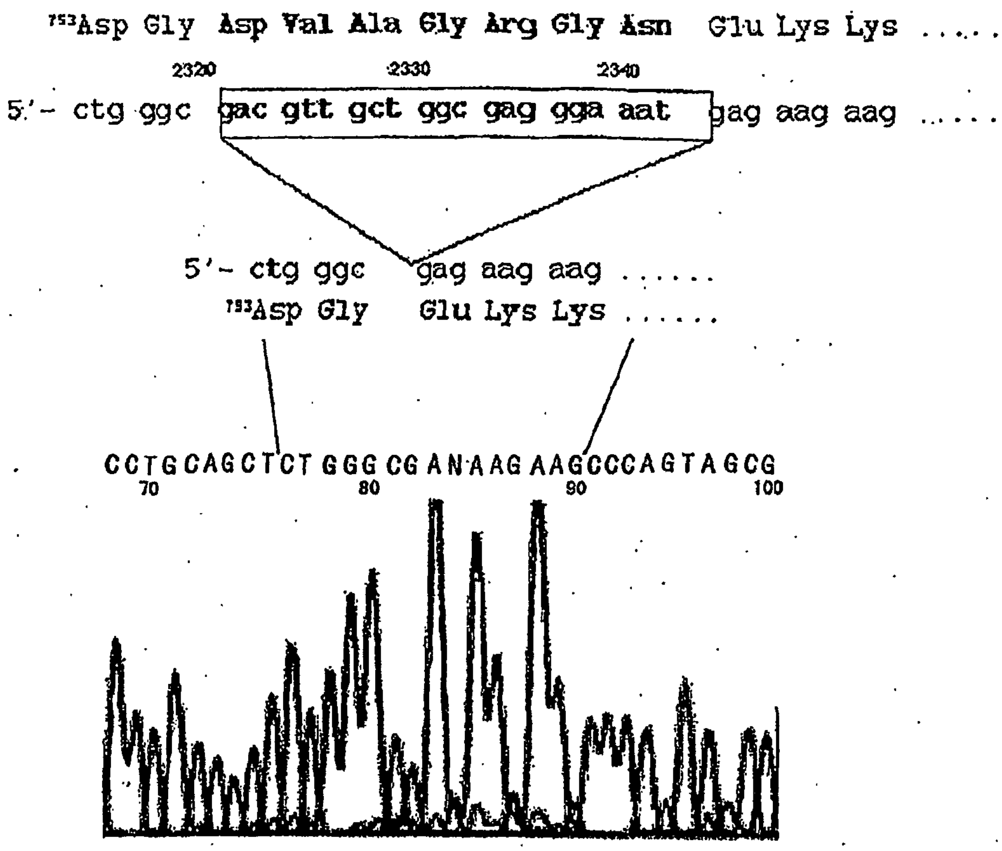
753Asp Gly Asp Val Ala Gly Arg Gly Asn Glu Lys Lys .....
2320 2330 2340
5'- ctg ggc gac gtt gct ggc gag gga aat gag aag aag .....
5'- ctg ggc gag aag aag ......
753Asp Gly Glu Lys Lys ......
CCTGCAGCTCTGGGCGANAAGAAGCCCAGTAGCG
70 80 90 100

Fig. 26

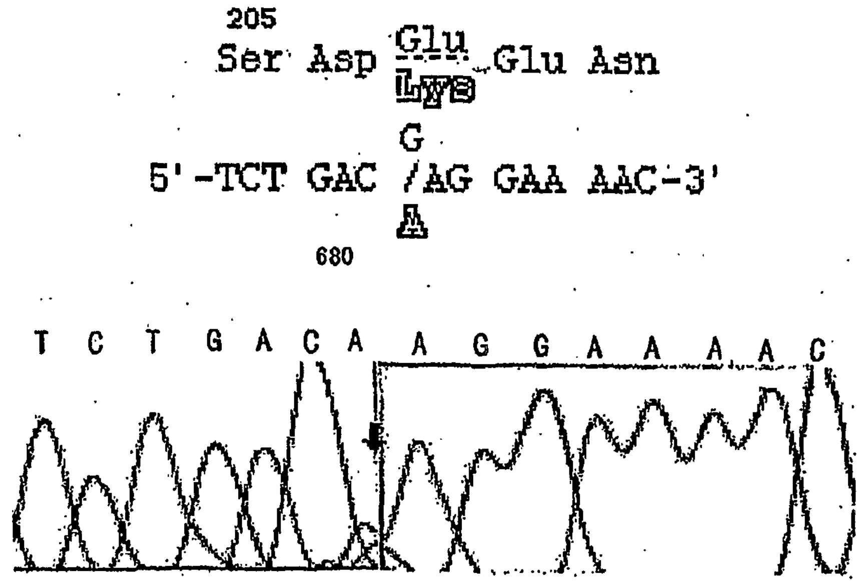
205
Ser Asp Glu Glu Asn
Lys
G
5'-TCT GAC /AG GAA AAC-3'
A
680
T C T G A C A A G G A A A A C

Fig. 27

205
Ser Asp Glu/Gln Glu Asn

G
5'-TCT GAC /AG GAA AAC-3'
C
680

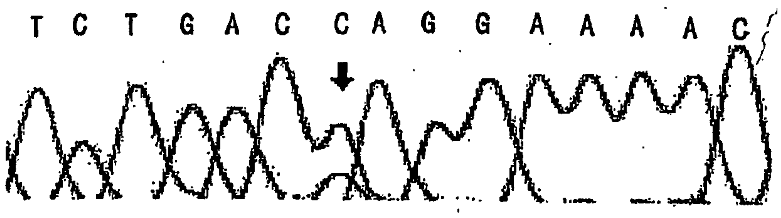


Fig. 28

3'-TCGTT GGTCCctgcgcag / TGGTCcctgcgca -5'
950 941

insertion A

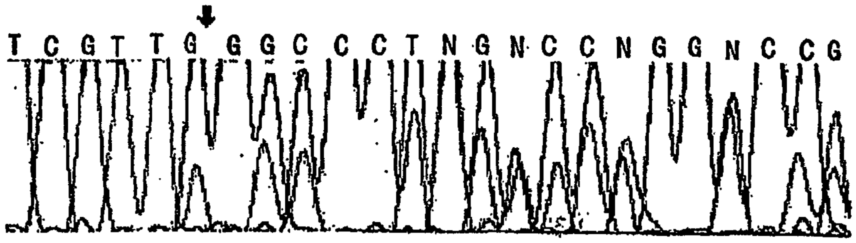


Fig. 29

3'-CCAAGCATTC GTTGGTCCct / GTTGGTCCct -5'
950 941

deletion C

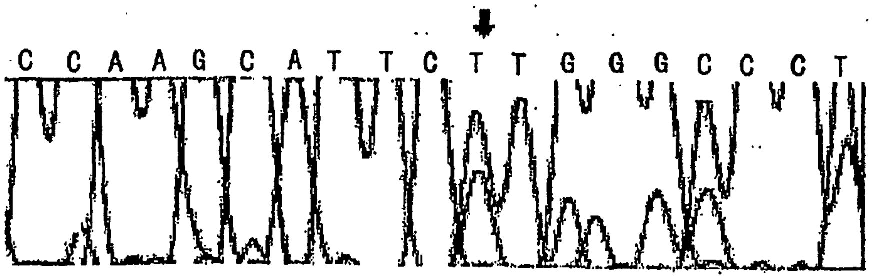

Fig. 30

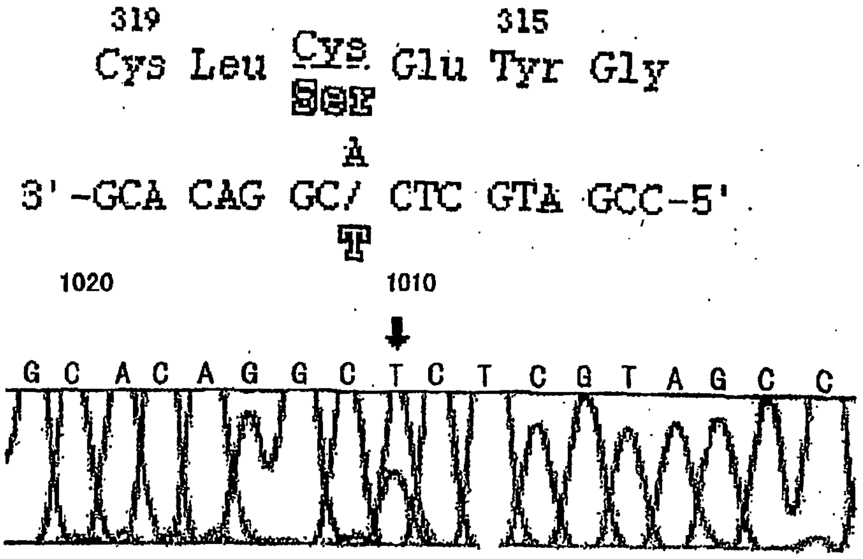
319
315
Cys Leu Cys Glu Tyr Gly
Ser
A
3'-GCA CAG GC/ CTC GTA GCC-5'
T
1020
1010
G C A C A G G C T C T C G T A G C C

Fig. 31

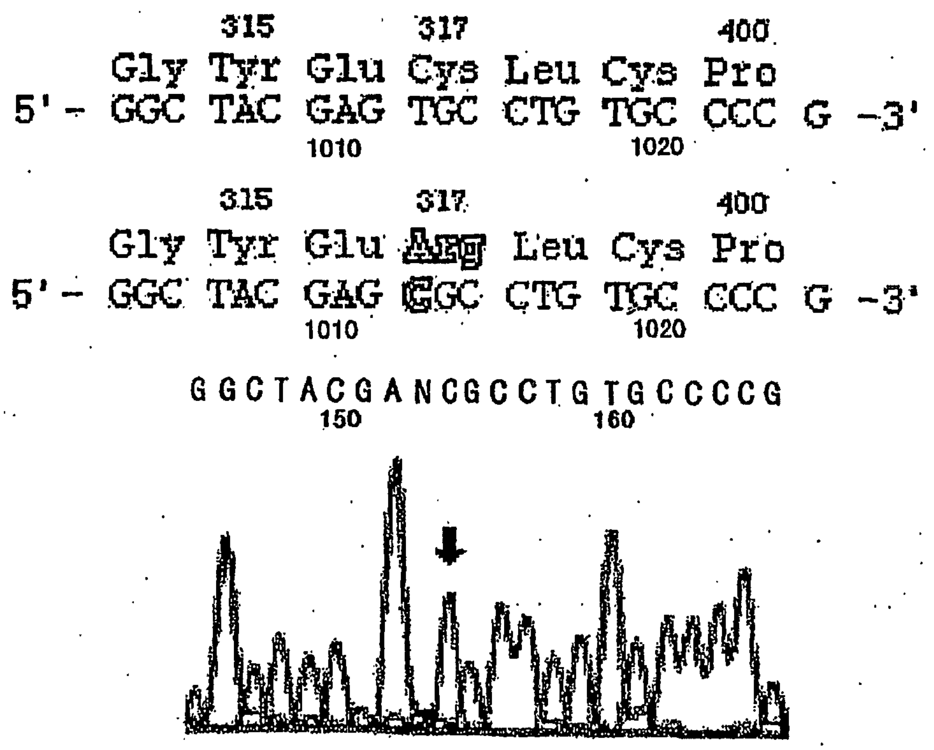
315
317
400
Gly Tyr Glu Cys Leu Cys Pro
5' - GGC TAC GAG TGC CTG TGC CCC G -3'
1010
1020
315
317
400
Gly Tyr Glu Arg Leu Cys Pro
5' - GGC TAC GAG CGC CTG TGC CCC G -3'
1010
1020
G G C T A C G A N C G C C T G T G C C C C G
150
160

# Fig. 32

1110 1120 1130
5'-GTGAACCTGGAGGGTGGCTACAAGTGCCAGTGT-3'
GAGGGTGGCTACAAGTGCCAGTGTGAGGAAGGCTTCCAGCT-3'
deletion 21bp

5'-GTGAACCTG GAGGGTGGCTACAAGTGCCAGTGT / TGTGAGGAAGGCTTCCAGCT -3'
1110 1120 1130

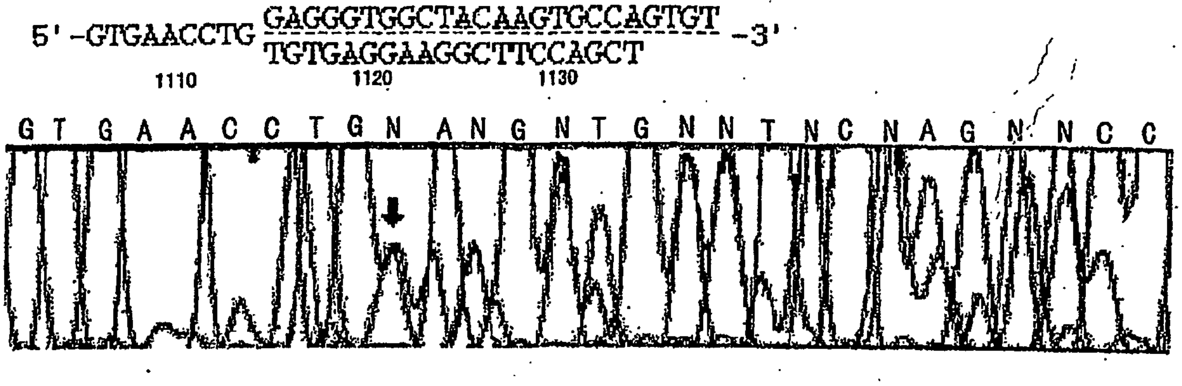


# Fig. 33

5'-ccagAT TAT CGA TGA GTG TCA GGA-3'
1061 1070
insertion T

3'-TCC TGA CAC TCA TCG ATA ATctgg-5'

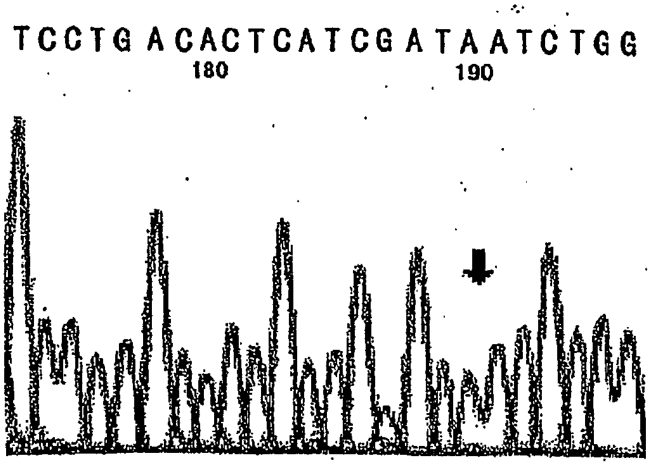

# Fig. 34

334

Ile Asp Glu Cys Gln Asp
Stop

G
5'-ATC GAT /AG TGT CAG GAT-3'
T

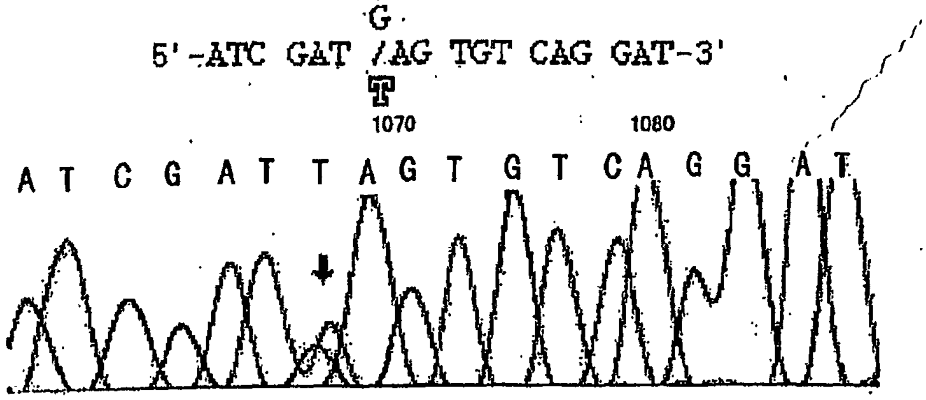


# Fig. 35

335

Asp Glu Cys Gln Asp Pro
Arg

T
5'-GAT GAG /GT CAG GAT CCC-3'
C

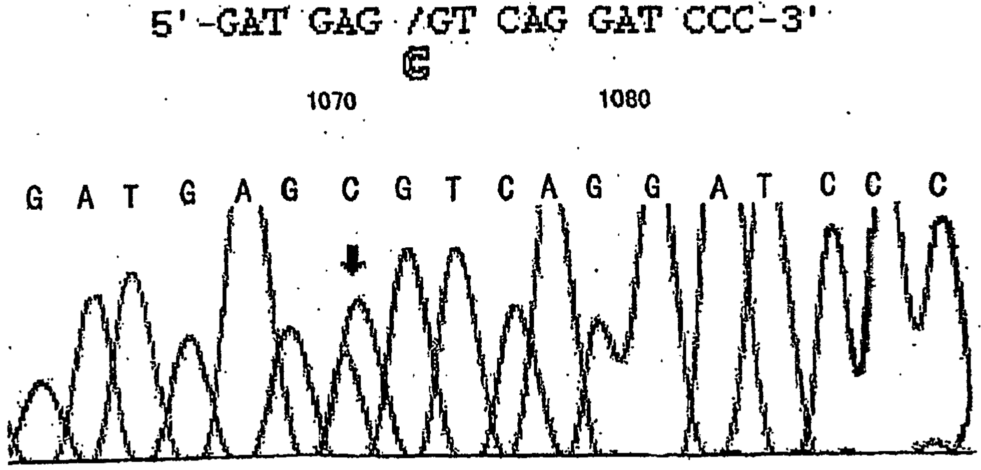

Fig. 36

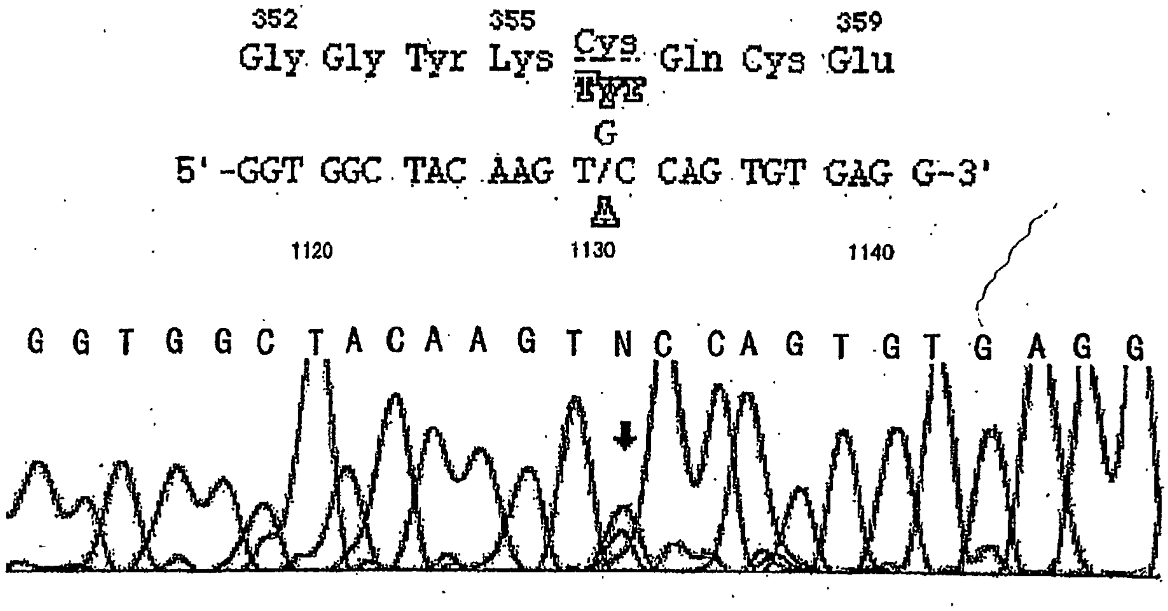
352 355 359
Gly Gly Tyr Lys Cys/Tyr Gln Cys Glu
G
5'-GGT GGC TAC AAG T/C CAG TGT GAG G-3'
A
1120 1130 1140
G G T G G C T A C A A G T N C C A G T G T G A G G

Fig. 37

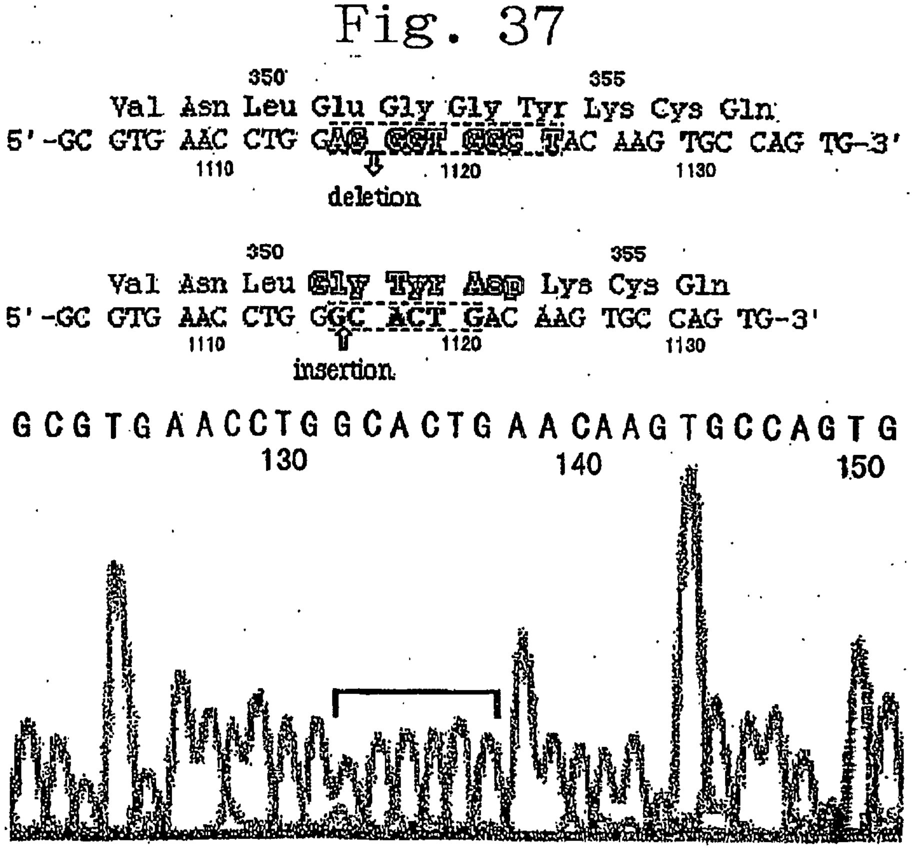
350 355
Val Asn Leu Glu Gly Gly Tyr Lys Cys Gln
5'-GC GTG AAC CTG GAG GGT GGC TAC AAG TGC CAG TG-3'
1110 1120 1130
deletion
350 355
Val Asn Leu Gly Tyr Asp Lys Cys Gln
5'-GC GTG AAC CTG GGC ACT GAC AAG TGC CAG TG-3'
1110 1120 1130
insertion
G C G T G A A C C T G G C A C T G A A C A A G T G C C A G T G
130 140 150

# Fig. 38

360 355

Gly Glu Glu Cys/Tyr Gln Cys Lys

G

3'-GCC TTC CTC A/A CTG GCA CTT-5'

T

1140 1130

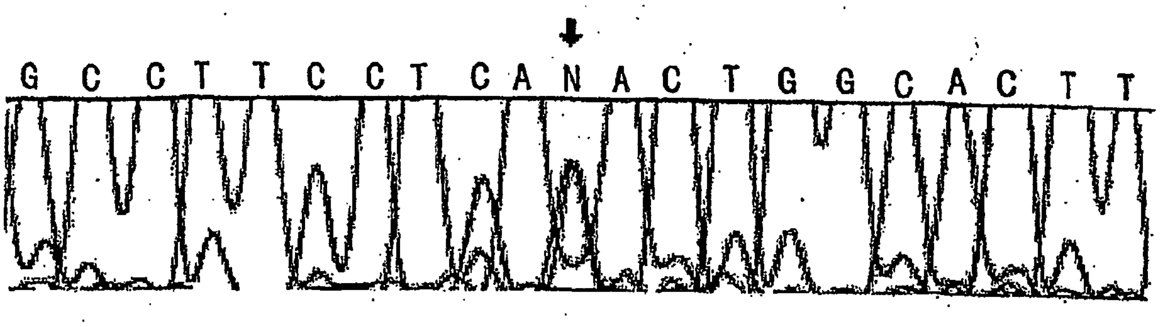


# Fig. 39

g

5'-gctcccc/gacccccagGC TCC A-3'

a

-10 -1 1190

c

3'-T GGA GCctgggggtc/ggggagc-5'

t

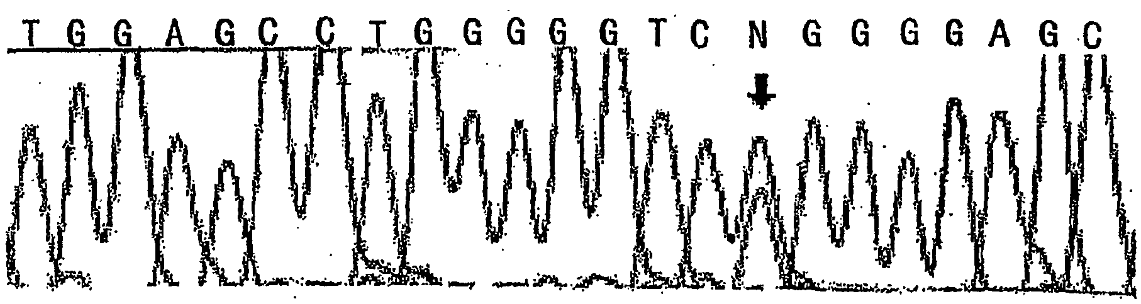

# Fig. 40

380 385
Tyr Leu Phe Phe Thr Asn Arg His
5'-TAC CTC TTC TTC ACC AAC CGG CAC-3'
1200 1210 1220

380 385
Tyr Leu Phe Leu Thr Asn Arg His
5'-TAC CTC TTC CTC ACC AAC CGG CAC-3'
1200 1210 1220

T A C C T C T T C C T C A C C A A C C G G C A C
120 130 140

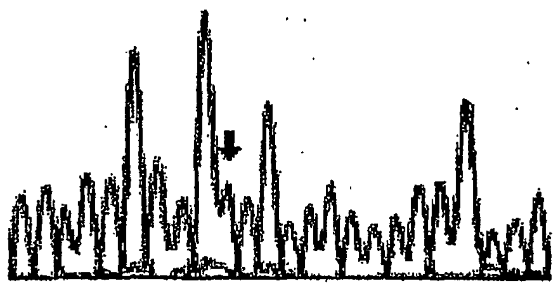

# Fig. 41

376 380
Ser Ile Ala Tyr Leu Phe Phe Thr
Ser Ile Ala Tyr Leu Phe Thr

5'-cagGC TCC ATC GCC ATC CTC TTC TTC ACC -3'
cagGC TCC ATC GCC ATC CTC TTC TTC ACC
1190 1200 1210
deletion TCT

3'-GGT GAA GA AGAGGTAGGCGATGGAGCctg -5'
GGTAGGCGATGGAGCctg

G G T G A A G A N G N N G G C G A T

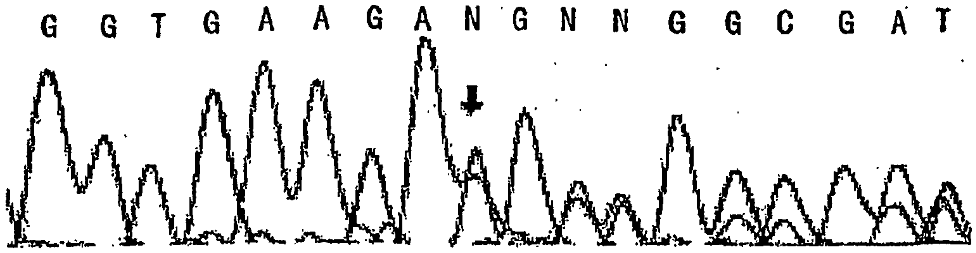

Fig. 42

382 385

Phe Thr Asn Arg/Trp His

C
5'-TTC ACC AAC /GG CAC G-3'
T

1210 1220

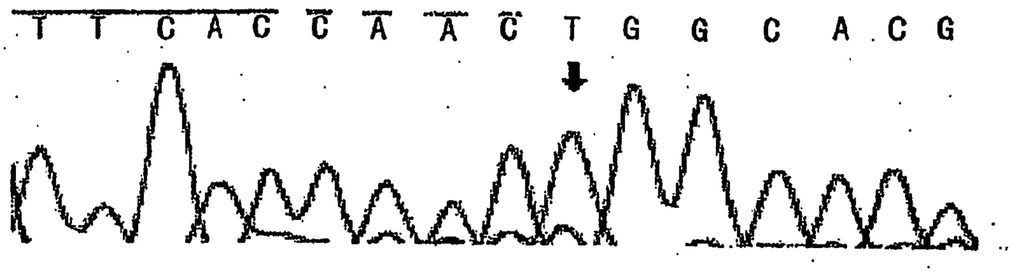


Fig. 43

385

Arg His Glu/Lys Val Arg

G
5'-AC CGG CAC /AG GTC AGG-3'
A

1220 1230

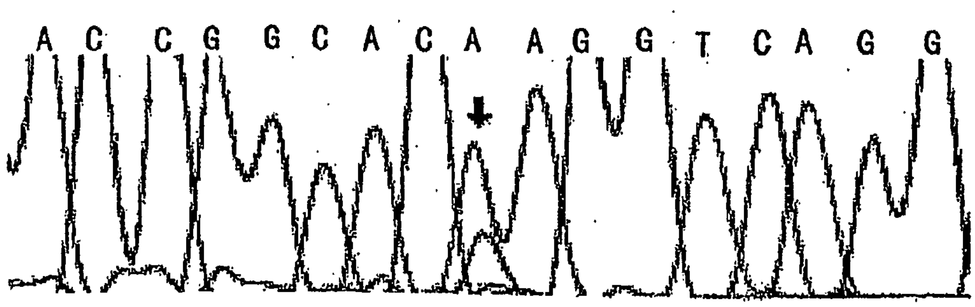

Fig. 44

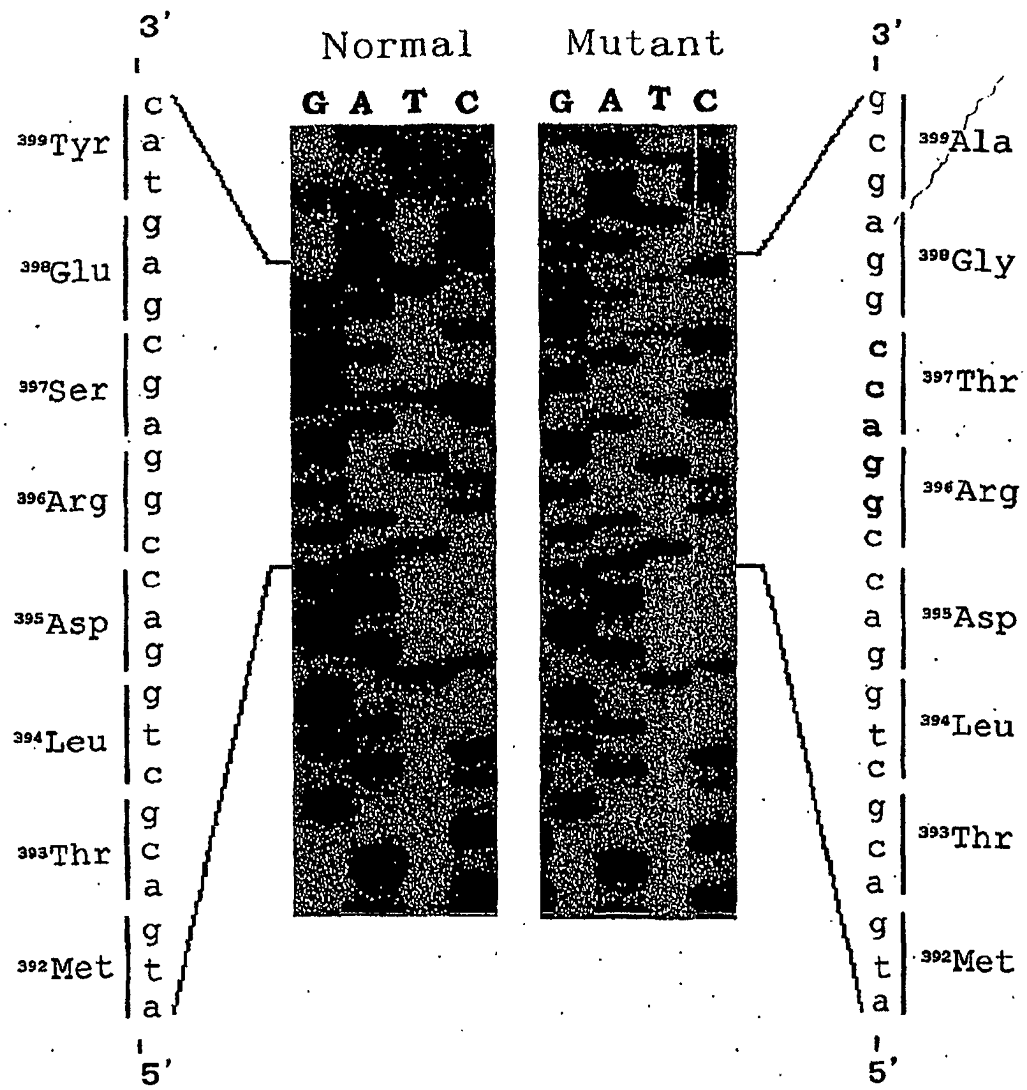
3'
Normal
Mutant
3'
G A T C
G A T C
399Tyr
398Glu
397Ser
396Arg
395Asp
394Leu
393Thr
392Met
c a t g a g c g a g g c c a g g t c g c a g t a
g c g a g g c c a g g c c a g g t c g c a g t a
399Ala
398Gly
397Thr
396Arg
395Asp
394Leu
393Thr
392Met
5'
5'

Fig. 45

400
Thr Ser Leu/Arg Ile Pro

T
5'-ACC AGC C/C ATC CCC-3'
G
1260 1270

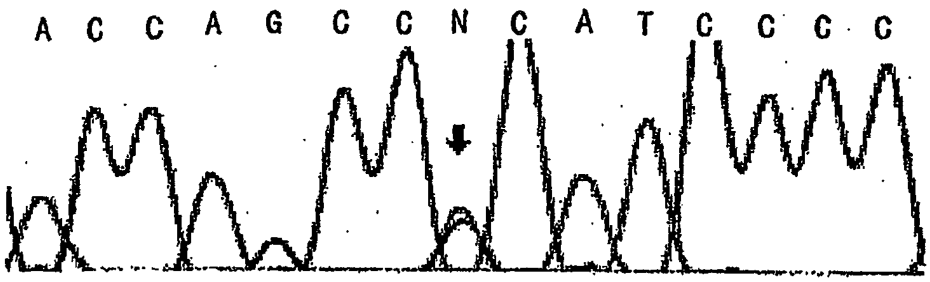


Fig. 46

410
Asn Val Val Ala Leu Asp Thr Glu
5'-AAC GTG GTC GCT CTG GAC ACG GAG-3'
1290 1300

410
Asn Val Val Thr Leu Asp Thr Glu
5'-AAC GTG GTC ACT CTG GAC ACG GAG-3'
1290 1300

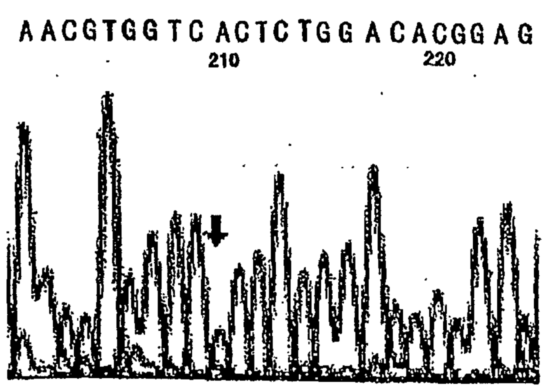

# Fig. 47

410

Val Ala Leu Asp Thr Glu
His

G
5'-GTC GCT CTG /AC ACG GAG-3'
C

1290 1300

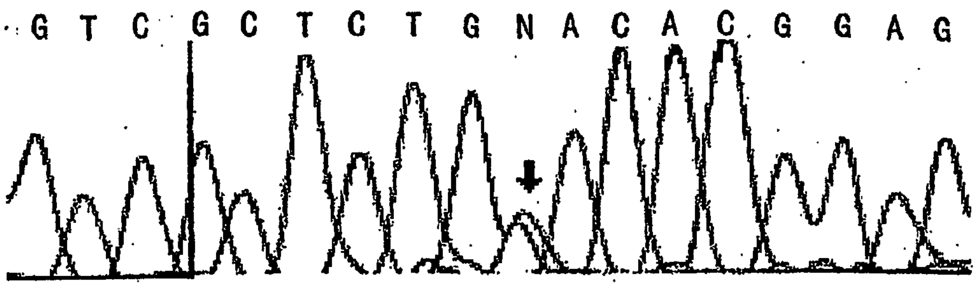


# Fig. 48

510 515
Met Tyr Trp Thr Asp Trp
stop

G
5'-ATG TAC TG/ ACT GAC TGG-3'
A

1591 1600

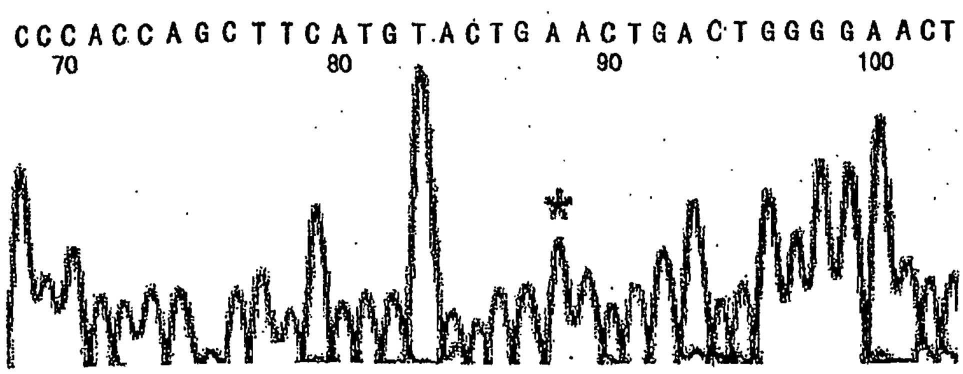

# Fig. 49

510 515

Phe Met Tyr Trp Thr Asp Trp Gly
stop

5'-C TTC ATG TAC TG G ACT GAC TGG GGA -3'
G ACT GAC TGG GGA A
1590 1600 1610

deletion G

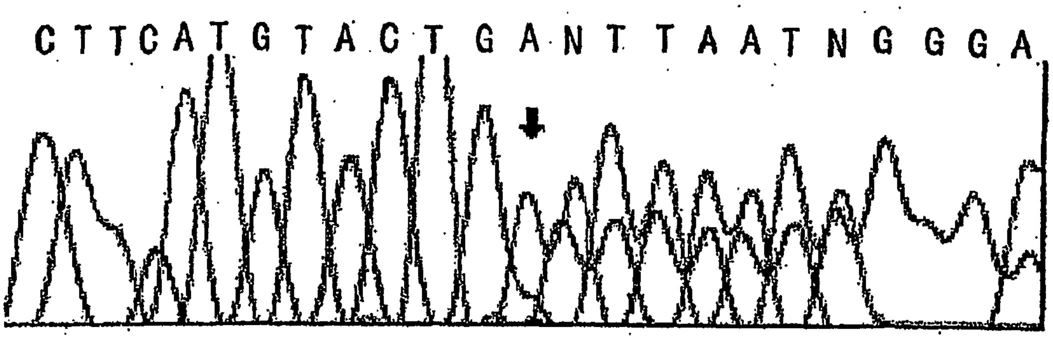


# Fig. 50

540 545
Gln Trp Pro Asn Gly Ile Thr Leu ---
Gln Trp His His Pro ---

5'-TT CAG TGG CCC AAT GGC ATC ACC CTA Gaa -3'
CAAT GGC ATC ACC CTA
1680 1690 1700

insertion C

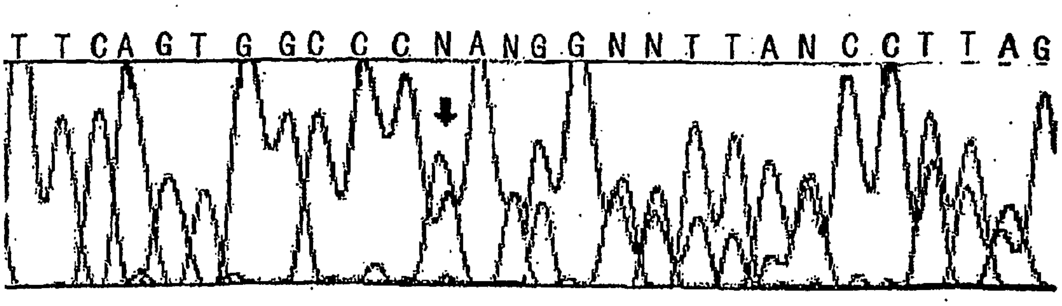

# Fig. 51

5'-GGT GTG GAC ATC TAC TCG CTG GTG ACT GAA-3'
1650 CTG GTG ACT GAA 1660 1670

⇧

deletion 11bp

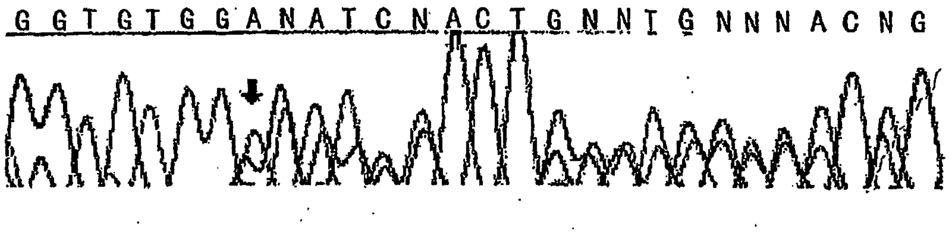


# Fig. 52

5'-GGT GTG GAC ATC TAC TCG CTG-3'
1650 1660

⇧

deletion T

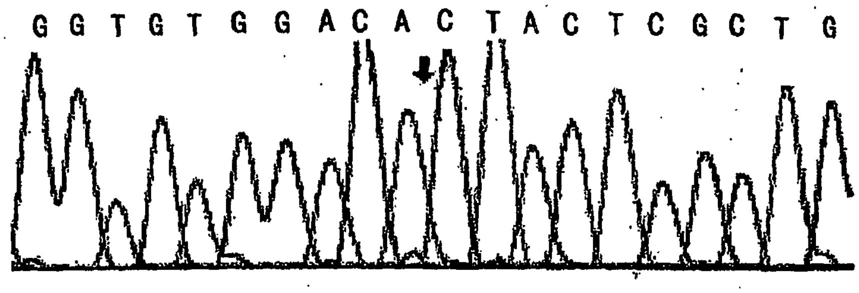


# Fig. 53

545
Gly Ile Thr Leu Asp
Val

C
5'-GGC ATC ACC /TA Ggtatg-3'
G
1700

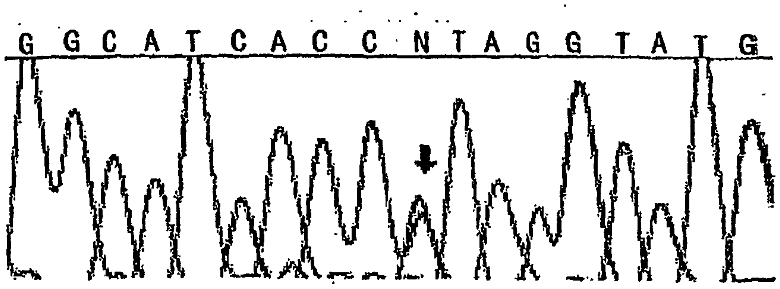

Fig. 54

exon 11 | intron 11

g
5'-CC CAG /tatgttcgc-3'
T
1705 +1

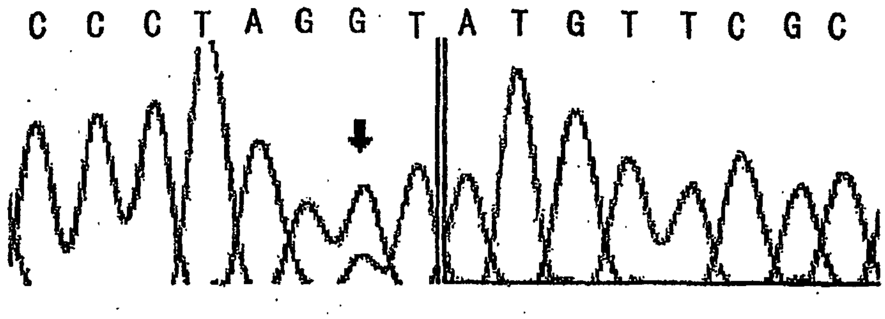


Fig. 55

exon 11← | →intron 11

g
5'-CC CAG /tatgttcgc-3'
T
1705 +1

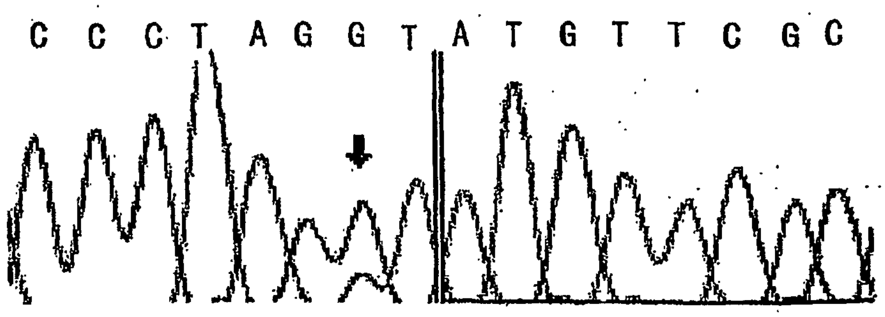

Fig. 56

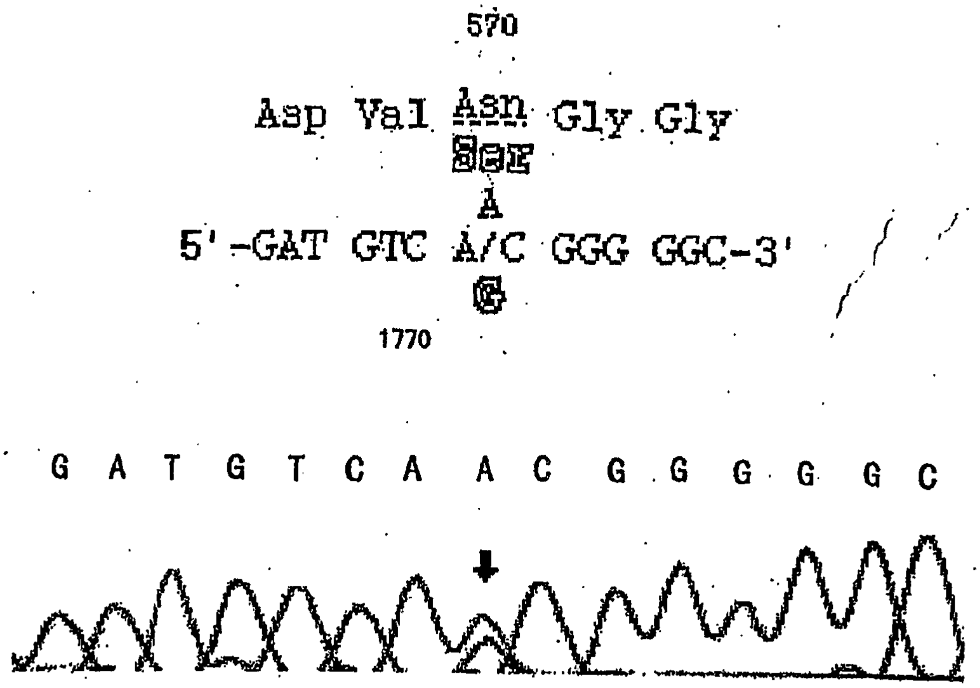
570
Asp Val Asn Gly Gly
Ser
A
5'-GAT GTC A/C GGG GGC-3'
G
1770
G A T G T C A A C G G G G G C

Fig. 57

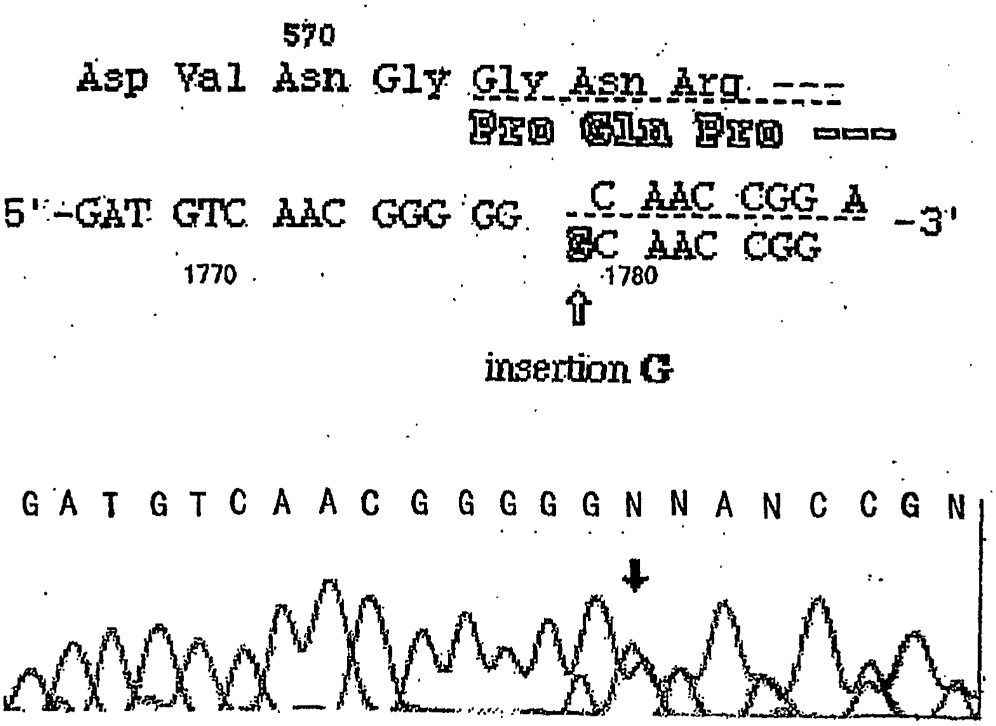
570
Asp Val Asn Gly Gly Asn Arg ---
Pro Gln Pro ---
5'-GAT GTC AAC GGG GG C AAC CGG A -3'
GC AAC CGG
1770
1780
insertion G
G A T G T C A A C G G G G G N N A N C C G N

Fig. 58

585 587

Arg Leu Ala/Ala His Pro/Ser Phe Ser

C C

5'-AGG CTG GC/ CAC /CC TTC TCC T-3'

T T

1810 1820 1830

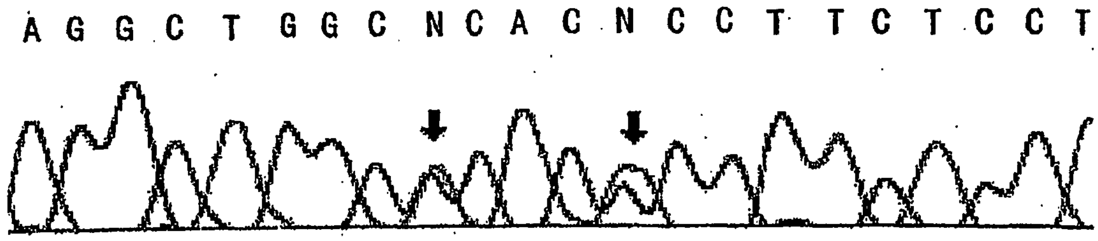


Fig. 59

590

Phe Ser Leu Ala/Ser Val Phe

G

5'-TTC TCC TTG /CC GTC TTT-3'

T

1830 1840

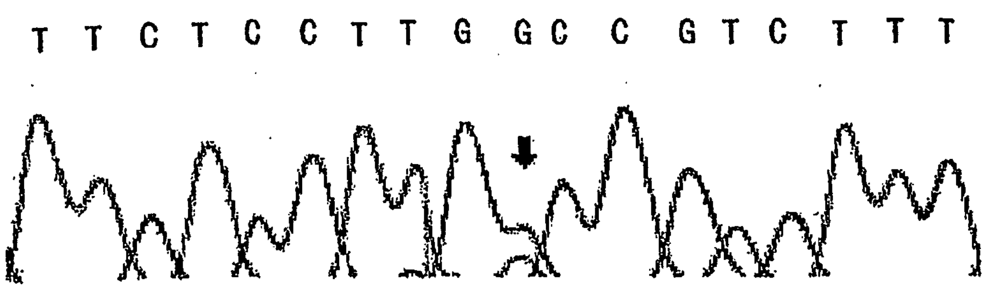

# Fig. 60

600 605

Phe Trp Thr Asp Ile Ile Asn Glu Ala Ile Phe Ser

Asn Glu Ala Ile Phe Ser

5'-TTT TGG ACA GAT ATC A TC AAC GAA GCC ATT TTC AGT GC -3'

TC AAC GAA GCC ATT TTC AGT GC

1860 1870 1880 1890

⇧

deletion TCA

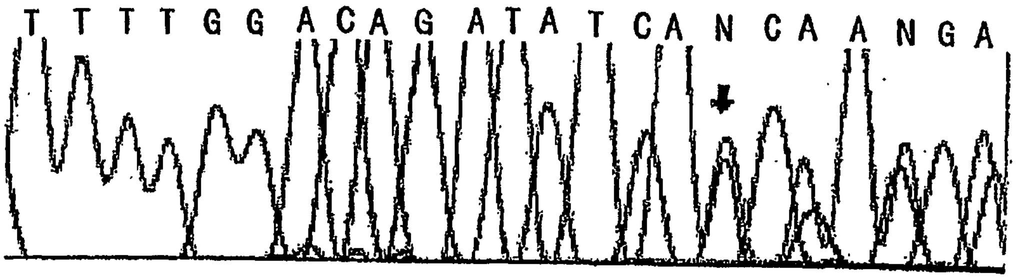


# Fig. 61

610

Ser Ala Asn Arg Leu Thr

Cys

C

5'-AGT GCC AAC /GC CTC ACA G-3'

T

1890 1900

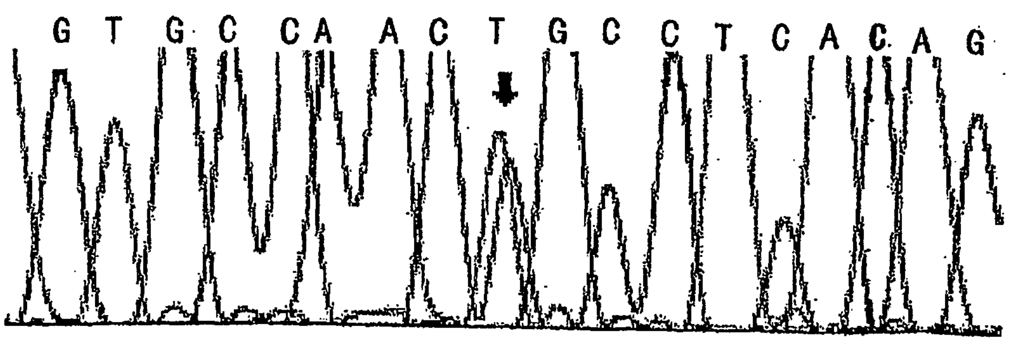

Fig. 62

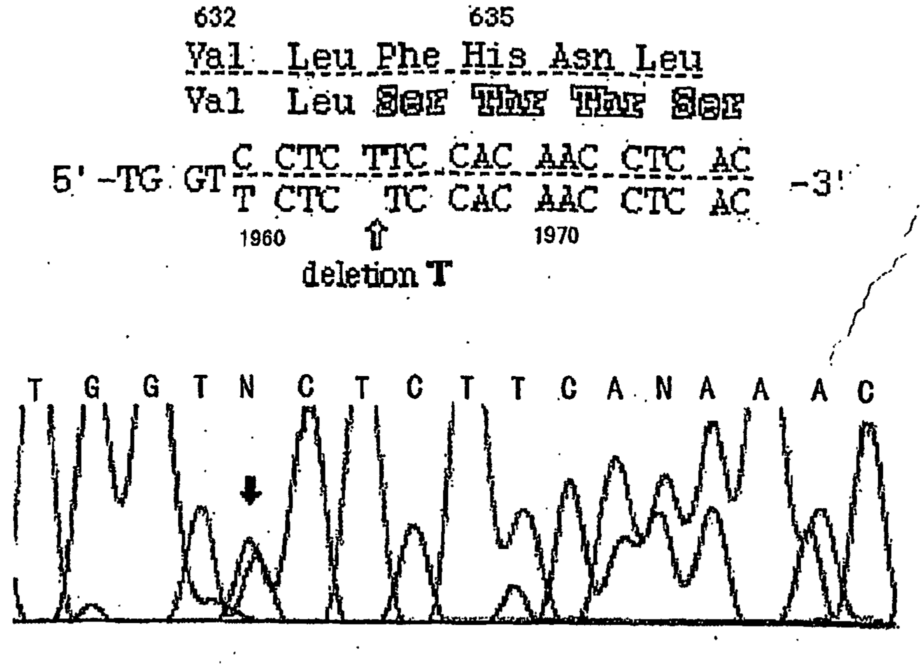
632 635
Val Leu Phe His Asn Leu
Val Leu Ser Thr Thr Ser
5'-TG GT C CTC TTC CAC AAC CTC AC -3'
T CTC TC CAC AAC CTC AC
1960 1970
deletion T
T G G T N C T C T T C A N A A A C

Fig. 63

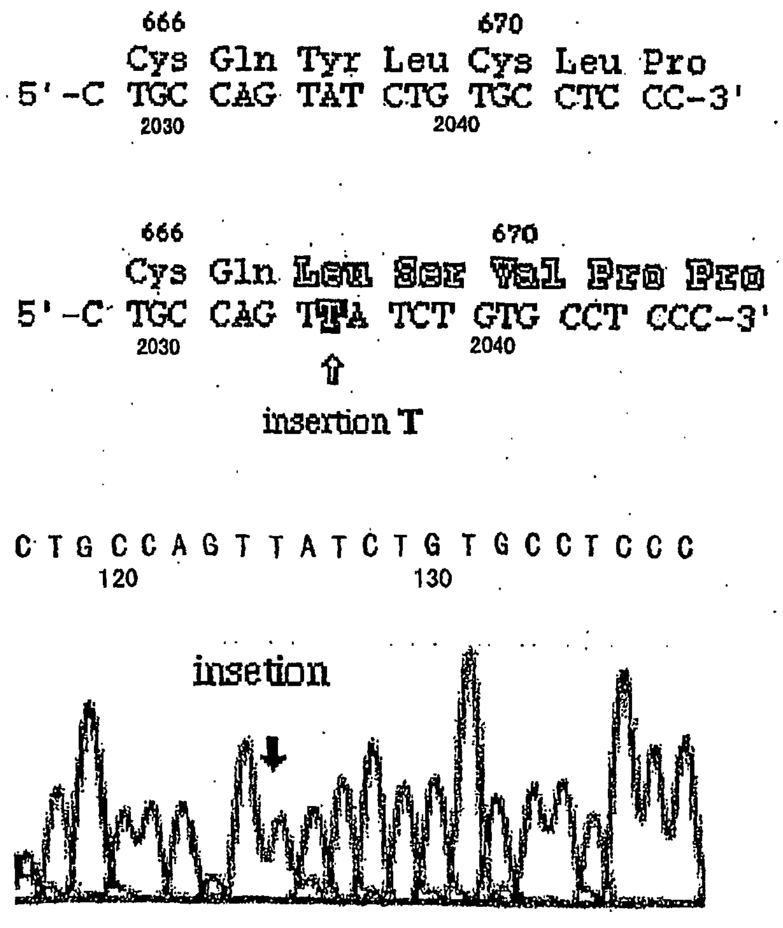
666 670
Cys Gln Tyr Leu Cys Leu Pro
5'-C TGC CAG TAT CTG TGC CTC CC-3'
2030 2040
666 670
Cys Gln Leu Ser Val Pro Pro
5'-C TGC CAG TTA TCT GTG CCT CCC-3'
2030 2040
insertion T
C T G C C A G T T A T C T G T G C C T C C C
120 130
insetion

# Fig. 64

690
Cys Leu Thr Glu / Lys

G
5'-C TGC CTC ACA /gtgtggca-3'
A

2130 2140

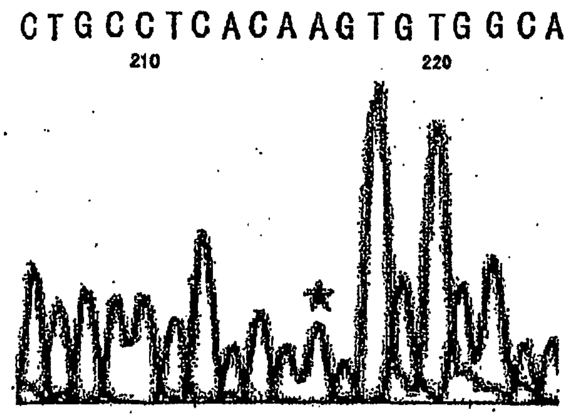


# Fig. 65

751 755 760
Leu Gly Asp Val Ala Gly Arg Gly Asn Glu Lys Lys

5'-CTG GGC GAC GTT GCT GGC CAG GGA AAT GAG AAG AAG-3'

2320 2330 2340

deletion 21bp

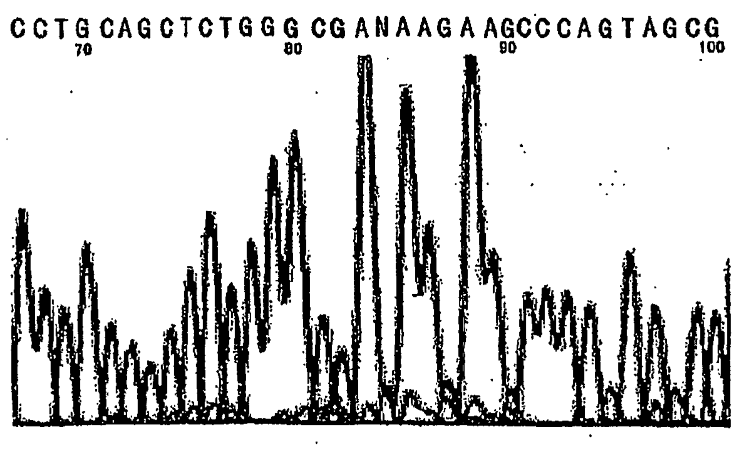

Fig. 66

780
Leu Leu Val/Ile Phe Leu Cys

G
5'-CTC CTC /TC TTC CTT TGC-3'
A
2400

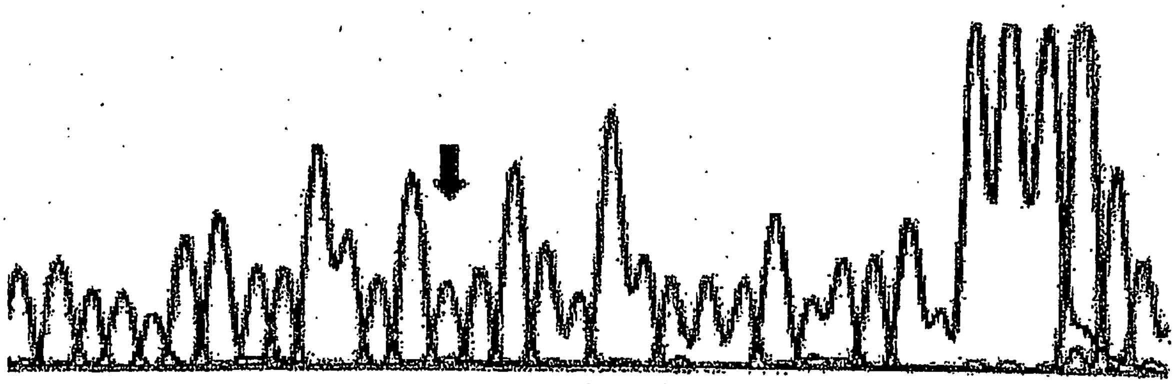

Fig. 67

790
Leu Trp Lys/Stop Asn

A
5'-CTA TGG /AG AAC T-3'
T
2430

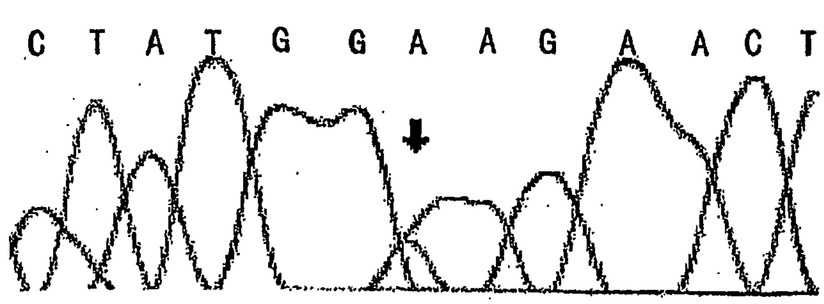

Fig. 68

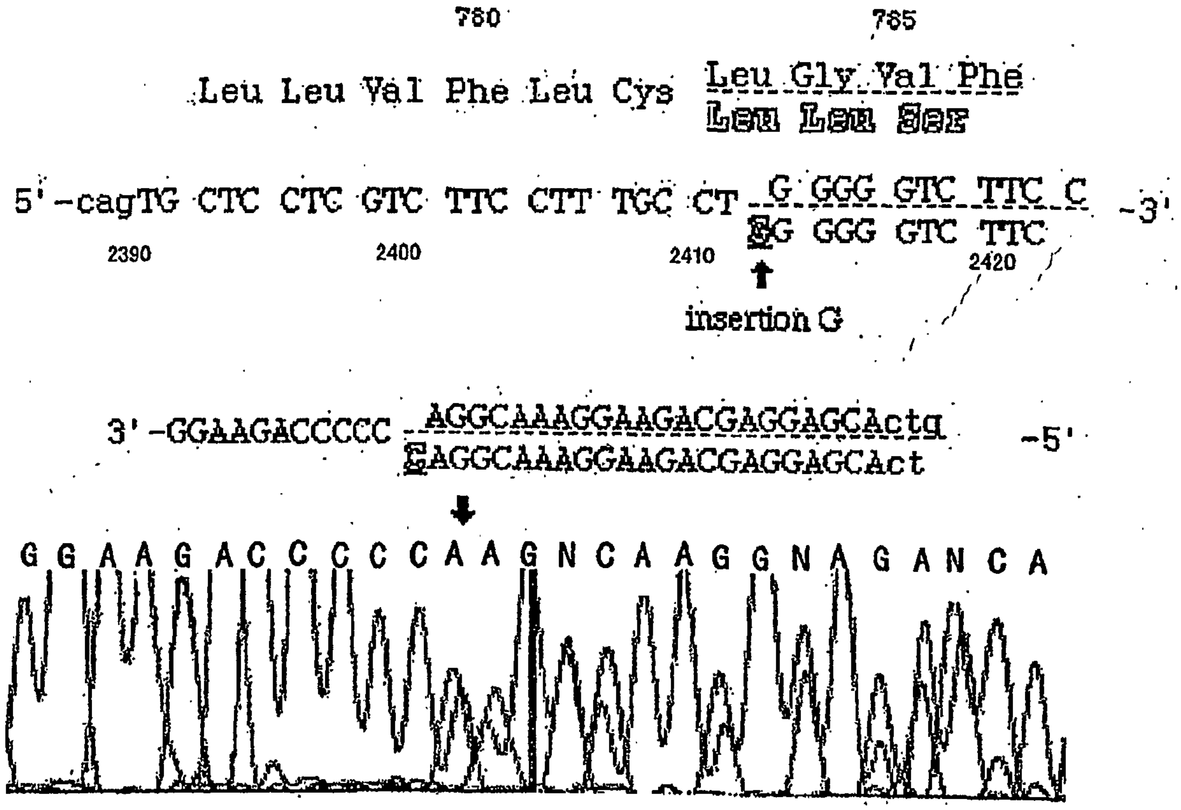

760
765
Leu Leu Val Phe Leu Cys Leu Gly Val Phe
Leu Leu Ser
5'-cagTG CTC CTC GTC TTC CTT TGC CT G GGG GTC TTC C -3'
GG GGG GTC TTC
2390
2400
2410
2420
insertion G
3'-GGAAGACCCCC AGGCAAAGGAAGACGAGGAGCActg -5'
GAGGCAAAGGAAGACGAGGAGCAct
G G A A G A C C C C C A A G N C A A G G N A G A N C A


Fig. 69

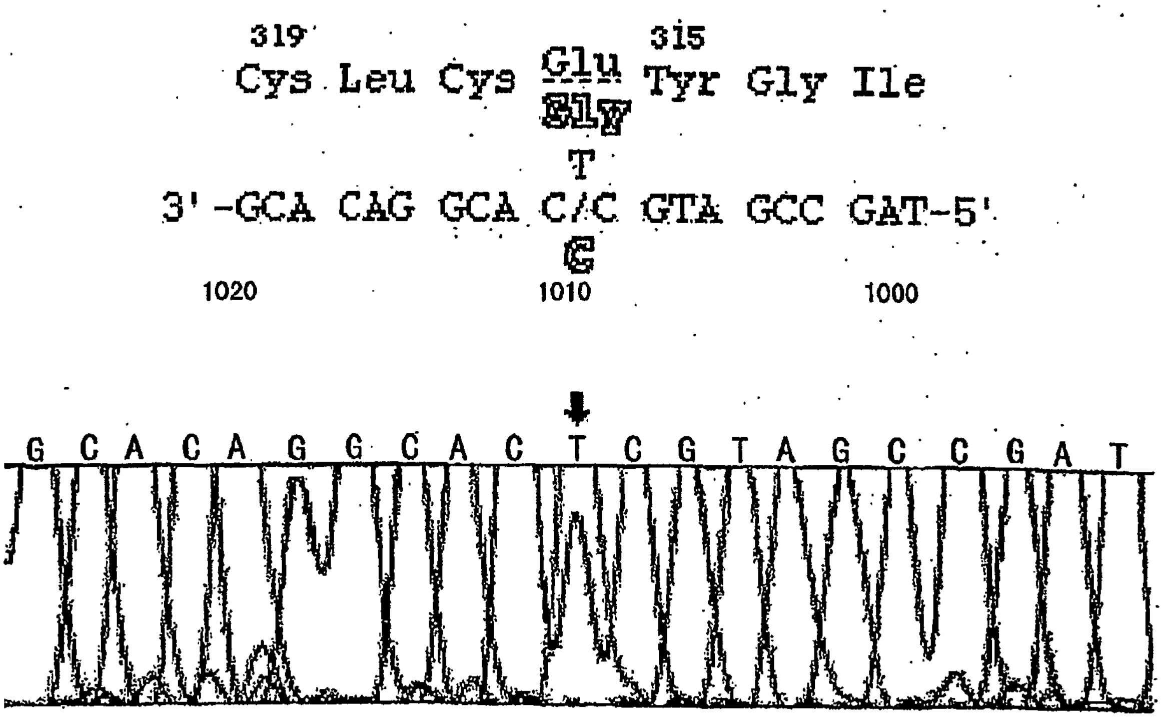

319
315
Cys Leu Cys Glu Tyr Gly Ile
Gly
T
3'-GCA CAG GCA C/C GTA GCC GAT-5'
G
1020
1010
1000
G C A C A G G C A C T C G T A G C C G A T

# Fig. 70

5

Gly Asp Arg Cys/Cys Glu Arg

T

5'-G GGC GAC AGA TG/ GAA AGA-3'

C

70 80

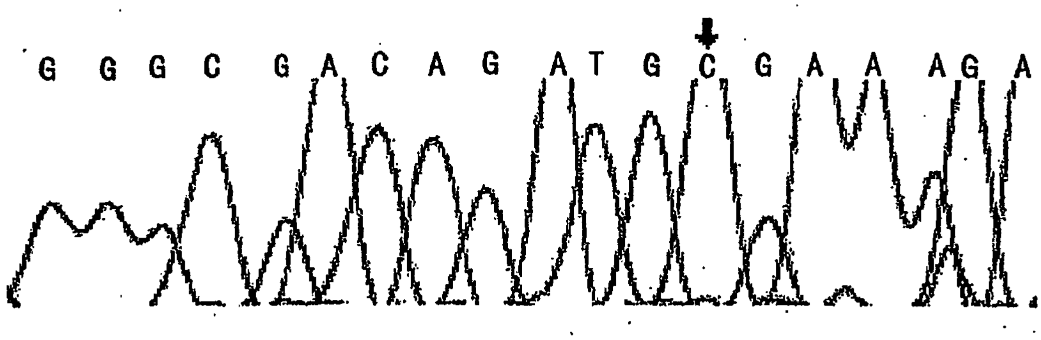


# Fig. 71

190

Ile His Ser/Ser Ser Trp Arg

C

5'-ATC CAC TC/ AGC TGG CGC-3'

T

630 640

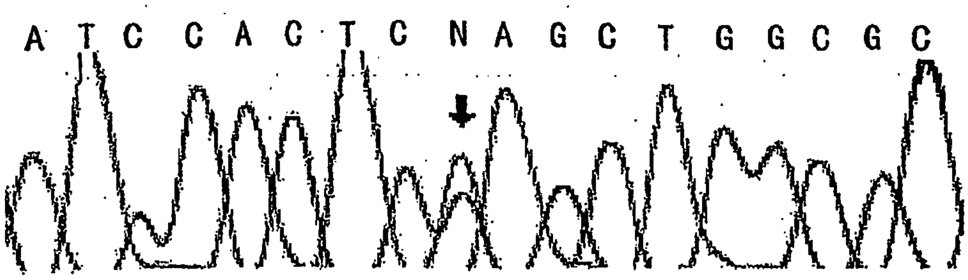

Fig. 72

300

Asn Asn Gly Gly Cys Ser

5'-AAC AAC GGC GGC TGT TC-3'

965 970 975

300

Asn Asn Gly Gly Cys Ser

5'-AAC AAC GGT GGC TGT TC-3'

965 970 975

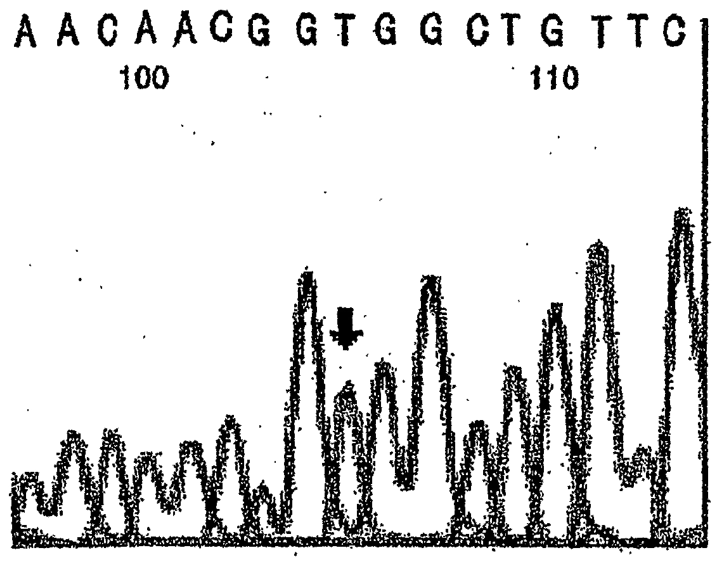

# Fig. 73

310 315
Asp Leu Lys Ile Gly Tyr Glu
5'-GAC CTT AAG ATC GGC TAC GAG-3'
1000 1010

310 315
Asp Leu Lys Ile Gly Tyr Glu
5'-GAC CTT AAG ATT GGC TAC GAG-3'
1000 1010

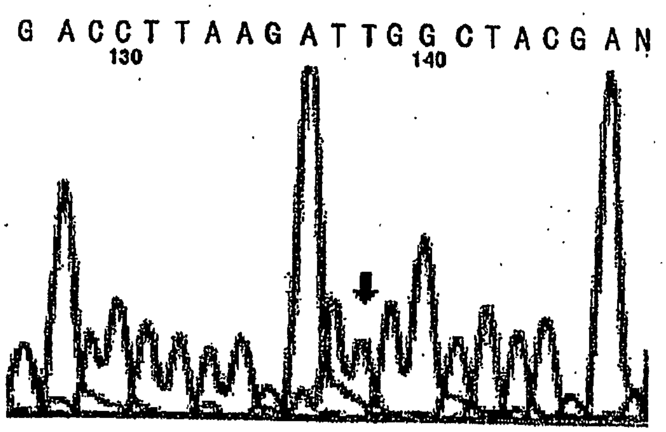

# Fig. 74

376 380
Ser Ile Ala Tyr Leu Phe
5'-cgag GC TCC ATC GCC TAC CTC TTC TT-3'
1190 1200

376 380
Ser Ile Ala Tyr Leu Phe
5'-cgag GC TCC ATT GCC TAC CTC TTC TT-3'
1190 1200

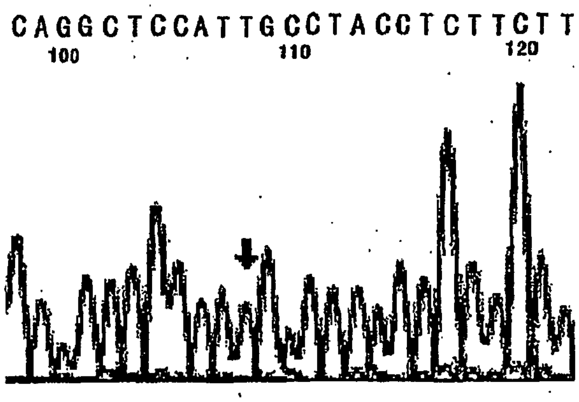


# Fig. 75

552
Gly Arg Leu Tyr Trp
Leu

C
5'-GGC CGC CT/ TAC TGG G-3'
T
1720 1730

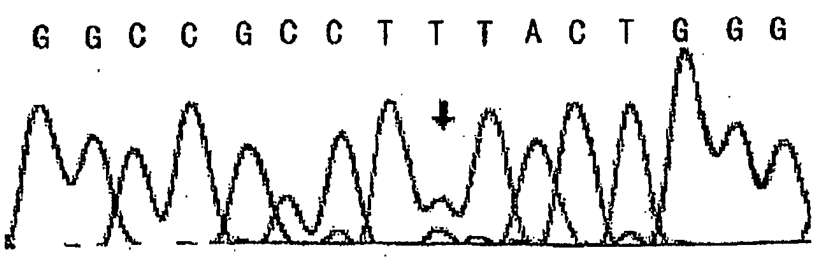

Fig. 76

570

Val Asn Gly Gly Asn
Asn

T
5'-GTC AA/ GGG GGC AAC C-3'
C

1770 1780

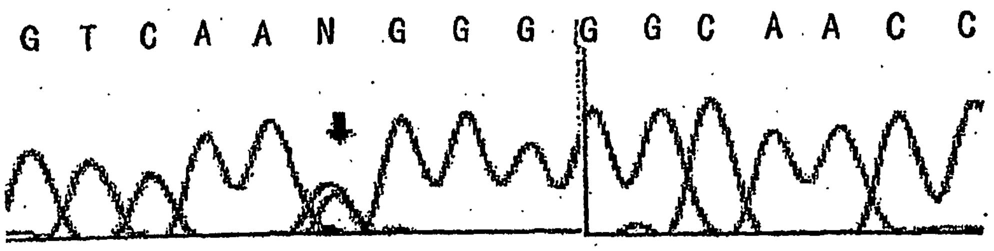


Fig. 77

631
Met Val Leu Phe
Val

C
5'-ATG GT/ CTC TTC-3'
T

1960

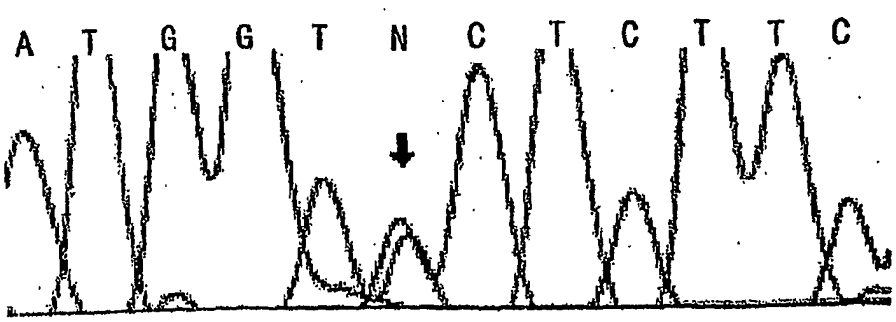

# INTERNATIONAL SEARCH REPORT

International application No.
PCT/JP01/06153

A. CLASSIFICATION OF SUBJECT MATTER
Int.Cl$^7$ C12N15/09, C12Q1/68

According to International Patent Classification (IPC) or to both national classification and IPC

B. FIELDS SEARCHED

Minimum documentation searched (classification system followed by classification symbols)
Int.Cl$^7$ C12N15/09, C12Q1/68

Documentation searched other than minimum documentation to the extent that such documents are included in the fields searched

Electronic data base consulted during the international search (name of data base and, where practicable, search terms used)
SwissProt/PIR/GeneSeq, GenBank/EMBL/DDBJ/GeneSeq,
CA (STN), REGISTRY (STN), WPI (DIALOG), BIOSIS (DIALOG), MEDLINE (STN)

C. DOCUMENTS CONSIDERED TO BE RELEVANT

| Category* | Citation of document, with indication, where appropriate, of the relevant passages | Relevant to claim No. |
|---|---|---|
| X | Yasuji KAJIHARA, "LDL Receptor to Sono Ligand, Doumyaku Kouka", March, 2000, Vol.27, Nos. 4 to 5, pages 93 to 98 | 1-3 |
| X | Yasuji KAJIHARA, "Shuyou Byoutai no Idenshi Ijou to Sono Rinshou-zou; Kazoku-sei Kou-Cholesterol Kesshou", Nihon Rinshou, December, 1999, Vol.57, No.12, pages 2770 to 2775 | 1-3 |
| X | Toshinori TOHO, "Nihonjin Kazoku-sei Kou-Cholesterol Kesshou ni okeru Teihijuu Lipo-Tanpaku Juyoutai Idenshi Heni to Sono Hindo ni Kansuru Kenkyuu", Kanazawa Daigaku Juu Zen Igakukai Zasshii, April, 1997, Vol. 106, No. 2, pages 257 to 275 | 1-3 |
| X | Masahiro TSUJI et al., "Aratana LDL Juyoutai Idenshi Heni wo mitometa Kazoku-sei Kou-Cholesterol Kesshou Gappei Tounyou-byou no 1 rei, Diabetes Journal, December, 1999, Vol.27, No.4, pages 142 to 145 | 1-3 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 September, 2001 (26.09.01) | 09 October, 2001 (09.10.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

INTERNATIONAL SEARCH REPORT

International application No.
PCT/JP01/06153

C (Continuation). DOCUMENTS CONSIDERED TO BE RELEVANT

| Category* | Citation of document, with indication, where appropriate, of the relevant passages | Relevant to claim No. |
|---|---|---|
| X | HIRAYAMA, T. et al., "Five familial hypercholesterolemic kindreds in Japan with novel mutations of the LDL receptor gene", J. Hum. Genet., (1998), Vol.43, No.4, pages 250 to 254 | 1-3 |
| X | WO 86/04090 A1 (University of Texas System), 17 July, 1986 (17.07.86), & EP 205574 A1 & US 4745060 A & JP 62-501327 A | 1-3 |

Form PCT/ISA/210 (continuation of second sheet) (July 1992)

**INTERNATIONAL SEARCH REPORT**

International application No.
PCT/JP01/06153

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

See extra sheet.

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:

An international search report has been prepared partly on a method of detecting lipid metabolic errors whereby risk factors for lipid metabolic errors are detected based on the relation of gene mutations concerning "1. gene mutations at the site encoding cysteine at the 25-position of low density lipoprotein receptor protein encoded by low density lipoprotein receptor gene" to lipid metabolic errors, and inventions corresponding to these gene mutations among the inventions as set forth in claims 2 and 3. See extra sheet as well.

**Remark on Protest** ☐ The additional search fees were accompanied by the applicant's protest.
☐ No protest accompanied the payment of additional search fees.

INTERNATIONAL SEARCH REPORT

International application No.
PCT/JP01/06153

Continuation of Box No. II of Continuation of first sheet (1)

Considering the statement in the description, the gene mutations 1 to 65 as set forth in claim 1 are a group of gene mutations to be noted as LDL receptor gene abnormalities in Japanese familial hypercholesterolemia (FH). It is stated that a risk factor for lipid metabolic errors can be detected by detecting an arbitrary one of these mutations and risk factor(s) of one or both of arteriosclerosis and ischemic heart diseases can be detected by using the lipid metabolic errors thus detected as indications.

However, a number of LDL receptor gene mutations in Japanese FH had been publicly known before the priority date of the present case. Also, it had been publicly known before the priority date of the present case to detect lipid metabolic errors such as FH by noting these mutations. Moreover, arteriosclerosis and ischemic heart diseases caused by FH had been a common technical knowledge for those skilled in the art before the priority date of the present case and it had been self-evident for those skilled in the art to anticipate the degree of the risk for arteriosclerosis or ischemic heart diseases by using lipid metabolic errors relating to FH as indications. (Refer to the following references, if necessary).

Taking the statement in the description (in particular, Examples) of the present case into consideration, the 65 gene mutations as described above are found out merely by comparing the base sequence of the normal LDL receptor gene with the base sequences of LDL receptor genes of 73 patients clinically diagnosed as suffering from FH. Therefore, it cannot be considered that the viewpoint of noting these 65 gene mutations is particularly superior to the viewpoint of using LDL receptor gene mutations in detecting arteriosclerosis, ischemic heart diseases, etc. which had been publicly known before the priority date of the present case.

Such being the case, these 65 mutations cannot be regarded as having a common technical feature contributing to the prior art and, therefore, are not considered as being so linked as to form a single general inventive concept.

Therefore, it is recognized that 65 inventions failing to satisfy the reluirement of unity of invention are described in claim 1.

Thus, the international searching authority has prepared an international search report partly on a method of detecting lipid metabolic errors whereby risk factors for lipid metabolic errors are detected based on the relation of gene mutations concerning "1. gene mutations at the site encoding cysteine at the 25-position of low density lipoprotein receptor protein encoded by low density lipoprotein receptor gene" to lipid metabolic errors, and inventions corresponding to these gene mutations among the inventions as set forth in claims 2 and 3.

An international search report has been prepared partly on a method of detecting lipid metabolic errors whereby risk factors for lipid metabolic errors are detected based on the relation of gene mutations concerning "1. gene mutations at the site encoding cysteine at the 25-position of low density lipoprotein receptor protein encoded by low density lipoprotein receptor gene" to lipid metabolic errors, and inventions corresponding to these gene mutations among the inventions as set forth in claims 2 and 3.

Form PCT/ISA/210 (extra sheet) (July 1992)

**INTERNATIONAL SEARCH REPORT**

International application No.
PCT/JP01/06153

Continuation of Box No. II of Continuation of first sheet (1)

Reference:

Koji KAJIHARA, LDL receptor and its ligand, Domyakukoka, March 2000, vol. 27, Nos.4-5, pp.93-98

Koji KAJIHARA, Gene abnormalities in major pathologic conditions and clinical data --- Familial hypercholesterolemia, Nippon Rinsho, December 1999, vol. 57, No.12, pp.2770-2775

Toshinori TOHO, Study on low density lipoprotein receptor gene mutations and frequencies thereof in Japanese familial hypercholesterolemia, Juzzen Igakukaishi, Kanazawa University, April 1997, Vol. 106, No.2, pp.257-275

Masahiro TSUJI, et al., A case of diabetes complicated with familial hypercholesterolemia showing novel LDL receptor gene mutation, Diabetes Journal, December 1999, vol.27, No.4, pp.142-145

HIRAYAMA, T. et al., Five familial hypercholesterolemic kindreds in Japan with novel mutations of the LDL receptor gene, J. Hum. Genet., 1998, Vol.43, No.4, pp.250-254

Form PCT/ISA/210 (extra sheet) (July 1992)

SEQUENCE LISTING

<110> BML, INC.

<120> Method of Detecting Lipid Metabolism Abnormality

<130> PBM48/PCT

<140>
<141>

<150> JP P2000-218039
<151> 2000-07-18

<160> 44

<170> PatentIn Ver. 2.0

<210> 1
<211> 5082
<212> DNA
<213> Hominidae

<220>
<221> CDS
<222> (64)..(2604)

<400> 1

-300 atcaagtcgcctgccctggcgacactttcgaaggactggagtgggaatcagagcttcacgggtta
aaagccgatgtcacatcggccgttcgaaactcctc -201

-200 ctcttgcagtgaggtgaagacatttgaaaatcaccccactgcaaactcctcccccctgctagaaa
cctcacattgaaatgctgtaaatgacgtgggccccg -101

-100 agtgcaatcgcgggaagccagggtttccagctaggacacagcaggtcgtgatccgggtcgggac
actgcctggcagaggctgcgagcagaggctgcgagc -1

1 atg ggg ccc tgg ggc tgg aaa ttg cgc tgg acc gtc gcc ttg ctc ctc gcc gcg gcg ggg 60
MET Gly Pro Trp Gly Trp Lys Leu Arg Trp Thr Val Ala Leu Leu Leu Ala Ala Ala Gly
-21
-2

act gca gtg ggc gac aga tgt gaa aga aac gag ttc cag tgc caa gac 108
Thr Ala Val Gly Asp Arg Cys Glu Arg Asn Glu Phe Gln Cys Gln Asp
1 5 10 15

ggg aaa tgc atc tcc tac aag tgg gtc tgc gat ggc agc gct gag tgc 156
Gly Lys Cys Ile Ser Tyr Lys Trp Val Cys Asp Gly Ser Ala Glu Cys
20 25 30

cag gat ggc tct gat gag tcc cag gag acg tgc ttg tct gtc acc tgc 204
Gln Asp Gly Ser Asp Glu Ser Gln Glu Thr Cys Leu Ser Val Thr Cys
35 40 45

aaa tcc ggg gac ttc agc tgt ggg ggc cgt gtc aac cgc tgc att cct 252
Lys Ser Gly Asp Phe Ser Cys Gly Gly Arg Val Asn Arg Cys Ile Pro
50 55 60

cag ttc tgg agg tgc gat ggc caa gtg gac tgc gac aac ggc tca gac 300
Gln Phe Trp Arg Cys Asp Gly Gln Val Asp Cys Asp Asn Gly Ser Asp
65 70 75

gag caa ggc tgt ccc ccc aag acg tgc tcc cag gac gag ttt cgc tgc 348
Glu Gln Gly Cys Pro Pro Lys Thr Cys Ser Gln Asp Glu Phe Arg Cys
80 85 90 95

cac gat ggg aag tgc atc tct cgg cag ttc gtc tgt gac tca gac cgg 396
His Asp Gly Lys Cys Ile Ser Arg Gln Phe Val Cys Asp Ser Asp Arg
100 105 110

gac tgc ttg gac ggc tca gac gag gcc tcc tgc ccg gtg ctc acc tgt 444
Asp Cys Leu Asp Gly Ser Asp Glu Ala Ser Cys Pro Val Leu Thr Cys
115 120 125

```
ggt ccc gcc agc ttc cag tgc aac agc tcc acc tgc atc ccc cag ctg     492
Gly Pro Ala Ser Phe Gln Cys Asn Ser Ser Thr Cys Ile Pro Gln Leu
        130                 135                 140

tgg gcc tgc gac aac gac ccc gac tgc gaa gat ggc tcg gat gag tgg     540
Trp Ala Cys Asp Asn Asp Pro Asp Cys Glu Asp Gly Ser Asp Glu Trp
    145                 150                 155

ccg cag cgc tgt agg ggt ctt tac gtg ttc caa ggg gac agt agc ccc     588
Pro Gln Arg Cys Arg Gly Leu Tyr Val Phe Gln Gly Asp Ser Ser Pro
160                 165                 170                 175

tgc tcg gcc ttc gag ttc cac tgc cta agt ggc gag tgc atc cac tcc     636
Cys Ser Ala Phe Glu Phe His Cys Leu Ser Gly Glu Cys Ile His Ser
                180                 185                 190

agc tgg cgc tgt gat ggt ggc ccc gac tgc aag gac aaa tct gac gag     684
Ser Trp Arg Cys Asp Gly Gly Pro Asp Cys Lys Asp Lys Ser Asp Glu
            195                 200                 205

gaa aac tgc gct gtg gcc acc tgt cgc cct gac gaa ttc cag tgc tct     732
Glu Asn Cys Ala Val Ala Thr Cys Arg Pro Asp Glu Phe Gln Cys Ser
        210                 215                 220

gat gga aac tgc atc cat ggc agc cgg cag tgt gac cgg gaa tat gac     780
Asp Gly Asn Cys Ile His Gly Ser Arg Gln Cys Asp Arg Glu Tyr Asp
    225                 230                 235

tgc aag gac atg agc gat gaa gtt ggc tgc gtt aat gtg aca ctc tgc     828
Cys Lys Asp Met Ser Asp Glu Val Gly Cys Val Asn Val Thr Leu Cys
240                 245                 250                 255

gag gga ccc aac aag ttc aag tgt cac agc ggc gaa tgc atc acc ctg     876
Glu Gly Pro Asn Lys Phe Lys Cys His Ser Gly Glu Cys Ile Thr Leu
                260                 265                 270

gac aaa gtc tgc aac atg gct aga gac tgc cgg gac tgg tca gat gaa     924
```

Asp Lys Val Cys Asn Met Ala Arg Asp Cys Arg Asp Trp Ser Asp Glu
275 280 285

ccc atc aaa gag tgc ggg acc aac gaa tgc ttg gac aac aac ggc ggc 972
Pro Ile Lys Glu Cys Gly Thr Asn Glu Cys Leu Asp Asn Asn Gly Gly
290 295 300

tgt tcc cac gtc tgc aat gac ctt aag atc ggc tac gag tgc ctg tgc 1020
Cys Ser His Val Cys Asn Asp Leu Lys Ile Gly Tyr Glu Cys Leu Cys
305 310 315

ccc gac ggc ttc cag ctg gtg gcc cag cga aga tgc gaa gat atc gat 1068
Pro Asp Gly Phe Gln Leu Val Ala Gln Arg Arg Cys Glu Asp Ile Asp
320 325 330 335

gag tgt cag gat ccc gac acc tgc agc cag ctc tgc gtg aac ctg gag 1116
Glu Cys Gln Asp Pro Asp Thr Cys Ser Gln Leu Cys Val Asn Leu Glu
340 345 350

ggt ggc tac aag tgc cag tgt gag gaa ggc ttc cag ctg gac ccc cac 1164
Gly Gly Tyr Lys Cys Gln Cys Glu Glu Gly Phe Gln Leu Asp Pro His
355 360 365

acg aag gcc tgc aag gct gtg ggc tcc atc gcc tac ctc ttc ttc acc 1212
Thr Lys Ala Cys Lys Ala Val Gly Ser Ile Ala Tyr Leu Phe Phe Thr
370 375 380

aac cgg cac gag gtc agg aag atg acg ctg gac cgg agc gag tac acc 1260
Asn Arg His Glu Val Arg Lys Met Thr Leu Asp Arg Ser Glu Tyr Thr
385 390 395

agc ctc atc ccc aac ctg agg aac gtg gtc gct ctg gac acg gag gtg 1308
Ser Leu Ile Pro Asn Leu Arg Asn Val Val Ala Leu Asp Thr Glu Val
400 405 410 415

gcc agc aat aga atc tac tgg tct gac ctg tcc cag aga atg atc tgc 1356
Ala Ser Asn Arg Ile Tyr Trp Ser Asp Leu Ser Gln Arg Met Ile Cys
420 425 430

```
agc acc cag ctt gac aga gcc cac ggc gtc tct tcc tat gac acc gtc    1404
Ser Thr Gln Leu Asp Arg Ala His Gly Val Ser Ser Tyr Asp Thr Val
                435                 440                 445

atc agc agg gac atc cag gcc ccc gac ggg ctg gct gtg gac tgg atc    1452
Ile Ser Arg Asp Ile Gln Ala Pro Asp Gly Leu Ala Val Asp Trp Ile
            450                 455                 460

cac agc aac atc tac tgg acc gac tct gtc ctg ggc act gtc tct gtt    1500
His Ser Asn Ile Tyr Trp Thr Asp Ser Val Leu Gly Thr Val Ser Val
    465                 470                 475

gcg gat acc aag ggc gtg aag agg aaa acg tta ttc agg gag aac ggc    1548
Ala Asp Thr Lys Gly Val Lys Arg Lys Thr Leu Phe Arg Glu Asn Gly
480                 485                 490                     495

tcc aag cca agg gcc atc gtg gtg gat cct gtt cat ggc ttc atg tac    1596
Ser Lys Pro Arg Ala Ile Val Val Asp Pro Val His Gly Phe Met Tyr
                    500                 505                 510

tgg act gac tgg gga act ccc gcc aag atc aag aaa ggg ggc ctg aat    1644
Trp Thr Asp Trp Gly Thr Pro Ala Lys Ile Lys Lys Gly Gly Leu Asn
                515                 520                 525

ggt gtg gac atc tac tcg ctg gtg act gaa aac att cag tgg ccc aat    1692
Gly Val Asp Ile Tyr Ser Leu Val Thr Glu Asn Ile Gln Trp Pro Asn
            530                 535                 540

ggc atc acc cta gat ctc ctc agt ggc cgc ctc tac tgg gtt gac tcc    1740
Gly Ile Thr Leu Asp Leu Leu Ser Gly Arg Leu Tyr Trp Val Asp Ser
    545                 550                 555

aaa ctt cac tcc atc tca agc atc gat gtc aat ggg ggc aac cgg aag    1788
Lys Leu His Ser Ile Ser Ser Ile Asp Val Asn Gly Gly Asn Arg Lys
560                 565                 570                     575

acc atc ttg gag gat gaa aag agg ctg gcc cac ccc ttc tcc ttg gcc    1836
```

Thr Ile Leu Glu Asp Glu Lys Arg Leu Ala His Pro Phe Ser Leu Ala
580 585 590

gtc ttt gag gac aaa gta ttt tgg aca gat atc atc aac gaa gcc att 1884
Val Phe Glu Asp Lys Val Phe Trp Thr Asp Ile Ile Asn Glu Ala Ile
595 600 605

ttc agt gcc aac cgc ctc aca ggt tcc gat gtc aac ttg ttg gct gaa 1932
Phe Ser Ala Asn Arg Leu Thr Gly Ser Asp Val Asn Leu Leu Ala Glu
610 615 620

aac cta ctg tcc cca gag gat atg gtc ctc ttc cac aac ctc acc cag 1980
Asn Leu Leu Ser Pro Glu Asp Met Val Leu Phe His Asn Leu Thr Gln
625 630 635

cca aga gga gtg aac tgg tgt gag agg acc acc ctg agc aat ggc ggc 2028
Pro Arg Gly Val Asn Trp Cys Glu Arg Thr Thr Leu Ser Asn Gly Gly
640 645 650 655

tgc cag tat ctg tgc ctc cct gcc ccg cag atc aac ccc cac tcg ccc 2076
Cys Gln Tyr Leu Cys Leu Pro Ala Pro Gln Ile Asn Pro His Ser Pro
660 665 670

aag ttt acc tgc gcc tgc ccg gac ggc atg ctg ctg gcc agg gac atg 2124
Lys Phe Thr Cys Ala Cys Pro Asp Gly Met Leu Leu Ala Arg Asp Met
675 680 685

agg agc tgc ctc aca gag gct gag gct gca gtg gcc acc cag gag aca 2172
Arg Ser Cys Leu Thr Glu Ala Glu Ala Ala Val Ala Thr Gln Glu Thr
690 695 700

tcc acc gtc agg cta aag gtc agc tcc aca gcc gta agg aca cag cac 2220
Ser Thr Val Arg Leu Lys Val Ser Ser Thr Ala Val Arg Thr Gln His
705 710 715

aca acc acc cgg cct gtt ccc gac acc tcc cgg ctg cct ggg gcc acc 2268
Thr Thr Thr Arg Pro Val Pro Asp Thr Ser Arg Leu Pro Gly Ala Thr
720 725 730 735

```
cct ggg ctc acc acg gtg gag ata gtg aca atg tct cac caa gct ctg    2316
Pro Gly Leu Thr Thr Val Glu Ile Val Thr Met Ser His Gln Ala Leu
                    740                     745                     750

ggc gac gtt gct ggc aga gga aat gag aag aag ccc agt agc gtg agg    2364
Gly Asp Val Ala Gly Arg Gly Asn Glu Lys Lys Pro Ser Ser Val Arg
            755                     760                     765

gct ctg tcc att gtc ctc ccc atc gtg ctc ctc gtc ttc ctt tgc ctg    2412
Ala Leu Ser Ile Val Leu Pro Ile Val Leu Leu Val Phe Leu Cys Leu
        770                     775                     780

ggg gtc ttc ctt cta tgg aag aac tgg cgg ctt aag aac atc aac agc    2460
Gly Val Phe Leu Leu Trp Lys Asn Trp Arg Leu Lys Asn Ile Asn Ser
    785                     790                     795

atc aac ttt gac aac ccc gtc tat cag aag acc aca gag gat gag gtc    2508
Ile Asn Phe Asp Asn Pro Val Tyr Gln Lys Thr Thr Glu Asp Glu Val
800                     805                     810                     815

cac att tgc cac aac cag gac ggc tac agc tac ccc tcg aga cag atg    2556
His Ile Cys His Asn Gln Asp Gly Tyr Ser Tyr Pro Ser Arg Gln Met
                    820                     825                     830

gtc agt ctg gag gat gac gtg gcg tga aca tct gcc tgg agt ccc gcc    2604
Val Ser Leu Glu Asp Asp Val Ala
            835

cctgcccaga acccttcctg agacctcgcc ggccttgttt tattcaaaga cagagaagac 2664
caaagcattg cctgccagag ctttgtttta tatatttatt catctgggag gcagaacagg 2724
cttcggacag tgcccatgca atggcttggg ttgggatttt ggtttcttcc tttcctgtga 2784
aggataagag aaacaggccc gggggggacca ggatgacacc tccatttctc tccaggaagt 2844
tttgagtttc tctccaccgt gacacaatcc tcaaacatgg aagatgaaag ggcaggggat 2904
gtcaggccca gagaagcaag tggctttcaa cacacaacag cagatggcac caacgggacc 2964
ccctggccct gcctcatcca ccaatctcta agccaaaccc ctaaactcag gagtcaacgt 3024
gtttacctct tctatgcaag ccttgctaga cagccaggtt agcctttgcc ctgtcacccc 3084
cgaatcatga cccacccagt gtctttcgag gtgggtttgt accttcctta agccaggaaa 3144
```

gggattcatg gcgtcggaaa tgatctggct gaatccgtgg tggcaccgag accaaactca 3204
ttcaccaaat gatgccactt cccagaggca gagcctgagt caccggtcac ccttaatatt 3264
tattaagtgc ctgagacacc cggttacctt ggccgtgagg acacgtggcc tgcacccagg 3324
tgtggctgtc aggacaccag cctggtgccc atcctcccga cccctaccca cttccattcc 3384
cgtggtctcc ttgcactttc tcagttcaga gttgtacact gtgtacattt ggcatttgtg 3444
ttattatttt gcactgtttt ctgtcgtgtg tgttgggatg ggatcccagg ccagggaaag 3504
cccgtgtcaa tgaatgccgg ggacagagag gggcaggttg accgggactt caaagccgtg 3564
atcgtgaata tcgagaactg ccattgtcgt ctttatgtcc gcccacctag tgcttccact 3624
tctatgcaaa tgcctccaag ccattcactt ccccaatctt gtcgttgatg ggtatgtgtt 3684
taaaacatgc acggtgaggc cgggcgcagt ggcctcacgc ctgtaatccc agcactttgg 3744
gaggccgagg cgggtggatc atgaggtcag gagatcgaga ccatcctggc taacaaggtg 3804
aaaccccgtc tctactaaaa atacaaaaaa ttagccgggc gcggtggtgg gcacctgtag 3864
tcccagctac tcgggaggct gaggcaggag aatggtgtga acccgggaag cggagcttgc 3924
agtgagccga gattgcgcca ctgcagtccg cagtctggcc tgggcgacag agcgagactc 3984
cgtctcaaaa aaaacaaaac aaaaaaaaac catgcatggt gcatcagcag cccatggcct 4044
ctggccaggc atggcgaggc tgaggtggga ggatggtttg agctcaggca tttgaggctg 4104
tcgtgagcta tgattatgcc actgctttcc agcctgggca acatagtaag accccatctc 4164
ttaaaaaatg aatttggcca gacacaggtg cctcacgcct gtaatcccag cactttggga 4224
ggctgagctg gatcacttga gttcaggagt tggagaccag gcctgagcaa caaagcgaga 4284
tcccatctct acaaaaacca aaaagttaaa aatcagctgg gtatggtggc acgtgcctgt 4344
gatcccagct acttgggagg ctgaggcagg aggatcgcct gagcccagga ggtggaggtt 4404
gcagtgagcc atgatcgagc cactgcactc cagcctgggc aacagatgaa gaccctattt 4464
cagaaataca actataaaaa aaataaataa atcctccagt ctggatcgtt tgacgggact 4524
tcaggttctt tctgaaatcg ccgtgttact gttgcactga tgtccggaga gacagtgaca 4584
gcctccgtca gactcccgcg tgaagatgtc acaagggatt ggcaattgtc cccagggaca 4644
aaacactgtg tcccccccag tgcagggaac cgtgataagc ctttctggtt tcggagcacg 4704
taaatgcgtc cctgtacaga tagtggggat tttttgttat gtttgcactt tgtatattgg 4764
ttgaaactgt tatcacttat atatatatat acacacatat atataaaatc tatttatttt 4824
tgcaaaccct ggttgctgta tttgttcagt gactattctc ggggccctgt gtaggggtt 4884
attgcctctg aaatgcctct tctttatgta caaagattat ttgcacgaac tggactgtgt 4944
gcaacgcttt ttgggagaat gatgtccccg ttgtatgtat gagtggcttc tgggagatgg 5004
gtgtcactt ttaaaccact gtatagaagg tttttgtagc ctgaatgtct tactgtgatc 5064
aattaaattt cttaaatg 5082

<210> 2
<211> 25
<212> DNA

<213> Hominidae

<400> 2
gagtgggaat cagagcttca cgggt 25

<210> 3
<211> 25
<212> DNA
<213> Hominidae

<400> 3
ccacgtcatt tacagcattt caatg 25

<210> 4
<211> 25
<212> DNA
<213> Hominidae

<400> 4
actcctcccc ctgctagaaa cctca 25

<210> 5
<211> 25
<212> DNA
<213> Hominidae

<400> 5
ttctggcgct tggagcaagc cttac 25

<210> 6
<211> 25
<212> DNA
<213> Hominidae

<400> 6
cctttctcct tttcctctct ctcag 25

<210> 7
<211> 25
<212> DNA
<213> Hominidae

<400> 7
aaaataaatg catatcatgc ccaaa 25

<210> 8
<211> 25
<212> DNA
<213> Hominidae

<400> 8
tgacagttca atcctgtctc ttctg 25

<210> 9
<211> 25
<212> DNA
<213> Hominidae

<400> 9
atagcaaagg cagggccaca cttac 25

<210> 10
<211> 25
<212> DNA
<213> Hominidae

<400> 10
gttgggagac ttcacacggt gatgg 25

<210> 11
<211> 25
<212> DNA
<213> Hominidae

<400> 11
acttaggcag tggaactcga aggcc 25

<210> 12
<211> 25
<212> DNA
<213> Hominidae

<400> 12
ccccagctgt gggcctgcga caacg 25

<210> 13
<211> 25
<212> DNA
<213> Hominidae

<400> 13
gggggagccc agggacaggt gatag 25

<210> 14
<211> 25
<212> DNA
<213> Hominidae

<400> 14
caacacactc tgtcctgttt tccag 25

<210> 15
<211> 25
<212> DNA
<213> Hominidae

<400> 15
ggaaaaccag atggccagcg ctcac 25

<210> 16
<211> 25
<212> DNA
<213> Hominidae

<400> 16
tccttcctct ctctggctct cacag 25

<210> 17
<211> 25
<212> DNA
<213> Hominidae

<400> 17
gcaagccgcc tgcaccgaga ctcac 25

<210> 18
<211> 25
<212> DNA
<213> Hominidae

<400> 18
agtctgactc cctggccctg cgcag 25

<210> 19
<211> 25

<212> DNA
<213> Hominidae

<400> 19
agggctcagt ccaccgggga atcac 25

<210> 20
<211> 25
<212> DNA
<213> Hominidae

<400> 20
ccaagcctct ttctctctct tccag 25

<210> 21
<211> 25
<212> DNA
<213> Hominidae

<400> 21
ccacccgccg ccttcccgtg ctcac 25

<210> 22
<211> 25
<212> DNA
<213> Hominidae

<400> 22
tccatcgacg ggtcccctct gaccc 25

<210> 23
<211> 25
<212> DNA
<213> Hominidae

<400> 23
agccctcatc tcacctgcgg gccaa 25

<210> 24
<211> 25
<212> DNA
<213> Hominidae

<400> 24
agatgagggc tcctggtgcg atgcc 25

<210> 25
<211> 25
<212> DNA
<213> Hominidae

<400> 25
gcccttggta tccgcaacag agaca 25

<210> 26
<211> 25
<212> DNA
<213> Hominidae

<400> 26
gatccacagc aacatctact ggacc 25

<210> 27
<211> 25
<212> DNA
<213> Hominidae

<400> 27

agccctcagc gtcgtggata cgcac 25

<210> 28
<211> 25
<212> DNA
<213> Hominidae

<400> 28
cagctattct ctgctctccc accag 25

<210> 29
<211> 25
<212> DNA
<213> Hominidae

<400> 29
tgggacggct gtcctcgcaa catac 25

<210> 30
<211> 25
<212> DNA
<213> Hominidae

<400> 30
gcacgtgacc tctccttatc cactt 25

<210> 31
<211> 25
<212> DNA
<213> Hominidae

<400> 31
cacctaagtg cttcgatctc gtacg 25

<210> 32
<211> 25
<212> DNA
<213> Hominidae

<400> 32
gtcatcttcc ttgctgcctg tttag 25

<210> 33
<211> 25
<212> DNA
<213> Hominidae

<400> 33
gtttccacaa ggaggtttca aggtt 25

<210> 34
<211> 25
<212> DNA
<213> Hominidae

<400> 34
cctgactccg cttcttctgc cccag 25

<210> 35
<211> 25
<212> DNA
<213> Hominidae

<400> 35
cgcagaaaca aggcgtgtgc cacac 25

<210> 36

<211> 25
<212> DNA
<213> Hominidae

<400> 36
gaagggcctg caggcacgtg gcact 25

<210> 37
<211> 25
<212> DNA
<213> Hominidae

<400> 37
gtgtggtggc gggcccagtc tttac 25

<210> 38
<211> 25
<212> DNA
<213> Hominidae

<400> 38
cctcactctt gcttctctcc tgcag 25

<210> 39
<211> 25
<212> DNA
<213> Hominidae

<400> 39
cgctggggga ccggcccgcg cttac 25

<210> 40
<211> 25
<212> DNA

<213> Hominidae

<400> 40
tgacagagcg tgcctctccc tacag 25

<210> 41
<211> 25
<212> DNA
<213> Hominidae

<400> 41
gctttctaga gagggtcaca ctcac 25

<210> 42
<211> 25
<212> DNA
<213> Hominidae

<400> 42
tccgctgttt accatttgtt ggcag 25

<210> 43
<211> 25
<212> DNA
<213> Hominidae

<400> 43
aataaaacaa ggccggcgag gtctc 25